(19) Europäisches Patentamt
European Patent Office
Office européen des brevets

(11) **EP 3 872 172 A1**

(12) **EUROPEAN PATENT APPLICATION**

(43) Date of publication:
01.09.2021 Bulletin 2021/35

(51) Int Cl.:
*C12N 9/42* (2006.01)   *C12P 7/10* (2006.01)
*B82Y 30/00* (2011.01)   *C01B 32/20* (2017.01)
*C08K 3/04* (2006.01)   *D21H 11/18* (2006.01)
*D21H 13/50* (2006.01)

(21) Application number: 20382140.0

(22) Date of filing: 28.02.2020

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicants:
• CIC nanoGUNE - Asociación Centro de
Investigación Cooperativa en Nanociencias
20018 San Sebastian, Guipuzcoa (ES)
• Universidad del País Vasco/Euskal Herriko
Unibertsitatea
48940 Leoia, Bikaia (ES)

(72) Inventors:
• PÉREZ JIMÉNEZ, Raúl
E-20018 San Sebastian - Guipuzcoa (ES)
• ALONSO LEMA, Borja
E-20018 San Sebastian - Guipuzcoa (ES)
• ECEIZA MENDIGUREN, María Aranzazu
E-48940 Leioa - Vizcaya (ES)

(74) Representative: ABG Intellectual Property Law,
S.L.
Avenida de Burgos, 16D
Edificio Euromor
28036 Madrid (ES)

(54) **CONDUCTIVE CELLULOSE COMPOSITE MATERIALS AND USES THEREOF**

(57) The present invention refers to a cellulose composite material, methods of preparation and uses thereof. The composite material comprises type I cellulose fibers selected from the group consisting of cellulose nanocrystals, cellulose nanofibers and a mixture thereof and a carbon material selected from the group consisting of carbon nanotubes, carbon nanofibers, graphene, graphene oxide and a mixture thereof. The invention further relates to conductive paint compositions comprising said composite material.

EP 3 872 172 A1

SEQUENCE LISTING

<110> CIC NANOGUNE – ASOCIACIÓN CENTRO DE INVESTIGACIÓN
COOPERATIVA EN NANOCIENCIAS
UNIVERSIDAD DEL PAÍS VASCO / EUSKAL HERRIKO UNIBERTSITATEA

<120> CONDUCTIVE CELLULOSE COMPOSITE MATERIALS AND USES THEREOF

<130> P19044EP00

<160> 34

<170> PatentIn version 3.5

<210> 1
<211> 297
<212> PRT
<213> Artificial Sequence

<220>
<223> Ancestral endoglucanase catalytic domain

<400> 1

```
Thr Pro Val Glu Thr His Gly Gln Leu Ser Val Lys Gly Gly Gln Leu
1               5                   10                  15


Val Asp Glu Asn Gly Lys Pro Val Gln Leu Arg Gly Met Ser Ser His
            20                  25                  30


Gly Leu Gln Trp Phe Gly Asp Phe Val Asn Lys Asp Ser Met Lys Trp
            35                  40                  45


Leu Arg Asp Asp Trp Gly Ile Asn Val Phe Arg Val Ala Met Tyr Thr
        50                  55                  60


Ala Glu Gly Gly Tyr Ile Thr Asn Pro Ser Val Lys Asn Lys Val Lys
65                  70                  75                  80


Glu Ala Val Glu Ala Ala Ile Asp Leu Gly Met Tyr Val Ile Ile Asp
                85                  90                  95


Trp His Ile Leu Ser Asp Asn Asp Pro Asn Thr Tyr Lys Glu Gln Ala
            100                 105                 110


Lys Ala Phe Phe Gln Glu Met Ala Ala Lys Tyr Gly Asn Tyr Pro Asn
            115                 120                 125


Val Ile Tyr Glu Ile Cys Asn Glu Pro Asn Gly Gly Val Thr Trp Ser
        130                 135                 140


Asn Gln Ile Lys Pro Tyr Ala Glu Glu Val Ile Pro Ala Ile Arg Ala
145                 150                 155                 160
```

```
Asn Asp Pro Asp Asn Ile Ile Ile Val Gly Thr Pro Thr Trp Ser Gln
            165             170             175

Asp Val His Asp Ala Ala Asp Asn Pro Leu Pro Tyr Ser Asn Ile Met
            180             185             190

Tyr Ala Leu His Phe Tyr Ala Gly Thr His Gly Gln Ser Leu Arg Asp
            195             200             205

Lys Ile Asp Tyr Ala Leu Ser Lys Gly Val Ala Ile Phe Val Thr Glu
    210             215             220

Trp Gly Thr Ser Asp Ala Ser Gly Asn Gly Gly Pro Phe Leu Asn Glu
225             230             235             240

Ser Gln Lys Trp Ile Asp Phe Met Asn Ser Arg Asn Ile Ser Trp Ala
            245             250             255

Asn Trp Ser Leu Ser Asp Lys Ser Glu Thr Ser Ala Ala Leu Met Pro
            260             265             270

Gly Ala Ser Pro Thr Gly Gly Trp Thr Asp Ser Asn Leu Ser Ala Ser
            275             280             285

Gly Lys Phe Val Arg Glu Gln Ile Arg
    290             295
```

```
<210>  2
<211>  297
<212>  PRT
<213>  Artificial Sequence

<220>
<223>  Ancestral endoglucanase catalytic domain

<400>  2

Thr Pro Val Glu Ile Asn Gly Gln Leu Gln Val Cys Gly Val Gln Leu
1               5               10              15

Cys Asn Gln Tyr Gly Lys Pro Ile Gln Leu Arg Gly Met Ser Thr His
            20              25              30

Gly Ile Gln Trp Phe Gly Asn Cys Val Asn Asn Ala Ser Leu Asp Ala
            35              40              45

Leu Ala Asn Asp Trp Arg Ala Asp Ile Phe Arg Ile Ala Met Tyr Ile
    50              55              60
```

```
Gln Glu Asp Gly Tyr Glu Thr Asn Pro Ala Gly Thr Asn Arg Val Asn
65              70              75                      80

Asn Leu Val Glu Glu Ala Thr Ala Arg Gly Met Tyr Val Leu Ile Asp
                85              90                      95

Trp His Ile Leu Thr Pro Gly Asp Pro Asn Tyr Asn Leu Asp Arg Ala
            100             105             110

Lys Thr Phe Phe Ala Glu Ile Ala Ala Arg His Ala Ser Lys Thr Asn
            115             120             125

Val Ile Tyr Glu Ile Ala Asn Glu Pro Asn Gly Gly Val Ser Trp Ser
            130             135             140

Asn Thr Ile Lys Ser Tyr Ala Glu Glu Val Ile Pro Val Ile Arg Ala
145             150             155             160

Asn Asp Pro Asp Ser Val Val Ile Val Gly Thr Arg Gly Trp Ser Ser
            165             170             175

Asp Ser Thr Glu Ile Val Asn Asn Pro Val Asn Ala Ser Asn Ile Met
            180             185             190

Tyr Ala Phe His Phe Tyr Ala Ala Ser His Arg Asp Asn Tyr Arg Asp
            195             200             205

Glu Val Glu Arg Ala Ala Ala Arg Gly Leu Pro Val Phe Val Thr Glu
            210             215             220

Phe Gly Thr Val Thr Tyr Thr Gly Asp Gly Gly Asn Asp Leu Ala Ser
225             230             235             240

Ser Gln Lys Trp Leu Asp Leu Leu Asp Ala Arg Lys Ile Gly Trp Ala
            245             250             255

Asn Trp Asn Phe Ser Asp Lys Ala Glu Ser Ser Ala Ala Leu Arg Pro
            260             265             270

Gly Thr Cys Ala Gly Gly Ser Trp Thr Gly Thr Ser Leu Thr Pro Ser
            275             280             285

Gly Val Phe Val Arg Glu Arg Ile Arg
    290             295
```

```
<210>   3
<211>   297
<212>   PRT
```

<213> Artificial Sequence

<220>
<223> Ancestral endoglucanase catalytic domain

<400> 3

```
Thr Pro Val Glu Thr His Gly Gln Leu Ser Val Lys Gly Gly Gln Leu
1               5               10                  15

Val Asp Glu Asn Gly Lys Pro Val Gln Leu Arg Gly Met Ser Ser His
            20                  25                  30

Gly Leu Gln Trp Phe Gly Asp Phe Val Asn Lys Asp Ser Met Lys Trp
        35                  40                  45

Leu Arg Asp Asp Trp Gly Ile Asn Val Phe Arg Val Ala Met Tyr Thr
        50                  55                  60

Ala Glu Asp Gly Tyr Ile Thr Asn Pro Ser Val Lys Asn Lys Val Lys
65                  70                  75                  80

Glu Ala Val Glu Ala Ala Ile Asp Leu Gly Met Tyr Val Ile Ile Asp
                85                  90                  95

Trp His Ile Leu Ser Asp Asn Asp Pro Asn Thr Tyr Lys Ala Gln Ala
            100                 105                 110

Lys Ala Phe Phe Gln Glu Met Ala Ala Leu Tyr Gly Asn Tyr Pro Asn
        115                 120                 125

Val Ile Tyr Glu Ile Ala Asn Glu Pro Asn Gly Asn Val Thr Trp Asn
        130                 135                 140

Asn Gln Ile Lys Pro Tyr Ala Glu Glu Val Ile Pro Val Ile Arg Ala
145                 150                 155                 160

Lys Asp Pro Asp Asn Ile Ile Ile Val Gly Thr Gly Thr Trp Ser Gln
                165                 170                 175

Asp Val His Asp Ala Ala Asp Asn Pro Leu Pro Asp Ser Asn Ile Met
            180                 185                 190

Tyr Ala Leu His Phe Tyr Ala Gly Thr His Gly Gln Phe Leu Arg Asp
        195                 200                 205

Arg Ile Asp Tyr Ala Leu Ser Lys Gly Ala Ala Ile Phe Val Thr Glu
        210                 215                 220
```

```
Trp Gly Thr Ser Asp Ala Ser Gly Asn Gly Gly Pro Phe Leu Pro Glu
225             230             235             240


Ser Gln Glu Trp Ile Asp Phe Met Asn Ser Arg Lys Ile Ser Trp Ala
            245             250             255


Asn Trp Ser Leu Ser Asp Lys Ser Glu Thr Ser Ala Ala Leu Met Pro
            260             265             270


Gly Ala Ser Pro Thr Gly Gly Trp Thr Glu Ser Gln Leu Ser Ala Ser
            275             280             285


Gly Lys Phe Val Arg Glu Gln Ile Arg
    290             295
```

```
<210>  4
<211>  150
<212>  PRT
<213>  Artificial Sequence

<220>
<223>  Carbohydrate binding domain

<400>  4
```

```
Gln Glu Asn Gly Ile Ser Val Gln Tyr Arg Ala Gly Asp Gly Ser Met
1               5               10              15


Asn Ser Asn Gln Ile Arg Pro Gln Leu Gln Ile Lys Asn Asn Gly Asn
            20              25              30


Thr Thr Val Asp Leu Lys Asp Val Thr Ala Arg Tyr Trp Tyr Asn Ala
            35              40              45


Lys Asn Lys Gly Gln Asn Val Asp Cys Asp Tyr Ala Gln Leu Gly Cys
    50              55              60


Gly Asn Val Thr Tyr Lys Phe Val Thr Leu His Lys Pro Lys Gln Gly
65              70              75              80


Ala Asp Thr Tyr Leu Glu Leu Gly Phe Lys Asn Gly Thr Leu Ala Pro
            85              90              95


Gly Ala Ser Thr Gly Asn Ile Gln Leu Arg Leu His Asn Asp Asp Trp
            100             105             110


Ser Asn Tyr Ala Gln Ser Gly Asp Tyr Ser Phe Phe Lys Ser Asn Thr
            115             120             125


Phe Lys Thr Thr Lys Lys Ile Thr Leu Tyr Asp Gln Gly Lys Leu Ile
```

                    130                    135                    140


          Trp Gly Thr Glu Pro Asn
          145                    150



          <210>  5
          <211>  15
          <212>  PRT
          <213>  Artificial Sequence

          <220>
          <223>  Linking domain

          <400>  5

          Gly Thr Lys Asp Ile Pro Glu Thr Pro Ala Lys Asp Lys Pro Thr
          1               5                   10                  15



          <210>  6
          <211>  87
          <212>  PRT
          <213>  Artificial Sequence

          <220>
          <223>  Carbohydrate binding domain

          <400>  6

          Phe Thr Val Gln Trp Lys Lys Ser Ser Ser Trp Glu Glu Gly Gly Lys
          1               5                   10                  15


          Lys Cys Gly Gly Tyr Glu Ile Val Ile Thr Asn Asn Gly Asp Thr Val
                      20                  25                  30


          Asn Ser Trp Thr Ala Lys Val Thr Val Pro Gly Asn Thr Lys Leu Met
                      35                  40                  45


          Ser Gln Trp Asn Gly Ile Phe Ser Ile Ser Gly Asn Thr Met Thr Val
                      50                  55                  60


          Lys Asn Glu Ser Tyr Asn Gly Thr Ile Glu Lys Gly Lys Ser Ile Ser
          65                  70                  75                  80


          Phe Gly Phe Asn Tyr Ser Ala
                              85



          <210>  7
          <211>  87
          <212>  PRT
          <213>  Artificial Sequence

          <220>
          <223>  Carbohydrate binding domain

```
<400>   7

Tyr Gln Leu Lys Val Thr Val Ser Ser Ser Trp Glu Ser Gly Asp Gly
1               5                   10                  15


Tyr Gly Gly Thr Tyr Ser Leu Ser Phe Thr Asn Arg Ser Ala Ala Val
            20                  25                  30


Lys Ala Trp Asp Ala Gln Ile Glu Val Pro Glu Gly Ser Lys Val Thr
        35                  40                  45


Glu Ala Trp Gly Cys Glu Thr Gly Ile Asp Gly Thr Ile Leu Thr Val
        50                  55                  60


Thr Ala Lys Asp Trp Gly Ala Ala Val Ala Lys Gly Ser Thr Val Glu
65                  70                  75                  80


Ile Gly Phe Asn Met Asp Thr
                    85


<210>   8
<211>   84
<212>   PRT
<213>   Artificial Sequence

<220>
<223>   Carbohydrate binding domain

<400>   8

Tyr Lys Ala Asp Phe Lys Val Val Asn Ser Trp Glu Glu Gly Gly Lys
1               5                   10                  15


Lys Cys Tyr Gln Leu Ser Gly Thr Leu Thr Asn Leu Ser Ser Ser Ile
            20                  25                  30


Ser Ser Trp Thr Val Val Phe Asp Ala Gly Asn Gly Ala Glu Ile Lys
        35                  40                  45


Gln Phe Trp Asn Ser Lys Cys Thr Ile Ser Gly Asn Lys Ile Thr Val
        50                  55                  60


Gly Pro Ala Asp Tyr Asn Ser Arg Ile Gly Thr Gly Ala Ser Val Ser
65                  70                  75                  80


Asp Val Gly Met


<210>   9
<211>   87
<212>   PRT
```

<210> Artificial Sequence

<220>
<223> Carbohydrate binding domain

<400> 9

Phe Tyr Ala Glu Ile Gly His Asp Ser Thr Trp Glu Ala Ser Gly Lys
1               5                   10                  15

Thr Cys Ala Thr Glu Asn Ile Asn Ile Tyr Asn Lys Thr Ser Ser Val
            20                  25                  30

Ser Gly Trp Lys Leu Asp Val Ile Tyr Lys Gly Lys Pro Ala Ile Glu
        35                  40                  45

Asp Ile Trp Asn Gly Glu Lys Lys Ile Asn Glu Tyr Thr Val Ser Ile
    50                  55                  60

Thr Pro Ala Asp Tyr Asn Gln Asp Ile Pro Ala Gly Gly Ser Val Asn
65                  70                  75                  80

Val Gly Tyr Asn Ile Ala Ser
                85

<210> 10
<211> 87
<212> PRT
<213> Artificial Sequence

<220>
<223> Carbohydrate binding domain

<400> 10

Tyr Tyr Ala Ser Ile Ser Asp Asn Ser Ser Trp Gln Glu Asn Gly Lys
1               5                   10                  15

Asn Ala Ala Thr Lys Asn Val Ile Ile Tyr Asn Lys Asp Lys Lys Val
            20                  25                  30

Thr Gly Trp Lys Ile Glu Leu Val Phe Ala Ser Glu Pro Glu Leu Ala
        35                  40                  45

Asp Ile Trp Gly Gly Lys Ala Glu Val Asn Gly Asp Thr Ile Thr Val
    50                  55                  60

Val Gly Tyr Asp Tyr Thr Ala Glu Leu Lys Ala Gly Gly Asn Val Asn
65                  70                  75                  80

Phe Gly Phe Asn Val Lys Ala
                85

<210> 11
<211> 77
<212> PRT
<213> Artificial Sequence

<220>
<223> Carbohydrate binding domain

<400> 11

Trp Ala Glu Gln Asn Gln Thr Ala Phe Gln Tyr Glu Leu Leu Phe Ser
1               5                   10                  15


Asn Gln Ser Glu Ala Phe Gly Thr Trp Lys Val Ile Leu Asp Thr Gly
            20                  25                  30


Lys Glu Val Lys Val Gln Asn Ser Trp Asn Cys Ser Leu Glu Ala Asp
        35                  40                  45


Gly Asn Leu Leu Thr Phe Thr Pro Ala Asp Tyr Asn Ala Lys Leu Ala
    50                  55                  60


Lys Gly Ala Glu Met Ala Asp Val Gly Met Ile Leu Val
65                  70                  75


<210> 12
<211> 85
<212> PRT
<213> Artificial Sequence

<220>
<223> Carbohydrate binding domain

<400> 12

Met Gln Phe Lys Gln Ser Asn Ser Trp Glu Gly Asn Gly Arg Phe Tyr
1               5                   10                  15


Ala Gln Tyr Asp Leu Ile Ile Asp Asn Lys Glu Asp Val Ile Ser Asp
            20                  25                  30


Trp Thr Phe Lys Ile Asn Thr Thr Asn Gly Thr Thr Leu Glu Gln Ser
        35                  40                  45


Trp Asn Cys Thr Val Asp Thr Ser Asp Leu Gln Trp Ser Val Val Pro
    50                  55                  60


Val Asp Tyr Asn Lys Ile Ile Glu Ser Gln Ala Gln Met Asn Asn Val
65                  70                  75                  80


Gly Phe Ile Ile Ser

85

```
<210>    13
<211>    147
<212>    PRT
<213>    Artificial Sequence

<220>
<223>    Carbohydrate binding domain

<400>    13

Asn Gly Ile Ser Val Gln Tyr Arg Ala Gly Asp Gly Ser Met Asn Ser
1               5                   10                  15


Asn Gln Ile Arg Pro Gln Leu Gln Ile Lys Asn Asn Gly Asn Thr Thr
            20                  25                  30


Val Asp Leu Lys Asp Val Thr Ala Arg Tyr Trp Tyr Asn Ala Lys Asn
        35                  40                  45


Lys Gly Gln Asn Val Asp Cys Asp Tyr Ala Gln Leu Gly Cys Gly Asn
    50                  55                  60


Val Thr Tyr Lys Phe Val Thr Leu His Lys Pro Lys Gln Gly Ala Asp
65                  70                  75                  80


Thr Tyr Leu Glu Leu Gly Phe Lys Asn Gly Thr Leu Ala Pro Gly Ala
                85                  90                  95


Ser Thr Gly Asn Ile Gln Leu Arg Leu His Asn Asp Asp Trp Ser Asn
            100                 105                 110


Tyr Ala Gln Ser Gly Asp Tyr Ser Phe Phe Lys Ser Asn Thr Phe Lys
            115                 120                 125


Thr Thr Lys Lys Ile Thr Leu Tyr Asp Gln Gly Lys Leu Ile Trp Gly
    130                 135                 140


Thr Glu Pro
145


<210>    14
<211>    137
<212>    PRT
<213>    Artificial Sequence

<220>
<223>    Carbohydrate binding domain

<400>    14
```

EP 3 872 172 A1

```
Asn Gly Ile Ser Val Gln Tyr Arg Ala Gly Asp Gly Ser Met Asn Ser
1               5                   10                  15

Asn Gln Ile Arg Pro Gln Leu Gln Ile Lys Asn Asn Gly Asn Thr Thr
            20                  25                  30

Val Asp Leu Lys Asp Val Thr Ala Arg Tyr Trp Tyr Lys Ala Lys Asn
            35                  40                  45

Lys Gly Gln Asn Val Asp Cys Asp Tyr Ala Gln Ile Gly Cys Gly Asn
            50                  55                  60

Val Thr Tyr Lys Phe Val Thr Leu His Lys Pro Lys Gln Gly Ala Asp
65                  70                  75                  80

Thr Tyr Leu Glu Leu Gly Phe Lys Asn Gly Thr Leu Ala Pro Gly Ala
                85                  90                  95

Ser Thr Gly Asn Ile Gln Leu Arg Leu His Asn Asp Asp Trp Ser Asn
            100                 105                 110

Tyr Ala Gln Ser Gly Asp Tyr Ser Phe Phe Lys Ser Asn Thr Phe Lys
            115                 120                 125

Thr Thr Lys Lys Ile Thr Leu Tyr Asp
        130                 135


<210>   15
<211>   147
<212>   PRT
<213>   Artificial Sequence

<220>
<223>   Carbohydrate binding domain

<400>   15

Asn Gly Ile Ser Val Gln Tyr Lys Ala Gly Asp Gly Gly Val Asn Ser
1               5                   10                  15

Asn Gln Ile Arg Pro Gln Leu His Ile Lys Asn Asn Gly Asn Ala Thr
            20                  25                  30

Val Asp Leu Lys Asp Val Thr Ala Arg Tyr Trp Tyr Asn Ala Lys Asn
            35                  40                  45

Lys Gly Gln Asn Phe Asp Cys Asp Tyr Ala Gln Ile Gly Cys Gly Asn
            50                  55                  60

Leu Thr His Lys Phe Val Thr Leu His Lys Pro Lys Gln Gly Ala Asp
```
41

```
                65                      70                     75                      80


        Thr Tyr Leu Glu Leu Gly Phe Lys Thr Gly Thr Leu Ser Pro Gly Ala
                        85                  90                  95


        Ser Thr Gly Asn Ile Gln Leu Arg Leu His Asn Asp Asp Trp Ser Asn
                    100                 105                 110


        Tyr Ala Gln Ser Asp Asp Tyr Ser Phe Phe Gln Ser Asn Thr Phe Lys
                    115                 120                 125


        Thr Thr Lys Lys Ile Thr Leu Tyr His Gln Gly Lys Leu Ile Trp Gly
                130                 135                 140


        Thr Glu Pro
        145


        <210>  16
        <211>  63
        <212>  PRT
        <213>  Artificial Sequence

        <220>
        <223>  Carbohydrate binding domain

        <400>  16

        Pro Gly Glu Pro Glu Pro Asp Pro Gly Glu Pro Asp Pro Thr Pro Pro
        1               5                   10                  15


        Ser Asp Gly Asp Tyr Pro Ala Trp Asp Pro Asn Thr Ile Tyr Thr Asp
                    20                  25                  30


        Glu Ile Val Tyr His Asn Gly Gln Leu Trp Gln Ala Lys Trp Trp Thr
                    35                  40                  45


        Gln Asn Gln Glu Pro Gly Asp Tyr Gly Pro Trp Glu Pro Leu Asn
            50                  55                  60


        <210>  17
        <211>  145
        <212>  PRT
        <213>  Artificial Sequence

        <220>
        <223>  Carbohydrate binding domain

        <400>  17

        Gly Asn Leu Val Val Gln Tyr Lys Val Gly Asp Thr Ser Ala Thr Asp
        1               5                   10                  15
```

Asn Gln Met Lys Pro Ser Phe Asn Ile Lys Asn Asn Gly Thr Thr Pro
            20                  25              30

Val Asn Leu Ser Gly Leu Lys Leu Arg Tyr Tyr Phe Thr Lys Asp Gly
            35                  40              45

Thr Asp Met Ser Ala Ser Ile Asp Trp Ala Gln Ile Gly Ala Ser Asn
            50                  55              60

Ile Ser Ala Ala Phe Ala Asp Phe Thr Gly Ser Asn Thr Asp Thr Tyr
65                  70              75              80

Val Glu Leu Ser Phe Ser Ala Gly Ser Ile Pro Ala Gly Gly Gln Thr
                85                  90              95

Gly Asp Ile Gln Leu Arg Met Tyr Lys Thr Asp Trp Ser Asn Phe Asn
            100                 105             110

Glu Ala Asn Asp Tyr Ser Tyr Asp Gly Ala Lys Thr Tyr Ala Asp Trp
            115                 120             125

Asn Arg Val Thr Leu His Gln Asn Gly Thr Leu Val Trp Gly Thr Thr
            130                 135             140

Pro
145


<210>   18
<211>   57
<212>   PRT
<213>   Artificial Sequence

<220>
<223>   Carbohydrate binding domain

<400>   18

Asp Pro Gly Glu Pro Asp Pro Thr Pro Pro Ser Asp Gly Glu Tyr Pro
1                   5                   10                  15

Ala Trp Asp Pro Asn Gln Ile Tyr Thr Asn Glu Ile Val Tyr His Asn
            20                  25              30

Gly Gln Leu Trp Gln Ala Lys Trp Trp Thr Gln Asn Gln Glu Pro Gly
            35                  40              45

Asp Tyr Gly Pro Trp Glu Pro Leu Asn
            50                  55


<210>   19

```
<211>  144
<212>  PRT
<213>  Artificial Sequence

<220>
<223>  Carbohydrate binding domain

<400>  19

Gly Ser Leu Val Leu Gln Tyr Arg Ala Ala Asp Thr Asn Ala Gly Asp
1                5                   10                  15


Asn Ala Ile Lys Pro His Phe Asn Ile Arg Asn Thr Gly Ala Ser Pro
            20                  25                  30


Val Asp Leu Ser Gly Val Lys Leu Arg Tyr Tyr Phe Thr Lys Asp Gly
        35                  40                  45


Ile Pro Leu Ser Phe Ala Val Asp Trp Ala Gln Val Gly Ser Pro Asn
    50                  55                  60


Val Lys Gly Thr Phe Gly Ser Ala Ser Gly Ala Gly Ala Asp Thr Tyr
65                  70                  75                  80


Leu Glu Val Ser Phe Thr Gly Ser Ile Pro Ala Gly Gly Gln Thr Gly
                85                  90                  95


Glu Ile Gln Thr Arg Ile His Lys Ser Asp Trp Ser Ser Phe Gln Glu
            100                 105                 110


Ser Gly Asp Tyr Ser Tyr Asp Pro Gly Lys Thr Tyr Ala Asp Trp Ser
            115                 120                 125


Lys Val Thr Leu Tyr Arg Asp Gly Thr Arg Val Trp Gly Val Glu Pro
        130                 135                 140


<210>  20
<211>  147
<212>  PRT
<213>  Artificial Sequence

<220>
<223>  Carbohydrate binding domain

<400>  20

Asn Ile Thr Ser Ala Val Tyr Thr Ile Ser Asn Thr Asp Pro Val Lys
1                5                   10                  15


Gln Val Ser Ala Pro Thr Phe Ser Leu Gln Ser Ala Gln Thr Val Thr
            20                  25                  30
```

44

Leu Thr Ser Ser Asp Asn Asp Ser Val Ile His Tyr Thr Thr Asp Gly
        35              40              45

Thr Pro Thr Ser Ser Ser Pro Val Tyr Thr Asn Met Thr Asp Ser Ser
        50              55              60

Val Val Thr Asn Thr Tyr Thr Ile Thr Asn Glu Thr Ser Asn Ser Pro
65              70              75              80

Val Glu Val Glu Val Glu Tyr Lys Thr Thr Val Lys Val Thr Leu Thr
            85              90              95

Asn Asn Ser Ser Thr Pro Ile Asn Gly Trp Lys Leu Ser Trp Ile Asn
        100             105             110

Asn Met Trp Thr Ala Asn Tyr Ser Ile Lys Asp Ser Ala Ile Val Lys
        115             120             125

Asp Tyr Asn Asn Ile Ile Pro Ala Asn Gly Gly Thr Gln Ile Phe Gly
        130             135             140

Phe Ser Ile
145


<210> 21
<211> 147
<212> PRT
<213> Artificial Sequence

<220>
<223> Carbohydrate binding domain

<400> 21

Gln Gly Ile Val Leu Gln Tyr Arg Thr Gly Asp Thr Asn Thr Lys Asp
1           5               10              15

Asn Ala Ile Arg Pro Glu Phe Asn Ile Lys Asn Thr Gly Asn Thr Ala
        20              25              30

Val Lys Leu Ser Asp Leu Lys Ile Arg Tyr Tyr Tyr Thr Asp Glu Ser
        35              40              45

Lys Gly Gln Gln Leu Phe Val Asp Trp Ala Lys Val Gly Asn Glu Lys
        50              55              60

Val Lys Ala Thr Phe Val Ala Leu Pro Glu Pro Lys Ala Lys Ala Asp
65              70              75              80

Lys Tyr Val Glu Ile Ser Phe Thr Asp Gly Thr Ile Gln Pro Gly Gly

45

```
                            85                      90                      95

        Glu Ser Gly Glu Ile Gln Pro Arg Ile His Ala Ala Asn Trp Ser Asn
                    100                 105                 110

        Phe Asp Glu Thr Asn Asp Tyr Ser Tyr Gly Ala Ser Gln Thr Phe Ala
                    115                 120                 125

        Asn Trp Asp His Ala Thr Val Tyr Gln Gln Gly Lys Leu Val Trp Gly
                    130                 135                 140

        Ile Glu Pro
        145


        <210>   22
        <211>   147
        <212>   PRT
        <213>   Artificial Sequence

        <220>
        <223>   Carbohydrate binding domain

        <400>   22

        Glu Gly Ile Val Leu Gln Tyr Arg Thr Asp Asp Thr Asn Ala Lys Asp
        1               5               10                  15

        Asn Ala Ile Arg Pro Gln Phe Asn Ile Lys Asn Thr Gly Lys Thr Ala
                    20                  25                  30

        Val Lys Leu Ser Asp Leu Lys Ile Arg Tyr Tyr Tyr Thr Asp Glu Ser
                    35                  40                  45

        Lys Ala Gln Gln Phe Phe Val Asp Trp Ala Lys Ile Gly Asn Glu Lys
                    50                  55                  60

        Val Lys Ala Thr Phe Val Thr Leu Pro Asn Pro Lys Ser Lys Ala Asp
        65                  70                  75                  80

        Lys Tyr Val Glu Ile Ser Phe Thr Asp Gly Thr Ile Gln Pro Gly Gly
                    85                  90                  95

        Glu Thr Gly Glu Ile Gln Ser Arg Ile His Ala Ala Asn Trp Ser Asn
                    100                 105                 110

        Phe Asp Glu Thr Asn Asp Tyr Ser Tyr Gly Ala Ala Gln Thr Phe Ala
                    115                 120                 125

        Asp Trp Asp His Gly Thr Val Tyr Gln Gln Gly Lys Leu Val Trp Gly
                    130                 135                 140
```

46

Ile Glu Pro
145

<210> 23
<211> 107
<212> PRT
<213> Artificial Sequence

<220>
<223> Carbohydrate binding domain

<400> 23

Gln Ala Ala Gly Leu Thr Ala Thr Val Thr Lys Glu Ser Ser Trp Asp
1               5                   10                  15

Asn Gly Tyr Ser Ala Ser Val Thr Val Arg Asn Asp Thr Ser Ser Val
            20                  25                  30

Ser Gln Trp Glu Val Val Leu Thr Leu Pro Gly Gly Thr Thr Val Ala
        35                  40                  45

Gln Val Trp Asn Ala Gln His Thr Ser Ser Gly Asn Ser His Thr Phe
    50                  55                  60

Thr Gly Val Ser Trp Asn Ser Thr Ile Pro Pro Gly Gly Thr Ala Ser
65                  70                  75                  80

Phe Gly Phe Ile Ala Ser Gly Ser Gly Glu Pro Thr His Cys Thr Ile
                85                  90                  95

Asn Gly Ala Pro Cys Asp Glu Gly Ser Glu Pro
                100                 105

<210> 24
<211> 106
<212> PRT
<213> Artificial Sequence

<220>
<223> Carbohydrate binding domain

<400> 24

Ala Ala Thr Gly Cys Ser Val Thr Tyr Thr Thr Asn Ser Trp Ser Asn
1               5                   10                  15

Gly Phe Thr Ala Asn Val Thr Val Thr Asn Leu Gly Asp Ala Ile Gly
            20                  25                  30

Asn Trp Thr Leu Gly Phe Ser Phe Pro Ser Gly Gln Arg Val Thr Gln

```
                35                      40                      45


        Gly Trp Ser Ala Ile Trp Ser Gln Ser Gly Asn Ala Val Thr Ala Arg
            50                  55                  60


        Ser Glu Ser Trp Asn Gly Asn Leu Ala Thr Gly Ala Ser Thr Ser Ile
        65                  70                  75                  80


        Gly Phe Asn Gly Ser Phe Thr Gly Ser Pro Thr Ser Phe Thr Leu Asn
                        85                  90                  95


        Gly Val Pro Cys Thr Gly Ser Thr Thr Thr
                    100                 105


        <210>   25
        <211>   103
        <212>   PRT
        <213>   Artificial Sequence

        <220>
        <223>   Carbohydrate binding domain

        <400>   25

        Glu Ala Ala Ser Phe Thr Val Thr Asn Ser Trp Ser Thr Gly Tyr Gln
        1               5                   10                  15


        Gly Glu Val Thr Val Ser Asn Pro Ala Ser Ser Ile Asn Thr Trp Lys
                    20                  25                  30


        Val Gln Leu Thr Leu Pro Ala Gly Ser Thr Ile Gly Gln Ala Trp Asn
                    35                  40                  45


        Ala Thr Leu Ala Thr Gly Gly Gln Thr Phe Thr Phe Thr Pro Ala Gly
            50                  55                  60


        Trp Asn Gly Thr Ile Ala Gly Gly Ser Ala Thr Ser Phe Gly Phe Val
        65                  70                  75                  80


        Val Thr Gly Thr Gly Arg Pro Thr Ser Cys Thr Val Asn Gly Gln Ala
                        85                  90                  95


        Cys Thr Gly Leu Thr Gly Ala
                        100


        <210>   26
        <211>   107
        <212>   PRT
        <213>   Artificial Sequence

        <220>
```

<223> Carbohydrate binding domain

<400> 26

Ala Ala Thr Gly Cys His Val Asp Tyr Thr Val Thr Asn Gln Trp Gln
1               5                   10                  15

Gly Gly Phe Gln Ala Ala Val Lys Val Thr Asn Leu Gly Asp Ala Ile
            20                  25                  30

Thr Gly Trp Leu Leu Arg Phe Thr Phe Pro Ala Gly Gln Lys Val Ala
        35                  40                  45

Gln Gly Trp Asn Ala Thr Trp Ala Gln Ser Gly Ala Thr Ala Thr Ala
        50                  55                  60

Ala Asn Ala Asp Trp Asn Arg Thr Leu Thr Thr Gly Ala Thr Thr Glu
65                  70                  75                  80

Leu Gly Phe Thr Gly Thr Thr Thr Gly Val Pro Ala Ser Phe Thr Leu
                85                  90                  95

Asn Gly Val Thr Cys Thr Gly Ser Thr Thr Thr
            100                 105

<210> 27
<211> 106
<212> PRT
<213> Artificial Sequence

<220>
<223> Carbohydrate binding domain

<400> 27

Ala Ala Thr Gly Cys Ala Val Thr Tyr Thr Thr Asn Ser Trp Gln Gly
1               5                   10                  15

Gly Phe Thr Ala Thr Val Ala Val Arg Asn Leu Gly Asp Pro Val Ser
            20                  25                  30

Asn Trp Thr Leu Gly Phe Thr Phe Pro Gly Gly Gln Arg Val Val Gln
        35                  40                  45

Gly Trp Ser Ala Thr Trp Gln Gln Ser Gly Ser Ala Val Thr Ala Arg
        50                  55                  60

Ser Leu Asp Tyr Asn Gly Ala Leu Gly Thr Gly Ala Ser Thr Thr Ile
65                  70                  75                  80

Gly Phe Asn Gly Ser Trp Thr Gly Ser Pro Thr Ser Phe Thr Leu Asn

```
                    85                  90                  95


        Gly Thr Val Cys Thr Gly Gly Thr Ser Thr
                    100                 105



<210>   28
<211>   106
<212>   PRT
<213>   Artificial Sequence

<220>
<223>   Carbohydrate binding domain

<400>   28

Ala Ala Thr Gly Cys Ala Val Thr Tyr Thr Thr Asn Ser Trp Gln Gly
1               5                   10                  15


Gly Phe Thr Ala Thr Val Ala Val Arg Asn Leu Gly Asp Pro Val Ser
            20                  25                  30


Asn Trp Thr Leu Gly Phe Thr Phe Pro Gly Gly Gln Arg Val Val Gln
            35                  40                  45


Gly Trp Ser Ala Thr Trp Gln Gln Ser Gly Ser Ala Val Thr Ala Arg
        50                  55                  60


Ser Leu Asp Tyr Asn Gly Ala Leu Gly Thr Gly Ala Ser Thr Thr Ile
65              70                  75                  80


Gly Phe Asn Gly Ser Trp Thr Gly Ser Pro Thr Ser Phe Thr Leu Asn
                85                  90                  95


Gly Thr Val Cys Thr Gly Gly Thr Ser Thr
                100                 105


<210>   29
<211>   100
<212>   PRT
<213>   Artificial Sequence

<220>
<223>   Carbohydrate binding domain

<400>   29

Glu Ser Glu Asn Ala Thr Ile Ser Arg Gly Val Val Glu Ala Asn His
1               5                   10                  15


Thr Gly Phe Thr Gly Ser Gly Phe Val Asn Tyr Asp Asn Val Thr Gly
            20                  25                  30
```

```
Ser Tyr Val Glu Tyr Thr Val Asn Ala Ala Gln Ala Gly Pro His Thr
        35                  40                  45

Leu Thr Phe Arg Tyr Ala Asn Gly Thr Thr Ala Asn Arg Pro Leu Asp
        50                  55                  60

Ile Thr Val Asn Gly Ser Ile Ala Val Asp Asp Leu Gly Phe Ala Gly
65                  70                  75                  80

Thr Gly Ala Trp Ser Thr Val Thr Thr Thr Val Asn Leu Ala Ala Gly
                85                  90                  95

Ser Asn Lys Ile
            100


<210>  30
<211>  107
<212>  PRT
<213>  Artificial Sequence

<220>
<223>  Carbohydrate binding domain

<400>  30

Asn Ala Ala Gly Cys Arg Val Asp Tyr Thr Val Thr Asn Gln Trp Gln
1               5                   10                  15

Gly Gly Phe Gln Ala Gly Val Lys Ile Thr Asn Leu Gly Asp Thr Val
            20                  25                  30

Arg Gly Trp Thr Leu Lys Phe Thr Leu Pro Thr Gly Gln Lys Val Val
            35                  40                  45

Gln Gly Trp Ser Ala Ala Trp Ser Gln Ser Gly Ser Thr Val Thr Val
        50                  55                  60

Ala Gly Ala Asp Trp Asn Gly Thr Leu Ala Thr Gly Ala Ser Ala Asp
65                  70                  75                  80

Thr Gly Phe Val Gly Ser Phe Thr Gly Lys Pro Thr Ala Phe Thr Leu
                85                  90                  95

Asn Gly Val Ala Cys Thr Gly Ser Val Asp Asp
            100                 105


<210>  31
<211>  105
<212>  PRT
<213>  Artificial Sequence
```

<220>
<223>   Carbohydrate binding domain

<400>   31

Ala Ala Thr Gly Cys Lys Ala Glu Tyr Thr Ile Thr Ser Gln Trp Glu
1               5                   10                  15

Gly Gly Phe Gln Ala Gly Val Lys Ile Thr Asn Leu Gly Asp Pro Val
            20                  25                  30

Ser Gly Trp Thr Leu Gly Phe Thr Met Pro Ala Gly Gln Arg Leu Val
            35                  40                  45

Gln Gly Trp Asn Ala Thr Trp Ser Gln Ser Gly Ser Ala Val Thr Ala
            50                  55                  60

Gly Gly Val Asp Trp Asn Arg Thr Leu Ala Thr Gly Ala Ser Ala Asp
65                  70                  75                  80

Leu Gly Phe Val Gly Ser Phe Thr Gly Ala Pro Thr Ser Phe Thr Leu
                85                  90                  95

Asn Gly Ala Thr Cys Ser Gly Ser Val
                100                 105


<210>   32
<211>   81
<212>   PRT
<213>   Artificial Sequence

<220>
<223>   Carbohydrate binding domain

<400>   32

Asp Gly Ile Gln Thr Glu Ser Thr Thr Asp Thr Gly Gly Gly Leu Asn
1               5                   10                  15

Ile Gly Trp Thr Asp Ala Gly Asp Trp Thr Ser Pro Ala Ala Gly Arg
            20                  25                  30

Tyr Lys Val Ser Tyr Arg Asn Ser Gly Met Leu Gln Leu Glu Ala Ala
            35                  40                  45

Gly Gly Phe Pro Thr Tyr Gly Ser Ile Thr Gly Gly Trp Gln Ser Trp
            50                  55                  60

Gln Thr Ile Ser His Glu Val Asn Leu Asn Trp Ile Lys Val Glu Pro
65                  70                  75                  80

Ala

<210> 33
<211> 81
<212> PRT
<213> Artificial Sequence

<220>
<223> Carbohydrate binding domain

<400> 33

Pro Thr Glu Pro Thr Asn Pro Gly Asn Gly Thr Thr Gly Asp Val Val
1               5                   10                  15

Leu Gln Tyr Arg Asn Val Asp Asn Asn Pro Ser Asp Asp Ala Ile Arg
            20                  25                  30

Met Ala Val Asn Ile Lys Asn Thr Gly Ser Thr Pro Ile Lys Leu Ser
            35                  40                  45

Asp Leu Gln Val Arg Tyr Tyr Phe His Asp Asp Gly Lys Pro Gly Ala
        50                  55                  60

Asn Leu Phe Val Asp Trp Ala Asn Val Gly Pro Asn Asn Ile Val Thr
65                  70                  75                  80

Ser

<210> 34
<211> 81
<212> PRT
<213> Artificial Sequence

<220>
<223> Carbohydrate binding domain

<400> 34

Pro Thr Glu Pro Thr Asn Pro Gly Asn Gly Thr Thr Gly Asp Ile Val
1               5                   10                  15

Leu Gln Tyr Arg Asn Val Asp Asn Asn Pro Ser Asp Asp Ala Ile Arg
            20                  25                  30

Met Ala Phe Asn Ile Lys Asn Thr Gly Ser Thr Pro Ile Lys Leu Ser
            35                  40                  45

Asp Leu Gln Val Arg Tyr Tyr Phe His Asp Asp Gly Lys Pro Gly Ala
        50                  55                  60

53

```
Asn Leu Phe Val Asp Trp Ala Asn Val Gly Pro Asn Asn Ile Val Thr
65                  70                  75                  80

Ser
```

## Description

### FIELD OF THE INVENTION

The present invention relates to cellulose composite materials comprising type I cellulose fibers and a carbon material selected from the group consisting of carbon nanotubes, carbon nanofibers, graphene, graphene oxide and a mixture thereof. The invention further relates to conductive paint compositions comprising said composite materials.

### BACKGROUND

Cellulose is the most available biopolymer on earth and the primary reinforcement component in the cell wall of plants. Each cellulose fiber is formed by the union of fibrils, which are long thread-like bundle of molecules laterally stabilized by intermolecular hydrogen bonds. Each fibril can be considered as a string of cellulose crystals linked along the fibril axis by disordered amorphous domains. Cellulose is a semi-crystalline polymer comprising crystalline and amorphous regions. Crystalline domains are very packed together and are very difficult to degrade by either chemical or enzymatic treatment. The amorphous regions are easier to degrade. The proportion between the amorphous and the crystalline regions depends on the cellulose source and on the treatment used to extract the fibers. The main portion of cellulose is constituted by crystallites with interspersed amorphous regions, called native cellulose or cellulose type I, whereas cellulose type II, III, and IV are amorphous. The native and most abundant form of cellulose is type I, and all polymorphs can be produced from it with different physicochemical treatments. Cellulose I shows crystalline structures cellulose type I$\alpha$ and I$\beta$, its proportion depending on the origin of cellulose; thus, algae and bacterial cellulose comprises mainly cellulose type I$\alpha$ whereas cellulose type I$\beta$ is mainly present in the cell wall of superior plants and tunicates. Cellulose II is the second most abundant cellulose form; in this polymorph, cellulose chains have an antiparallel organization. This distribution allows hydrogen bonds to form between neighboring hydroxyl groups which improves the interlayer attraction forces, but there are less secondary hydrogen bonds. These interactions provide cellulose II with added stability and are responsible for the irreversibility in the conversion convert cellulose II into I. Also, cellulose II is more thermostable and mechanically weaker than cellulose I because of its different chain organization. Cellulose II can be prepared from cellulose I by mercerization (alkaline treatment) or regeneration. From cellulose I and II the other polymorphs can be produced. Cellulose III is made from cellulose I or cellulose II by ammonia treatment that penetrates and degrades the cellulose crystal structure. Cellulose IV is produced from cellulose III by glycerol and heat treatment at 260 °C. Cellulose III can be transformed into their previous polymorphs by alkaline treatment. With the use of appropriate methods, the size of cellulose can be reduced up to the nanoscale.

Currently, there are different methods for nanocellulose production: mechanical, chemical, enzymatic treatments or a combination of them. Mechanical treatments consist on high-pressure homogenization cycles that generally generate cellulose nanofibers (Iwamoto, S. et al., Appl. Phys. A 220 89; 461-466). This method needs numerous repeating steps and chemicals or enzymatic treatments (Henriksson, M. et al., Eur. Polym. J. 2007, 43: 3434-3441) are usually needed in order to reduce energy consumption. Chemical treatments are the main procedure for cellulose nanocrystals isolation. Sulfuric acid hydrolysis is the common reagent that swells cellulose amorphous regions keeping the crystalline part. During this process, sulfate groups attach to the cellulose nanocrystals surface by an esterification reaction. Sulfates stabilize nanocrystals in water suspension but have other effects in the cellulose nanocrystals physicochemical properties such as a decrease in crystallinity and thermal stability. Moreover, this method produces large amounts of residual wastewaters after the neutralization and dialysis steps, making it very pollutant. Enzymatic treatment is the preferred path for biomass hydrolysis and it also represents an ideal alternative for nanocellulose production. Despite the fact that enzymatic hydrolysis has been used in combination with mechanical and chemical treatments to improve yields (Zhu, J.Y. et al., Green Chem. 2011, 13: 1339-1344; Satyarmurthy, P. et al., Carbohydr. Polym. 2011, 83: 122-129), nanocellulose isolation using only enzymes remains largely unexplored, with only one notable example utilizing a cellulase enzyme complex from *Caldicellulosiruptor bescii* (Yarbrough, J. M. et al., Acs Nano 2017, 11: 3101-3109). Commercial fungal cellulases cocktails are optimized for sugar production and are not suitable for nanocellulose obtention. Nevertheless, typical fungal enzymes used in biomass treatment have shown limitations in the production of cellulose nanocrystals (Yarbrough, J. M. et al., Acs Nano 2017, 11: 3101-3109). Therefore, the development of highly efficient enzymes suited for nanocellulose production as well as the optimization of the obtention process are still needed for the enzymatic production of nanocellulose.

Nanocellulose is a novel light-weight and low priced biomaterial that has extraordinary features such as biocompatibility, renewability, sustainability, flexibility, as well as electrical, thermal and mechanical features.

Numerous applications have been proposed for nanocellulose, ranging from biomedicine to electronics. These applications are determined by the type of nanocellulose used which depends on the size, shape, crystallinity, and its source. Composites based on nanocellulose are being used for coatings, pharmaceuticals, laminates, textile, optical films, and smart materials, among others. Cellulose has a hierarchical organization from where different types of nanocellulose can be derived. The three main types of nanocellulose are cellulose nanofibers, cellulose nanocrystals, and bacterial cellulose. In the context of the present invention, unless otherwise stated, nanocellulose is a general term comprising cellulose nanofibers, cellulose nanocrystals or a mixture of both.

However, the terms nanofibrillated cellulose, nanofibrillar cellulose, nanofibers, nanofibrils, cellulose nanofibrils, and cellulose nanofibers seem to be used interchangeably in the literature, including as synonyms of microfibrillated cellulose, microfibers, and microfibrils (MFC). Cellulose nanocrystals (cellulose nanocrystals, also called nanowhiskers) are needlelike crystalline particles with lengths of hundreds of nanometers depending on their source and isolation treatment.

Cellulose nanofibers are thin fibers longer than cellulose nanocrystals with lengths of up to several micrometers, although cellulose nanocrystals have higher mechanical stability in comparison with cellulose nanofibers due to their crystalline structure. Cellulose nanocrystals have high surface areas and tensile strength that can be compared with other materials such as Kevlar or carbon nanotubes.

Carbon conductive materials add a great value to cellulose composite materials. The best example being graphene, two-dimensional honeycomb structure comprising tightly packed $sp^2$ carbon atoms. Graphene has a high surface area, good mechanical strength, high carrier mobility at room temperature, quantum hall effect, increased electrical and thermal conductivity, optical transparency and excellent thermal conductivity. Graphene Oxide (GO), an oxidized functional derivative of graphene, contains hydroxyl, ether, carboxyl and carbonyl functional groups. A. Kafy et al. (Sensors and Actuators A 247, 2016, 221-226) reports on a cellulose nanocrystals/GO composite film, wherein the cellulose nanocrystals was obtained via $H_2SO_4$ hydrolysis, and its use as a humidity sensor.

However, there is a need for nanocellulose/graphene composite materials having high conductive and improved mechanical and thermal properties.

## SUMMARY OF THE INVENTION

The inventors have found that cellulose nanofibers and cellulose nanocrystals produced by hydrolysis with an ancestral endoglucanase reconstructed from bacteria species mixed well with different forms of graphene and have also successfully transferred graphene layers on top of the cellulose fibers of the invention, achieving hybrid conductive biomaterials including materials in the form of layered films with conductive properties. These materials, that showed high conductive, mechanical and thermal properties, could not be prepared when using commercially available AcCNC.

Accordingly, in a first aspect, the invention is directed to a cellulose composite material comprising:

a. Type I cellulose fibers selected from the group consisting of cellulose nanocrystals, cellulose nanofibers and a mixture thereof; and
b. A carbon material selected from the group consisting of carbon nanotubes, carbon nanofibers, graphene, graphene oxide and a mixture thereof.

The material of the invention can be prepared in a variety of different formats, depending on the intended use.

In this way, a second aspect of the invention is a method for the preparation of the cellulose composite material of the invention, comprising the steps of:

a. preparing a suspension comprising type I cellulose fibers selected from the group consisting of cellulose nanocrystals, cellulose nanofibers and a mixture thereof, and a carbon material selected from the group consisting of carbon nanotubes, carbon nanofibers, graphene, graphene oxide and a mixture thereof;
b. mixing the suspension; and
c. optionally reducing said carbon material in the mixture of the previous step.

A third aspect of the invention is a method for the preparation of the cellulose composite material of the invention, comprising the step of:

a. transferring at least one layer of a carbon material selected from the group consisting of carbon nanotubes, carbon nanofibers, graphene, graphene oxide and a mixture thereof onto a matrix of type I cellulose fibers selected from the group consisting of cellulose nanocrystals, cellulose nanofibers and a mixture thereof to form a layered composite material.

In a fourth aspect, the invention is directed at a cellulose composite material obtainable by the method according to any of the second or third aspects of the invention.

The cellulose composite material of the invention has several advantageous properties, especially in terms of stability, flexibility and mechanical strength, electric conductivity and cost. Thus, in a fifth aspect, the invention is directed to the use of the cellulose composite material of the invention in dyes, including conductive dyes or paints, electronics, optoelectronics, transistors, touch screens, medicine, biomedicine, tissue engineering, health diagnostics, sensors and biosensors, food security, packaging, textiles, including electronic textiles, labels, radio frequency tags, automotive industry, energy industry including production and storage and construction materials.

Finally, a particularly interesting application for the cellulose composite material of the invention is its use in paints of different viscosities for the production of 3D printed conductive scaffolds or inks.

Therefore, a sixth and final aspect of the invention relates to a conductive paint composition, comprising:

a. the cellulose composite material of the invention;
b. a polymer binder; and
c. a carrier solvent.

## DESCRIPTION OF THE DRAWINGS

These and other characteristics and advantages of the invention will become clearly understood in view of the detailed description of the invention which becomes apparent from a preferred embodiment of the invention, given just as an example and not being limited thereto, with reference to the drawings.

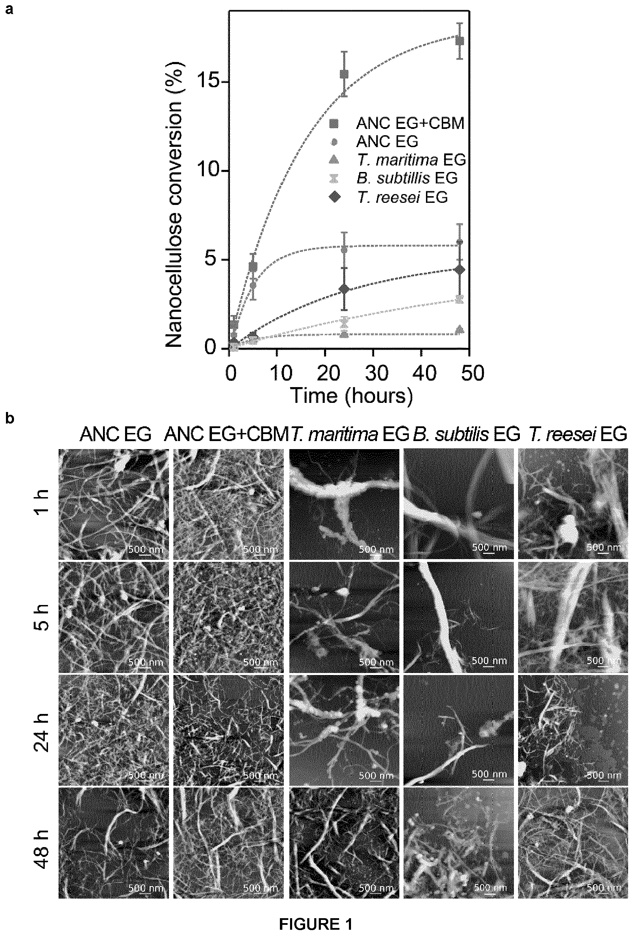

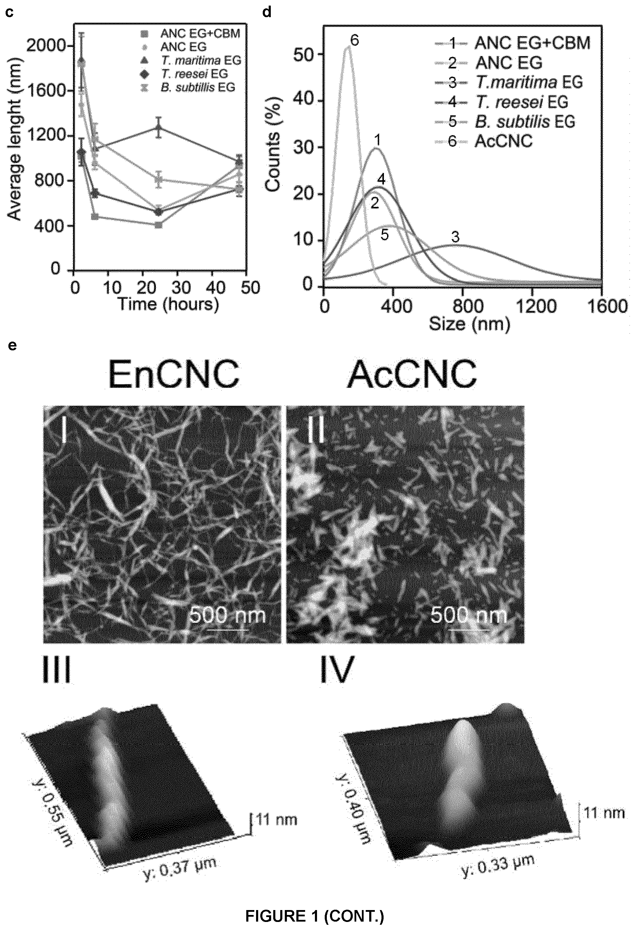

**Figure 1. Nanocellulose yields from enzymatic hydrolysis, average particle length and AFM images.** (a) Nanocellulose conversion from filter paper using ANC EG, ANC EG+CBM and EG from *T. maritima, B. subtillis* and *T. reesei* at 50°C at different times. ANC EG+CBM had around three times the activity of ANC EG and even bigger differences respect to modern enzymes. The average yield and S.D. were calculated from three independent experiments. (b) AFM images of nanoparticles produced by EG hydrolysis at different time points (5 $\mu$m x 5 $\mu$m). The images show the nanoparticles that the EGs produced at 1, 5, 24, and 48 hours using filter paper as a substrate. The enzymatic hydrolysis produced both cellulose nanofibers and cellulose nanocrystals depending on the enzyme and the time of the process. The scale bar represents 500 nm in all images. (c) Average size of nanocellulose from filter paper using ANC EG, ANC EG+CBM and EG from *T. maritima, B. subtillis* and *T. reesei* at 50°C at different times. The smaller length was measured after 24 hours ANC EG+CBM hydrolysis. The average yield and S.D. were calculated from three independent experiments. (d) Comparison of size distribution of nanocellulose produced with enzymatic hydrolysis and AcCNC. Acid hydrolysis produces more homogeneous suspension of nanocellulose than the enzymatic treatment, and from all EGs, ANC EG+CBM produced the most homogeneous population. The distribution was calculated with the length of 100 particles from each condition. (e) AFM images of nanoparticles produced with ANC EG+CBM hydrolysis and AcCNC (3 $\mu$m x 3 $\mu$m). Height images (I) of nanocellulose produced by ancestral endoglucanase with CBM at 24 hours hydrolysis using filter paper as substrate and (II) of nanocellulose produced by sulfuric acid hydrolysis purchased from Maine University. (III) A 3D image of a single EnCNC, showing the needle-like shape and (IV) a 3D image from the AcCNC sample with ribbon-like shape.

**Figure 2. Size distribution** of nanocellulose particles produced with the (a) ANC EG, (b) ANC EG+CBM, (c) *T. maritima* EG (d) *B.subtillis* EG (e) *T. reesei* EG using filter paper as a substrate at different time points. The figure shows the smaller and more homogeneous size of the nanoparticle's population using ANC EG+CBM. The distribution was calculated with the length of 100 particles from each condition.

**Figure 3. Nanocellulose physicochemical characterization.** Comparison of FTIR spectra from 4000 to 650 cm$^{-1}$ of filter paper, AcCNC and nanocellulose produced by ANC EG and ANC EG+CBM hydrolysis at 24 hours. (a) Spectra from 4000 to 650 cm$^{-1}$. (b) amplification from 1800 to 650 cm$^{-1}$ and (c) from 800 to 650 cm$^{-1}$. In the figure it can be observed that filter paper produces a similar spectra to that of EnCNC, while the AcCNC spectrum is remarkably different.

**Figure 4.** FTIR spectra of nanocellulose produced by ANC EG hydrolysis of filter paper at 1, 5, 24, 48 and 72 hours. (a) Spectra from 4000 to 650 cm$^{-1}$, (b) an amplification from 1800 to 650 cm$^{-1}$, and (c) from 800 to 650 cm$^{-1}$. In the figure it can be observed that ANC EG did not produce any chemical changes with regards to the filter paper.

**Figure 5.** FTIR spectra of nanocellulose produced by ANC EG+CBM hydrolysis of filter paper at 1, 5, 24, 48 and 72 hours. (a) Spectra from 4000 to 650 cm$^{-1}$, (b) an amplification from 1800 to 650 cm$^{-1}$ and (c) from 800 to 650 cm$^{-1}$. In the figure it can be observed that ANC EG+CBM did not produce any chemical changes with regards to the filter paper.

**Figure 6.** Second derivative FTIR spectra of filter paper, EnCNC from hydrolysis with both ANC EGs and AcCNC, (a) between 3000-2850 cm$^{-1}$, (b) between 1500-1100 cm$^{-1}$ and (c) from 1000-750 cm$^{-1}$.

**Figure 7.** Aqueous suspension of EnCNC and AcCNC. The high charged surface of AcCNC improves the dispersion in water while the EnCNC was less stable. Illustration of free -OH groups in the EnCNC surface and the random -OSO$_3^-$ groups anchored by the sulfuric acid treatment to the AcCNC surface.

**Figure 8.** (a) Nano-FTIR spectra of nanocellulose particles produced after 5 hours hydrolysis of ANC EG+CBM and of a commercially available AcCNC suspension. In the AFM images the circles mark the regions selected for the measurements. (b) Spectra from 3600 to 3200 cm$^{-1}$ and (c) from 1800 to 650 cm$^{-1}$.

**Figure 9.** X-ray diffraction analysis of the filter paper, EnCNC produced by 24 hours hydrolysis by ANC EG and ANC EG+CBM and AcCNC. Figure shows the diffractograms of cellulose I in the filter paper and EnCNC, and in the case of AcCNC the diffractogram corresponds to the cellulose I and II pattern.

**Figure 10.** CP/MAS$^{13}$C NMR spectra of (from bottom to top) the filter paper, the EnCNC produced by 24 hours hydrolysis with ANC EG, the EnCNC produced by 24 hours hydrolysis with ANC EG+CBM, and AcCNC. The figure shows the spectra of cellulose I in the case of filter paper (bottom) and EnCNC (two of the middle), and a mixture of cellulose I and cellulose II in the case of AcCNC spectrum (top spectrum).

**Figure 11.** Thermogravimetric analysis curves of filter paper, EnCNC produced by 24 hours hydrolysis by ANC EG and ANC EG+CBM and AcCNC. Weight loss curves (a) and derivative (b) from TGA curves. The curves showed higher thermal stability of EnCNC compared to AcCNC.

**Figure 12.** Photographs of EnCNC and AcCNC films. The EnCNC film was transparent and had good integrity. The AcCNC was also transparent but significantly weaker.

**Figure 13.** AFM images of graphene oxide suspension. (a) Height images of (10 μm x10 μm). (b) Phase images of (10 μm x10 μm). (c) Height images of (3 μm x3 μm). (d) Phase images of (3 μm x 3 μm). Graphene sheets have a size around 1 μm and they tend to stack to each other.

**Figure 14.** SEM images of films prepared with EnCNC and EnCNC with different graphene content: 2, 5, and 10 wt%. The film surface was rougher with the increment of rGO.

**Figure 15.** Raman spectroscopy from EnCNC (1) and EnCNC with 2% rGO (2), 5% rGO (3) and with 10% rGO (4) contents. Typical D and G band from graphene could be seen in the spectra of nanopapers with rGO, confirming graphene addition.

**Figure 16.** Comparison of FTIR spectra of GO (1), rGO (2), EnCNC (3) and EnCNC with 2% rGO (4), 5% rGO (5) and with 10% rGO (6) contents. Spectra were measured from 4000 to 900 cm$^{-1}$.

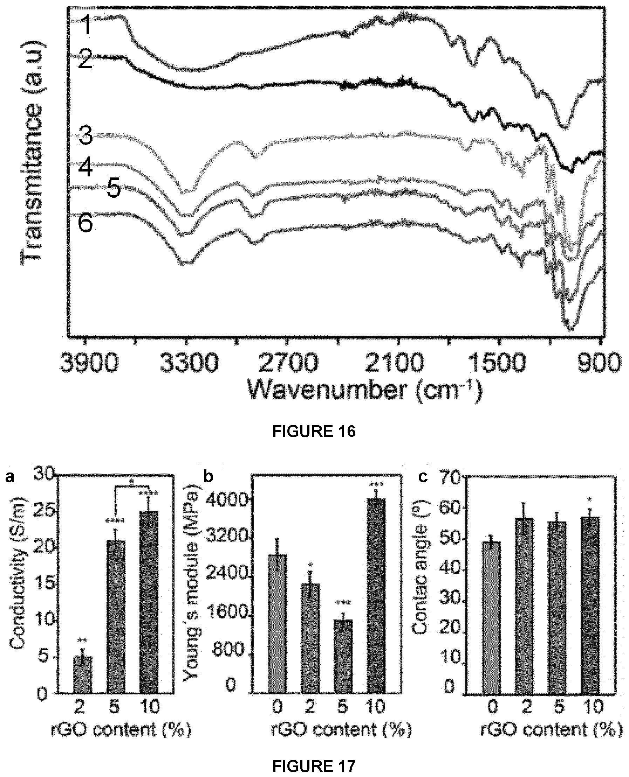

**Figure 17.** (a) Conductive properties of EnCNC films with different rGO content. The conductivity of the nanopapers increased with rGO content due to the better contact between graphene nanosheets. The average values and S.D. were calculated from five independent experiments (*P < 0.05; **P < 0.01; ***P < 0.001 ****P < 0.0001). (b). Young's Modulus of the EnCNC film and EnCNC films at different rGO concentrations. Young modulus of the EnCNC films decreased with 2 and 5 wt% rGO content, but increased with 10 wt% rGO content. The average values and S.D. were calculated from five independent experiments (*P < 0.05; **P < 0.01; ***P < 0.001 ****P < 0.0001). (c). Water contact angle (°) measurement for neat EnCNC and nanocomposites with different rGO content. Contact angle values increased when rGO content in the film increased. The average values and S.D. were calculated from five independent experiments (*P < 0.05; **P < 0.01; ***P < 0.001 ****P < 0.0001).

**Figure 18.** Weight loss curves of EnCNC film and the EnCNC films with different rGO content.

**Figure 19. CVD graphene over nanocellulose film.** Raman spectroscopy from EnCNC, EnCNC ML and EnCNC BL. The typical G and 2D bands from graphene were present in the EnCNC ML and EnCNC BL samples suggesting a successfully transfer over the film.

**Figure 20.** UV-Vis transmittance measurements of EnCNC, EnCNC ML and EnCNC BL. The monolayer and bilayer deposition over the EnCNC film produced a slightly drop in the transmittance.

**Figure 21.** Sheet resistance measurement of EnCNC ML and EnCNC BL. The average values and S.D. were calculated from two independent experiments (*P < 0.05; **P < 0.01; ***P < 0.001 ****P < 0.0001).

**Figure 22.** EFM height and phase images of EnCNC, EnCNC ML and EnCNC BL. Topography of the films shown in the height images and the phase showed the response of the films to different voltage vias 0, 2, 4 and 6 V, from the AFM tip to the sample.

**Figure 23.** EFM phase images from EnCNC ML and EnCNC BL applying constant voltage -5, 0 and 5 V. The images shown the response of the surface to the voltage and the conductivity was mostly uniform in the film.

**Figure 24.** Topography profile from AFM images of EnCNC, EnCNC ML and EnCNC BL.

## DETAILED DESCRIPTION OF THE INVENTION

Unless defined otherwise, all technical and scientific terms used herein have the same meaning as commonly understood to one of ordinary skill in the art to which this disclosure belongs. The skilled person readily understands that the sum of percentage values cannot be greater than 100%. For this reason, if any combination of percentage values, given in ranges, adds up to a value which is greater than 100%, then the maximum values of the ranges are to be interpreted as limited to the maximum value possible, as long as the sum is 100%.

The inventors have developed a cellulose composite material that is surprisingly stable, in particular thermally stable and stable in aqueous medium, and has conductive properties.

The term "cellulose" as used herein, refers to an organic compound with CAS number 9004-34-6, a polysaccharide consisting of a linear chain of several hundred to many thousands of $\beta(1\rightarrow4)$ linked D-glucose units. Type I cellulose is known in the art as native cellulose.

The term "type I cellulose", as used herein, refers to the native and most abundant form of cellulose. In type I cellulose has the chains of the polymer in a parallel organization. Type I cellulose can also be organized in two polymorphs, cellulose I$\alpha$ and I$\beta$. These two type I polymorphs can be found together, and the ratio between them varies with the cellulose source. Cellulose I$\alpha$ is abundant in algae and bacterial cellulose and cellulose I$\beta$ is more present in higher plants and tunicates. Cellulose II is the second most abundant cellulose form; in this polymorph, cellulose chains have an antiparallel organization. Cellulose I can be distinguished from the other cellulose polymorphs by any suitable technique, for example from FTIR spectra in the spectral region comprised from 800 to 650 cm$^{-1}$.

Accordingly, in a first aspect, the invention is directed to a cellulose composite material comprising:

a. Type I cellulose fibers selected from the group consisting of cellulose nanocrystals, cellulose nanofibers and a mixture thereof; and
b. A carbon material selected from the group consisting of carbon nanotubes, carbon nanofibers, graphene, graphene oxide and a mixture thereof.

Cellulose composite material

In the context of the present invention, the term "cellulose fibers", used interchangeably with "nanocellulose", refers to cellulosic materials and, unless stated otherwise, is used to refer to either cellulose nanofibers, cellulose nanocrystals, or to a mixture of both. In the context of this invention, the broader *genus* "cellulose fibers" is not synonym to the narrower *species* "cellulose nanofibers".

The cellulose fibers of the invention, which are selected from the group consisting of cellulose nanocrystals, cellulose nanofibers and a mixture thereof, have at least one dimension in the nanometer range. Preferably, the cellulose fibers of the invention have at least their diameter in the nanometer range.

Cellulose nanocrystals have normally an average diameter (also identified as width) between 2-60 nm and a length of 10 nm to 2 $\mu$m, depending on the cellulose source and the isolation treatment. Typically, cellulose nanofibers have lengths of from 1 $\mu$m to 10 $\mu$m and diameters (also identified as widths) of from 5 nm to 60 nm. Cellulose nanofibers differ with regards to cellulose nanocrystals in at least the length to width aspect ratio (length/diameter).

The diameter and length of the cellulose nanocrystals and/or cellulose nanofibers can be calculated using any suitable technique known by the skilled person, for example, by atomic force microscopy (AFM), as detailed in the examples herein included.

In the context of the present invention, the term "carbon material" is to be interpreted, unless stated otherwise, as a group consisting of carbon nanotubes, carbon nanofibers, graphene, graphene oxide and a mixture thereof. These materials have remarkable electrical conductivity, tensile strength and thermal conductivity. The term "carbon nanofibers" is synonym to "vapor grown carbon fibers" or "vapor grown carbon nanofibers" and refers to cylindrical nanostructures (at least one dimension in the nanometer range) with graphene layers arranged as stacked cones, cups or plates. "Carbon nanofibers" differ from what is termed as "carbon nanotubes" in that carbon nanotubes are characterized by graphene layers wrapped into perfect cylinders. Carbon nanotubes encompasses single-wall carbon nanotubes (SWCNTs) with diameters in the range of a nanometer but can also refer to multi-wall carbon nanotubes (MWCNTs) consisting of nested single-wall carbon nanotubes. The carbon nanotube may be one or more selected from the group consisting of single-walled, double-walled, thin multi-walled, multi-walled, and roped types. The term "graphene" is to be interpreted as an allotrope of carbon in the form of a single layer of atoms in a two-dimensional hexagonal lattice, characterized by tightly packed carbon atoms and a sp$^2$ orbital hybridization. Graphene is a zero-gap semiconductor and displays remarkable electron mobility at room temperature, with reported values in excess of 15000 cm$^2$ x V$^{-1}$ x s$^{-1}$. The term "graphene oxide" refers to the oxidized form of graphene, and it can be obtainable by techniques known to the skilled person. For example, through chemical modification of graphite in acidic medium. Graphene oxide displays higher solubility when compared to that of graphene.

In a particular embodiment of the invention, the cellulose composite material is an electrically conductive composite material.

In the context of the present invention, "electrically conductive composite material" refers to a material as an object or type of material that allows the flow of charge (electrical current) in one or more directions. Electrical current is generated by the flow of negatively charged electrons, positively charged holes, and positive or negative ions in some cases. Therefore, in a particular embodiment of the invention, the cellulose composite material of the invention is capable of conducting electricity, i.e., has electrical conductivity, or simply conductivity. Electrical conductivity (or conductivity, measured as Siemens per meter, S/m) is a fundamental property of a material that quantifies how strongly it conducts electric current.

In a particular embodiment of the invention, the cellulose composite material is characterized by a conductivity value equal to or greater than 5 S/m, measured by the electrical impedance four-terminal sensing technique.

In a particular embodiment of the invention, the cellulose fibers and the carbon material are homogeneously distributed within the composite material. Preferably, the cellulose composite material comprises at least 0.1%, at least 0.2%, at least 0.4%, at least 0.5%, at least 0.6%, at least 0.8%, at least 1%, at least 2%, at least 5%, at least 10%, at least 20%, at least 25% or at least 30% w/w of carbon material, with regards to the sum of the weights of the cellulose fibers and said carbon material.

In a particular embodiment, the cellulose fibers are free of surface functionalization.

The present invention also contemplates that the composite material is a layered material. In this particular case, the cellulose fibers selected from the group consisting of cellulose nanocrystals, cellulose nanofibers and a mixture thereof act as a support matrix onto which the carbon material is deposited.

Thus, in another particular embodiment of the invention, the composite is a layered composite material comprising a matrix of cellulose fibers and at least one layer of carbon material on top of at least a first surface of said matrix. In a preferred embodiment, the layered composite material comprises at least two layers or at least three layers of carbon material on top of at least said first surface of said matrix. In another particular embodiment, the matrix of cellulose fibers has a first and a second surface and the layered composite material has at least one layer of carbon material on top of each of the first and second surfaces of said matrix.

There are different methods for providing layered materials available to the skilled person. In a preferred embodiment, the at least one layer of said carbon material is a layer deposited by means of chemical vapor deposition. This technique provides single layers onto substrates. Thus, in a particular embodiment, the at least one layer of carbon material has an average thickness of one $sp^2$ hybridized carbon atom. The thickness can be measured by, for example, tapping mode AFM.

In a particular embodiment, the cellulose composite material of the invention is a material comprising:

a. Type I cellulose fibers selected from the group consisting of cellulose nanocrystals, cellulose nanofibers and a mixture thereof; and
b. A carbon material selected from the group consisting of carbon nanotubes, graphene, graphene oxide and a mixture thereof.

In another particular embodiment, the cellulose composite material of the invention is a material comprising:

a. Type I cellulose fibers selected from the group consisting of cellulose nanocrystals, cellulose nanofibers and a mixture thereof; and
b. A carbon material selected from the group consisting of graphene, graphene oxide and a mixture thereof.

In yet another particular embodiment, the cellulose composite material of the invention is a material comprising:

a. Type I cellulose fibers selected from the group consisting of cellulose nanocrystals, cellulose nanofibers and a mixture thereof; and
b. A carbon material consisting of graphene.

In a preferred embodiment, the cellulose composite material of the invention is a material comprising:

a. Type I cellulose fibers consisting of cellulose nanocrystals; and
b. A carbon material selected from the group consisting of graphene, graphene oxide and a mixture thereof.

In this way, preferably, the cellulose composite material of the invention is such wherein the cellulose fibers are cellulose nanocrystals and wherein the carbon material is graphene, graphene oxide or a mixture thereof.

In a most preferred embodiment, the cellulose composite material of the invention is a material comprising:

a. Type I cellulose fibers consisting of cellulose nanocrystals; and
b. A carbon material consisting of graphene.

In a particular embodiment, the type I cellulose fibers are obtained by a method comprising hydrolyzing a substrate comprising cellulose with an enzyme having endoglucanase activity, as described below.

In a particular embodiment, the substrate comprising cellulose comprises amorphous and crystalline regions. In a particular embodiment, the substrate comprises type I cellulose. Type I cellulose fibers are known in the art as native cellulose fibers.

Cellulose nanocrystals

In the context of the present invention, the term "cellulose nanocrystals" (CNC) is synonym to "crystalline nanocellulose", "crystalline cellulose", "nanocrystalline cellulose", "nanowhiskers" or "cellulose nanowhiskers". As used herein, it refers to needle-like nanoparticles consisting of cellulose chain segments with very high crystallinity.

In this way, in a particular embodiment of the invention, the cellulose nanocrystals have a length $\geq$ 10 nm, $\geq$ 20 nm, $\geq$ 30 nm, $\geq$ 40 nm, $\geq$ 50 nm, preferably $\geq$ 60 nm, more preferably $\geq$ 70 nm, even more preferably 100 nm.

In a preferred embodiment, the cellulose nanocrystals have a length $\leq$ 900 nm, preferably $\leq$ 800 nm, more preferably have a length $\leq$ 700 nm, even more preferably $\leq$ 500 nm.

In a particular embodiment of the invention, the cellulose nanocrystals have a length comprised between 10 nm and 900 nm, between 10 nm and 800 nm, between 20 nm and 800 nm, between 20 nm and 700 nm, between 30 nm and 700 nm preferably between 50 nm and 700 nm, more preferably between 50 nm and 500 nm.

In another particular embodiment of the invention, the cellulose nanocrystals have a diameter, or width, comprised between 2 nm and 60 nm, between 5 nm and 50 nm, preferably between 10 nm and 40 nm, more preferably between 15 nm and 40 nm, even more preferably between 15 nm and 30 nm. In another particular embodiment, the cellulose nanocrystals have a diameter comprised between 2 nm and 30 nm, for example between 5 nm and 28 nm, between 10 nm and 25 nm, between 15 nm and 20 nm.

The cellulose nanocrystals have a length to width aspect ratio which is calculated from the possible combinations of length and width (or diameter) ratios identified in the previous paragraphs. It is generally accepted in the art that the aspect ratio of cellulose nanocrystals is generally lower than 100. In a particular embodiment of the invention, the cellulose nanocrystals have a length to width aspect ratio comprised between 5 and 100, between 10 and 100, preferably between 20 and 100, preferably between 20 and 80, more preferably between 30 and 80, even more preferably between 30 and 70. In another particular embodiment, the cellulose nanocrystals have a length to width aspect ratio comprised between 10 and 80, preferably between 20 and 60.

In a particular embodiment, the cellulose nanocrystals have a length comprised between 10 and 2000 nm, preferably between 50 and 1000 nm, and a length to width aspect ratio comprised between 5 and 100, preferably between 20 and 100.

The cellulose nanocrystals preserve the structure of native cellulose, and therefore are type I cellulose.

In a particular embodiment, the cellulose nanocrystals of the invention have a crystallinity index is $\geq$ 70%, $\geq$ 75%, preferably $\geq$ 80%, more preferably $\geq$ 85%, even more preferably $\geq$ 87%.

The crystallinity index can be determined by any suitable technique known by the skilled person, for example, X-Ray diffraction (XRD) as detailed in the examples herein included.

From the diffractograms, the crystallinity index (CI%) can be calculated using the Segal equation:

$$\text{Crystallinity index (\%)} = (I_{200} - I_{am})/I_{200} \times 100$$

where $I_{200}$ is the intensity of the cellulose crystalline peak and $I_{am}$ is the intensity of the amorphous peak.

In a particular embodiment, the cellulose nanocrystals have a crystallite size comprised between 4.0 and 7.0 nm, for example between 4.2 and 6.5 nm, between 4.5 nm and 6 nm, between 4.8 and 5.5 nm, preferably between 4.2 nm and 6.5 nm, more preferably about 5.0 nm.

The crystallite size can be determined by any suitable method known by the skilled person, for example, by X-Ray diffraction as detailed in the examples herein included. The crystallite size can be measured from the diffractograms using the Scherrer's equation:

$$\beta = \kappa\lambda/\tau cos\theta \times 100$$

where $\lambda$ is the wavelength of the incident X-ray, $\theta$ is the angle of the (200) plane, $\beta$ is the full-width at half maximum of the (200) peak, $\tau$ is the crystallite size and $\kappa$ is a constant value.

Cellulose nanofibers

In the context of the present invention, the term "cellulose nanofibers" (CNF) is synonym to "microfibrillated cellulose", "microfibers", "microfibrils", "nanofibrillated cellulose", "nanofibrils" or "cellulose nanofibrils". Cellulose nanofibers are long thin fibers with a length of several micrometers and diameters in the nanometer range, and include both amorphous and crystalline domains.

In this way, in a particular embodiment of the invention, the cellulose nanofibers have a length > 900 nm, $\geq$ 1

$\mu$m, $\geq$ 1.5 $\mu$m, preferably $\geq$ 2 $\mu$m, more preferably $\geq$ 2.5 $\mu$m, even more preferably $\geq$ 3 $\mu$m.

In a preferred embodiment, the cellulose nanofibers have a length $\leq$ 10 $\mu$m, more preferably have a length $\leq$ 8 $\mu$m, even more preferably $\leq$ 5 $\mu$m.

In a particular embodiment of the invention, the cellulose nanofibers have a length greater than 900 nm and equal to or lower than 10 $\mu$m, preferably between 1 $\mu$m and 8 $\mu$m, between 1 $\mu$m and 7 $\mu$m, between 1 $\mu$m and 5 $\mu$m, between 1.5 $\mu$m and 5 $\mu$m, more preferably between 2 $\mu$m and 5 $\mu$m.

In another particular embodiment of the invention, the cellulose nanofibers have a diameter, or width, comprised between 2 nm and 60 nm, between 5 nm and 50 nm, preferably between 10 nm and 40 nm, more preferably between 15 nm and 40 nm, even more preferably between 15 nm and 30 nm. In another particular embodiment, the cellulose nanofibers have a diameter comprised between 2 nm and 30 nm, for example between 5 nm and 28 nm, between 10 and 25 nm, between 15 nm and 20 nm.

The cellulose nanofibers have a length to width aspect ratio which is calculated from the possible combinations of length and width (or diameter) ratios identified in the previous paragraphs. It is generally accepted that cellulose nanofibers have an aspect ratio of over 100. In a particular embodiment of the invention, the cellulose nanofibers have a length to width aspect ratio comprised between 100 and 2000, between 100 and 1500, preferably between 100 and 1000, preferably between 150 and 1000, more preferably between 200 and 1000, even more preferably between 300 and 1000. In another particular embodiment, cellulose nanofibers have a length to width aspect ratio comprised between 100 and 900, preferably between 300 and 900.

In a particular embodiment, the cellulose nanofibers have a length comprised between 1 and 10 $\mu$m, preferably between 1 and 5 $\mu$m, and a length to width aspect ratio comprised between 100 and 2000, preferably between 100 and 1000.

The cellulose nanofibers preserve the structure of native cellulose, and therefore are type I cellulose.

Methods of the invention

The cellulose composite material of the invention can be prepared according to at least two methods; one leading to the formation of a homogeneous matrix comprising the components of the composite material and a second method leading to the formation of a layered material, wherein the carbon material is a layered carbon material in contact with at least one of the surfaces of a matrix comprising the cellulose fibers.

A second aspect of the invention is a method for the preparation of the cellulose composite material of the invention (first method of the invention), comprising the steps of:

a. preparing a suspension comprising type I cellulose fibers selected from the group consisting of cellulose nanocrystals, cellulose nanofibers and a mixture thereof, and a carbon material selected from the group consisting of carbon nanotubes, carbon nanofibers, graphene, graphene oxide and a mixture thereof;
b. mixing the suspension; and
c. optionally reducing said carbon material in the mixture of the previous step.

A particular embodiment of the first method for the preparation of the cellulose composite material of the invention, comprises the steps of:

a. preparing a suspension comprising type I cellulose fibers selected from the group consisting of cellulose nanocrystals, cellulose nanofibers and a mixture thereof, and a carbon material selected from the group consisting of carbon nanotubes, carbon nanofibers, graphene, graphene oxide and a mixture thereof;
b. mixing the suspension; and
c. reducing said carbon material in the mixture of the previous step.

In a particular embodiment, step c comprises the reduction of graphene oxide to graphene. This step is preferably conducted in the presence of a reducer or in a reducing medium selected from the group consisting of ascorbic acid, hydrazine, ultraviolet light, and mixtures thereof. Preferably, the reducer is ascorbic acid. If necessary, the reduction is carried out at a temperature higher than 25 °C. The skilled person will readily understand that the temperature must not be so high that the cellulose material is affected. In a particular embodiment, the reduction is carried out at a temperature comprised between 30 and 100 °C, preferably between 80 and 95°C.

A third aspect of the invention is a method for the preparation of the cellulose composite material of the invention (second method of the invention), comprising the step of:

a. transferring at least one layer of a carbon material selected from the group consisting of carbon nanotubes, carbon nanofibers, graphene, graphene oxide and a mixture thereof onto a matrix of type I cellulose fibers selected from the group consisting of cellulose nanocrystals, cellulose nanofibers and a mixture thereof to form a layered composite material.

In a preferred embodiment of any of the methods of the invention, the cellulose fibers are prepared by hydrolyzing a substrate comprising cellulose with an enzyme having endoglucanase activity. Preferably, the enzyme having endoglucanase activity comprises an endoglucanase catalytic domain comprising a sequence selected from the group consisting of SEQ ID NO: 1, SEQ ID NO: 2, SEQ ID NO: 3 and a functionally equivalent variant thereof that substantially maintains or improves its catalytic activity.

In a particular embodiment of any of the methods of the invention, the enzyme having endoglucanase activity comprises a carbohydrate binding domain.

In another particular embodiment of any of the methods of the invention, the cellulose fibers are isolated after the enzymatic hydrolysis step.

In yet another particular embodiment of any of the methods of the invention, the cellulose fibers are dried after the enzymatic hydrolysis step.

Hydrolysis of cellulose

In a particular embodiment of the composite material of the invention, the cellulose fibers are obtained by a method comprising hydrolyzing a substrate comprising cellulose.

The term "substrate comprising cellulose", as use herein, refers to any material comprising cellulose, preferably type I cellulose. Illustrative non-limitative examples of said substrates are lignocellulose biomass (composed mainly of cellulose, hemicellulose and lignin), corn stover, *Panicum virgatum* (switchgrass), Miscanthus grass species, wood chips and the byproducts of lawn and tree maintenance. Lignocellulosic biomass can be grouped into four main categories: (1) agricultural residues (including corn stover and sugarcane bagasse), (2) dedicated energy crops, (3) wood residues (including sawmill and paper mill discards), and (4) municipal paper waste. Illustrative lignocellulosic biomass sources include, but are not limited to grasses, rice hulls, bagasse, jute, hemp, flax, bamboo, sisal, abaca, straw, corn cobs, corn stover, alfalfa, hay, coconut hair, seaweed and algae.

In a particular embodiment, the substrate comprising cellulose comprises or consists on pure cellulose. The term "pure cellulose", as used herein, refers to cellulose that has generally been separated from other plant natural products, such as hemicellulose and/or lignin and typically has a purity of 90% w/w or more, e.g. 95% w/w or more. Examples of pure cellulose substrates are filter paper or cellulose threads. In a more particular embodiment, the substrate comprising or consisting of pure cellulose is filter paper.

In a particular embodiment, the substrate comprising cellulose is a lignocellulosic substrate. The term "lignocellulosic substrate", as used herein, refers to a material, usually derived from plant biomass, which comprises cellulose, hemicellulose and lignin. The lignocellulosic material can be derived from a single material or a combination of materials and/or can be non-modified and/or modified. Lignocellulosic material can be transgenic (i.e., genetically modified). Lignocellulose is generally found, for example, in the fibers, pulp, stems, leaves, hulls, canes, husks, and/or cobs of plants or fibers, leaves, branches, bark, and/or wood of trees and/or bushes. Examples of lignocellulosic materials include, but are not limited to, agricultural biomass, e.g., farming and/or forestry material and/or residues, branches, bushes, canes, forests, grains, grasses, short rotation woody crops, herbaceous crops, and/or leaves; oil palm fiber waste such as empty fruit bunch and palm trunk; energy crops, e.g., corn, millet, and/or soybeans; energy crop residues; paper mill residues; sawmill residues; municipal paper waste; orchard prunings; Willow coppice and Mallee coppice; wood waste; wood chip, logging waste; forest thinning; short-rotation woody crops; bagasse, such as sugar cane bagasse and/or sorghum bagasse, duckweed; wheat straw; oat straw; rice straw; barley straw; rye straw; flax straw; soy hulls; rice hulls; rice straw; tobacco; corn gluten feed; oat hulls; corn kernel; fiber from kernels; corn stover; corn stalks; com cobs; corn husks; canola; miscanthus; energy cane; prairie grass; gamagrass; foxtail; sugar beet pulp; citrus fruit pulp; seed hulls; lawn clippings; cotton, seaweed; trees; shrubs; wheat; wheat straw; products and/or by-products from wet or dry milling of grains; yard waste; plant and/or tree waste products; herbaceous material and/or crops; forests; fruits; flowers; needles; logs; roots; saplings; shrubs; switch grasses; vegetables; fruit peels; vines; wheat midlings; oat hulls; hard and soft woods; or any combination thereof. In another embodiment, the lignocellulosic material may be the product obtained by a processor selected from the group consisting of a dry grind ethanol production facility, a paper pulping facility, a tree harvesting operation, a sugar cane factory, or any combination thereof.

In a particular embodiment, the lignocellulosic material is paper pulp.

The term "paper pulp" means any pulp which can be used for the production of a paper material. For example, the pulp can be supplied as a virgin pulp, or can be derived from a recycled source. The pulp may be a wood pulp, a non-wood pulp or a pulp made from waste paper. A wood pulp may be made from softwood such as pine, redwood, fir, spruce, cedar and hemlock or from hardwood such as maple, alder, birch, hickory, beech, aspen, acacia and eucalyptus. A non-wood pulp may be made, e.g., from flax, hemp, bagasse, bamboo, cotton or kenaf. A waste paper pulp may be made by re-pulping waste paper such as newspaper, mixed office waste, computer print-out, white ledger, magazines, milk cartons, paper cups etc. The wood pulp may be mechanical pulp (such as ground wood pulp, GP), chemical pulp (such as Kraft pulp or sulfite pulp), semichemical pulp (SCP), thermomechanical pulp (TMP), chemithermomechanical pulp (CTMP), or bleached chemithermomechanical pulp (BCTMP).

Mechanical pulp is manufactured by the grinding and refining methods, wherein the raw material is subjected to periodical pressure impulses. TMP is thermomechanical pulp, GW is groundwood pulp, PGW is pressurized groundwood pulp, RMP is refiner mechanical pulp; PRMP is pressurized refiner mechanical pulp and CTMP is chemithermimechanical pulp.

Chemical pulp is manufactured by alkaline cooking whereby most of the lignin and hemicellulose components are removed. In Kraft pulping or sulphate cooking sodium sulphide or sodium hydroxide are used as principal cooking chemicals.

In a particular embodiment, the lignocellulosic material is unbleached kraft pulp (KAPPA). KAPPA is a pulp rich in cellulose (about 70%) but also comprising hemicellulose (about 10%), lignin (about 12%) and other components as described by Da Silva, T.A. et al., BioResources 2007, 2(4): 616-629. In another particular embodiment, the lignocellulosic material is bleached kraft pulp (BKP). BKP comprises more cellulose (about 80%), hemicellulose (12%) and a small lignin residue (about 1%) as described by Hu, J. et al., Sci. Rep. 2018, 8(1): 3195.

Enzymatic hydrolysis

In a preferred embodiment of any of the aspects of the invention, the cellulose fibers are obtained by hydrolyzing a substrate comprising cellulose with an enzyme having endoglucanase activity. Contrary to what is obtained upon acid hydrolysis, this preferred embodiment yields cellulose fibers preserve the crystalline structure of native cellulose, that is, yields type I cellulose fibers. Contrary to what is obtained upon acid hydrolysis, in particular upon hydrolysis with $H_2SO_4$, this preferred embodiment yields cellulose fibers that have no charged functional groups in its structure.

In this way, in a particular embodiment of the cellulose composite material of the invention, the cellulose fibers have an intrinsic net charge of zero. An intrinsic net charge of zero, as used herein, means that the cellulose material is electrically neutral when at rest. The skilled person will readily understand that neutral molecules may become temporarily electrically charged when exposed to movement or external fields, such as magnetic fields.

The term "enzyme having endoglucanase activity", "enzyme comprising an endoglucanase catalytic domain", or "endoglucanase" as used herein, refers to an enzyme that randomly cleaves (1-4)-β-D-glucosidic links in cellulose, lichenin and cereal β-D-glucans, thereby creating new chain ends. Endoglucanases also hydrolyse 1,4-linkages in β-D-glucans also containing 1,3-linkages. It has been classified by the Nomenclature Committee of the International Union of Biochemistry and Molecular Biology as EC 3.2.1.4. Endoglucanases derived from bacteria have a catalytic domain and a carbohydrate binding module attached by a linker or linking domain. However, in the present invention, the term "endoglucanase" is not restricted to enzymes comprising a carbohydrate binding motive, but encompasses any enzyme comprising an endoglucanase catalytic domain, therefore having endoglucanase activity, as previously defined. Illustrative non-limitative embodiments of enzymes having endoglucanase activity include BglC (*Bacillus subtilis*), EngA (*Bacillus amyloliquefaciens*) or Egl2 (*Trichoderma reesei*).

In a particular embodiment of any of the aspects of the invention, the enzyme having endoglucanase activity comprises an endoglucanase catalytic domain comprising a sequence selected from the group consisting of SEQ ID NO: 1, SEQ ID NO: 2, SEQ ID NO: 3 and a functionally equivalent variant thereof that substantially maintains or improves its catalytic activity.

The term "endoglucanase catalytic domain", as used herein, refers to a domain of an enzyme being responsible of its catalytic function, particularly of its endoglucanase function. It contains the active site, a set of amino acids with a special spatial arrangement that permits interaction with the substrate to effect the reaction.

In a particular embodiment of any of the methods of the invention, the endoglucanase catalytic domain comprises or consists of a sequence selected from the group consisting of SEQ ID NO: 1, 2, 3 and a functionally equivalent variant thereof.

In a particular embodiment, the endoglucanase catalytic domain comprises or consists of the sequence of SEQ ID NO: 1.

In a particular embodiment, the endoglucanase catalytic domain comprises or consists of a functionally equivalent variant of the sequences of SEQ ID NO: 1, 2, or 3, preferably of SEQ ID NO: 1.

The term "functionally equivalent variant" as used herein is understood to mean all those proteins derived from a sequence by modification, insertion and/or deletion or one or more amino acids, whenever the function is substantially maintained, particularly in the case of a functionally equivalent variant of an endoglucanase catalytic domain refers to maintaining the endoglucanase catalytic activity.

A functionally equivalent variant of the catalytic domain of sequence SEQ ID NO: 1, 2 or 3 can be an amino acid sequence derived from SEQ ID NO: 1, 2 or 3 comprising the addition, substitution or modification of one or more amino acid residues. By way of illustration, functionally equivalent variants of the catalytic domain of sequence SEQ ID NO: 1, 2 or 3 include sequences comprising the addition of 1 amino acid, 2 amino acids, 3 amino acids, 4 amino acids, 5 amino acids, 10 amino acids, 15 amino acids, 20 amino acids, 25 amino acids, 30 amino acids, 35 amino acids, 40 amino acids, 45 amino acids, 50 amino acids, 60 amino acids, 70 amino acids, 80 amino acids, 90 amino acids, 100 amino acids, 150 amino acids, 200 amino acids, at least 500 amino acids, at least 1000 amino acids or more at the amino terminus of the sequence SEQ ID NO: 1, 2 or 3, and/or comprising the addition of 1 amino acid, 2 amino acids, 3 amino acids, 4 amino acids, 5 amino acids, 10 amino acids, 15 amino acids, 20 amino acids, 25 amino acids, 30 amino acids, 35 amino acids, 40 amino acids, 45 amino acids, 50 amino acids, 60 amino acids, 70 amino acids, 80 amino acids, 90 amino acids, 100 amino acids, 150 amino acids, 200 amino acids, at least 500 amino acids, at least 1000 amino acids or more at the carboxy terminus of the sequence SEQ ID NO: 1, 2 or 3.

Functionally equivalent variants of a catalytic domain of sequence SEQ ID NO: 1, 2 or 3 also include sequences with a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with the sequences of SEQ ID NO: 1, 2 or 3.

The terms "identity", "identical" or "percent identity" in the context of two or more amino acid sequences, refer to two or more sequences or sub-sequences that are the same or have a specified percentage of amino acid residues that are the same, when compared and aligned (introducing gaps, if necessary) for maximum correspondence, not considering any conservative amino acid substitutions as part of the sequence identity. The percent identity can be measured using sequence comparison software or algorithms or by visual inspection. Various algorithms and software are known in the art that can be used to obtain alignments of amino acid or nucleotide sequences. One such non-limiting example of a sequence alignment algorithm is the algorithm described in Karlin et al., 1990, Proc. Natl. Acad. Sci., 87:2264-8, as modified in Karlin et al., 1993, Proc. Natl. Acad. Sci., 90:5873-7, and incorporated into the NBLAST and XBLAST programs (Altschul et al., 1991, Nucleic Acids Res., 25:3389-402). In certain embodiments, Gapped BLAST can be used as described in Altschul et al., 1997, Nucleic Acids Res. 25:3389-402. BLAST-2, WU-BLAST-2 (Altschul et al., 1996, Methods in Enzymology, 266:460-80), ALIGN, ALIGN-2 (Genentech, South San Francisco, California) or Megalign (DNASTAR) are additional publicly available software programs that can be used to align sequences. In certain embodiments, the percent identity between two nucleotide sequences is determined using the GAP program in GCG software (e.g., using a NWSgapdna.CMP matrix and a gap weight of 40, 50, 60, 70, or 90 and a length weight of 1, 2, 3, 4, 5, or 6). In certain alternative embodiments, the GAP program in the GCG software package, which incorporates the algorithm of Needleman and Wunsch (J. Mol. Biol. 48:444-53 (1970)) can be used to determine the percent identity between two amino acid sequences (e.g., using either a Blossum 62 matrix or a PAM250 matrix, and a gap weight of 16, 14, 12, 10, 8, 6, or 4 and a length weight of 1, 2, 3, 4, 5). Alternatively, in certain embodiments, the percent identity between nucleotide or amino acid sequences is determined using the algorithm of Myers and Miller (CABIOS, 4:11-7 (1989)). For example, the percent identity can be determined using the ALIGN program (version 2.0) and using a PAM120 with residue table, a gap length penalty of 12 and a gap penalty of 4. Appropriate parameters for maximal alignment by particular alignment software can be determined by one skilled in the art. In certain embodiments, the default parameters of the alignment software are used. In certain embodiments, the percentage identity "X" of a first amino acid sequence to a second sequence amino acid is calculated as 100 x (Y/Z), where Y is the number of amino acid residues scored as identical matches in the alignment of the first and second sequences (as aligned by visual inspection or a particular sequence alignment program) and Z is the total number of residues in the second sequence. If the second sequence is longer than the first sequence, then the percent identity may be determined only in the region of overlap between said first and second sequences. In this case, the same formula as above can be used but using as Z value the length of the region wherein the first and second sequence overlaps, said region having a length which is substantially the same as the length of the first sequence.

In a particular embodiment, the sequence identity is determined throughout the whole length of the sequence of the endoglucanase domain comprising the sequence of SEQ ID NO: 1, 2 or 3, or through the whole length of the variant or both.

As a non-limiting example, whether any particular polynucleotide has a certain percentage sequence identity (e.g., is at least 70% identical, at least 75% identical, at least 80% identical, at least 85% identical, at least 90% identical, and in some embodiments, at least 95%, 96%, 97%, 98%, or 99% identical) to a reference sequence can, in certain embodiments, be determined using the Bestfit program (Wisconsin Sequence Analysis Package, Version 8 for Unix, Genetics Computer Group, University Research Park, 575 Science Drive, Madison, WI 53711). Bestfit uses the local homology algorithm of Smith and Waterman, Advances in Applied Mathematics 2:482-9 (1981), to find the best segment of homology between two sequences. When using Bestfit or any other sequence alignment program to determine whether a particular sequence is, for instance, 95% identical to a reference sequence according to the present invention, the parameters are set such that the percentage of identity is calculated over the full length of the reference amino acid sequence and that gaps in homology of up to 5% of the total number of nucleotides in the reference sequence are allowed.

In some embodiments, two amino acid sequences are substantially identical, meaning they have at least 70%, at least 75%, at least 80%, at least 85%, at least 90%, and in some embodiments at least 95%, 96%, 97%, 98%, 99% amino acid residue identity, when compared and aligned for maximum correspondence, as measured using a sequence comparison algorithm or by visual inspection. Identity can exist over a region of the sequences that is at least about 10, about 20, about 40-60 residues in length or any integral value there between, and can be over a longer region than 60-80 residues, for example, at least about 90-100 residues, and in some embodiments, the sequences are substantially identical over the full length of the sequences being compared.

In a particular embodiment, suitable functionally equivalent variants of endoglucanase catalytic domain of SEQ ID NO: 1, 2 or 3 are those including one or more conservative substitutions of the amino acids. "Conservative amino acid substitutions" result from replacing one amino acid with another having similar structural and/or chemical properties For example, the following six groups each contain amino acids that are conservative substitutions for one another: 1) Alanine (A), Serine (S), Threonine (T); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); and 6) Phenylalanine (F), Tyrosine (Y), Tryptophan (W). Selection of such conservative amino acid substitutions is within the skill of one of ordinary skill in the art and is described, for example by Dordo et al. et al., [J. Mol. Biol, 1999, 217;721-739] and Taylor et al., [J. Theor. Biol., 1986, 119:205-218].

A functionally equivalent variant of the endoglucanase catalytic domain of sequence SEQ ID NO: 1, 2 or 3 substantially maintains or improves its endoglucanase catalytic activity.

The term "endoglucanase catalytic activity", as used herein, refers to the ability of hydrolysing 1,4-linkages in β-D-glucans also containing 1,3-linkages. Assays to determine the function of an enzyme are known by the skilled person and include, without limitation, initial rate assays, progress curve assays, transient kinetics assays and relaxation assays. Continuous assays of enzymatic activity include, without limitation, spectrophotometric, fluorometric, calorimetric, chemiluminiscent, light scattering and microscale thermopheresis assays. Discontinuous assays of enzymatic activity include, without limitation, radiometric and chromatographic assays. As the skilled person understands, factors that may influence enzymatic activity comprise salt concentration, temperature, pH, and substrate concentration. Particularly, the endoglucanase catalytic activity may be measured by means of a number of techniques assays that are conventional to the skilled person, including the viscosimetric methods using soluble derivatised cellulose, or by employing soluble or insoluble (crosslinked) dyed cellulose or mixed-linkage β-glucan, such as the carboxymethyl cellulose (CMC) assay, and the hydroxyethylcellulose (HEC) assay. In general, assays based on the use of dyed polysaccharides are standardised against a reducing sugar method that employs either CM-cellulose or β-glucan as substrate. In a particular embodiment, the CELLG3 assay (particularly K-CellG3, Megazyme International Ireland) may be used for specific endocellulases and measures activity.

As with other enzymes, the catalytic activity of the endoglucanase catalytic domain depends on a number of reaction parameters, including temperature and pH. Thus, in one embodiment, the functionally equivalent variant of the endoglucanase catalytic domain of sequence SEQ ID NO: 1, 2 or 3 maintains or improves its endoglucanase catalytic activity at a temperature of at least 0°C, at least 5°C, at least 10°C, at least 15°C, at least 20°C, at least 25°C, at least 30°C, at least 35°C, at least 37°C, at least 40°C, at least 45°C, at least 50°C, at least 55°C, at least 60°C, at least 65°C, at least 70°C, at least 75°C, at least 80°C, at least 85°C, at least 90°C, at least 95°C, at least 100°C, or higher. Likewise, in another embodiment the functionally equivalent variant of the endoglucanase catalytic domain of sequence SEQ ID NO: 1, 2 or 3 maintains or improves its endoglucanase catalytic activity at pH 0, or at least pH 0.1, or at least pH 0.5, or at least pH 1.0, or at least pH 1.5, or at least pH 2.0, or at least pH 2.5, or at least pH 3.0, or at least pH 3.5, or at least pH 4.0, or at least pH 4.5, or at least pH 5.0, or at least pH 5.5, or at least pH 6.0, or at least pH 6.5, or at least pH 7.0, or at least pH 7.5, or at least pH 8.0, or at least pH 8.5, or at least pH 9.0, or at least pH 9.5, or at least pH 10.0, or at least pH 10.5, or at least pH 11.0, or at least pH 11.5, or at least pH 12.0, or at least pH 12.5, or at least pH 13.0, or at least pH 13.5, or pH 14. All possible combinations of temperatures and pH are also contemplated by the invention.

In a particular embodiment, the functionally equivalent variant of the endoglucanase catalytic domain of SEQ ID NO: 1, 2 or 3 maintains at least 50%, at least 60%, at least 70%, at least 75%, at least 0%, at least 85%, at least 90%, at least 91%, at least 92%, at least 93%, at least 94%, at least 95%, at least 96%, at least 97%, at least 98%, at least 99%, or at least 100% of the endoglucanase catalytic activity of the catalytic domain of sequence SEQ ID NO: 1, 2 or 3; or improves it in at least 1%, at least 2%, at least 3%, at least 4%, at least 5%, at least 6%, at least 7%, at least 8%, at least 9%, at least 10%, at least 15%, at least 20%, at least 25%, at least 30%, at least 35%, at least 40%, at least 45%, at least 50%, at least 55%, at least 60%, or at least 65%, at least 70%, at least 75%, at least 80%, at least 85%, at least 90%, at least 95%, at least 100%, at least 150%, at least 200%, at least 300%, at least 400%, at least 500%, at least 1000%, or more.

In a particular embodiment, the functionally equivalent variant of the endoglucanase catalytic domain of SEQ ID NO: 1, 2 or 3 comprises or consist of an amino acid sequence with a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with the sequences of SEQ ID NO: 1, 2 or 3, and maintains at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, or at least 100% of the endoglucanase catalytic activity of the endoglucanase catalytic domain of sequence SEQ ID NO: 1, 2 or 3, or improves it in at least 1%, at least 2%, at least 3%, at least 4%, at least 5%, at least 6%, at least 7%, at least 8%, at least 9%, at least 10%, at least 15%, at least 20%, at least 25%, at least 30%, at least 35%, at least 40%, at least 45%, at least 50%, at least 55%, at least 60%, or at least 65%, at least 70%, at least 75%, at least 80%, at least 85%, at least 90%, at least 95%, at least 100%, at least 150%, at least 200%, at least 300%, at least 400%, at least 500%, at least 1000%, or more.

In a particular embodiment, the enzyme having endoglucanase activity comprises a carbohydrate binding domain.

The term "carbohydrate binding domain" or "carbohydrate binding module" or "CBM", as used herein, refers to a protein domain that is present in carbohydrate-active enzymes (for example endocellulases and exocellulases) and having carbohydrate-binding activity. Carbohydrate binding domains contributes to the catalytic efficiency by increasing enzyme-substrate complex formations.

In a particular embodiment, the CBM comprises, or consists of a sequence selected from the group consisting of SEQ ID NO: 4 and 6 to 34.

In a more particular embodiment, the carbohydrate binding domain comprises or consists of SEQ ID NO: 4 or a functionally equivalent variant thereof. In an even more particular embodiment, the carbohydrate binding domain comprises or consists of the sequence of SEQ ID NO: 4.

The particulars of a functionally equivalent variant in terms of sequence identity previously described in the context of the endoglucanase catalytic domain also apply to the carbohydrate binding domain, with the necessary amendments, as will be evident for the person skilled in the art.

Thus, functionally equivalent variants of a carbohydrate binding domain of sequence SEQ ID NO: 4 also include carbohydrate binding domains comprising or consisting of amino acid sequences with a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with the sequence SEQ ID NO: 4.

In a particular embodiment, the functionally equivalent variant of the carbohydrate binding domain of SEQ ID NO: 4 maintains at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, or at least 100% of its carbohydrate binding activity or improves it in at least 1%, at least 2%, at least 3%, at least 4%, at least 5%, at least 6%, at least 7%, at least 8%, at least 9%, at least 10%, at least 15%, at least 20%, at least 25%, at least 30%, at least 35%, at least 40%, at least 45%, at least 50%, at least 55%, at least 60%, or at least 65%, at least 70%, at least 75%, at least 80%, at least 85%, at least 90%, at least 95%, at least 100%, at least 150%, at least 200%, at least 300%, at least 400%, at least 500%, at least 1000%, or more.

The activity of a carbohydrate binding domain may be measured as the affinity of said domain for cellulose, for example using biotinylated glycan binding assay or enzyme-linked assay (Kim et al., Biotecnhology and bioprocess engineering 19: 575-580 (2013).

In a particular embodiment, the enzyme comprises an endoglucanase catalytic domain comprising or consisting of SEQ ID NO: 1 or a functionally equivalent variant thereof that substantially maintains or improves its endoglucanase catalytic activity and a carbohydrate binding domain, preferably a carbohydrate binding domain comprising or consisting of the sequence of SEQ ID NO: 4 or a functionally equivalent variant thereof.

In a particular embodiment the enzyme comprises an endoglucanase catalytic domain comprising or consisting of SEQ ID NO: 2 or a functionally equivalent variant thereof that substantially maintains or improves its endoglucanase catalytic activity and a carbohydrate binding domain, preferably a carbohydrate binding domain comprising or consisting of the sequence of SEQ ID NO: 4 or a functionally equivalent variant thereof.

In a particular embodiment, the enzyme comprises an endoglucanase catalytic domain comprising or consisting of SEQ ID NO: 3 or a functionally equivalent variant thereof that substantially maintains or improves its endoglucanase catalytic activity and a carbohydrate binding domain, preferably a carbohydrate binding domain comprising or consisting of the sequence of SEQ ID NO: 4 or a functionally equivalent variant thereof.

In a particular embodiment, the endoglucanase catalytic domain and the carbohydrate binding domain are connected by a linking domain.

The term "linking domain", as used herein, refers to a sequence between domains. Linkers are often composed of flexible residues like glycine and serine so that the adjacent protein domains are free to move relative to one another. Longer linkers are used when it is necessary to ensure that two adjacent domains do not sterically interfere with one another.

In a particular embodiment, the linking domain comprises or consist of the sequence of SEQ ID NO: 5 or a variant thereof, said linking domain being located between the catalytic domain and the carbohydrate binding domain.

Functionally equivalent variants of a linking domain of sequence SEQ ID NO: 5 also include amino acid sequences with a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with the sequence of SEQ ID NO: 5.

In a particular embodiment, the variant of the linking domain is a functionally equivalent variant thereof. The particulars of a functionally equivalent variant previously described in the context of the endoglucanase catalytic domain also apply to the linking domain, with the necessary amendments, as will be evident for the person skilled in the art.

In a particular embodiment, the enzyme comprises an endoglucanase catalytic domain comprising or consisting of SEQ ID NO: 1 or a functionally equivalent variant thereof that substantially maintains or improves its endoglucanase catalytic activity, a carbohydrate binding domain, preferably a carbohydrate binding domain comprising or consisting of SEQ ID NO: 4 or a functionally equivalent variant thereof, and a linking domain, preferably a linking domain comprising or consisting of the sequence of SEQ ID NO: 5 or a variant thereof.

In a particular embodiment, the enzyme comprises an endoglucanase catalytic domain comprising or consisting of SEQ ID NO: 2 or a functionally equivalent variant thereof that substantially maintains or improves its endoglucanase catalytic activity, a carbohydrate binding domain, preferably a carbohydrate binding domain comprising or consisting of SEQ ID NO: 4 or a functionally equivalent variant thereof, and a linking domain, preferably a linking domain comprising or consisting of the sequence of SEQ ID NO: 5 or a variant thereof.

In a particular embodiment, the enzyme comprises an endoglucanase catalytic domain comprising or consisting of SEQ ID NO: 3 or a functionally equivalent variant thereof that substantially maintains or improves its endoglucanase catalytic activity, a carbohydrate binding domain, preferably a carbohydrate binding domain comprising or consisting of SEQ ID NO: 4 or a functionally equivalent variant thereof, and a linking domain, preferably a linking domain comprising or consisting of the sequence of SEQ ID NO: 5 or a variant thereof.

When the enzyme comprising an endoglucanase catalytic domain further comprises a carbohydrate domain, preferably a carbohydrate domain comprising or consisting of SEQ ID NO: 4, and a linking domain, preferably a linking domain comprising or consisting of the sequence of SEQ ID NO: 5 or a variant thereof, the order of these components, from the N- terminus to the C-terminus can be endoglucanase catalytic domain, linking domain and carbohydrate binding domain, or carbohydrate domain, linking domain and endoglucanase catalytic domain.

The term "hydrolyzing cellulose", as used herein, refers to the cleave of chemical bonds of cellulose. Therefore, the conditions suitable for hydrolyzing cellulose are those conditions, which can be easily determined by the skilled person, under which at least part of the (1-4)-β-D-glucosidic links in cellulose are cleaved.

Without wanting to be bound to any theory, it is understood that endoglucanases, and as such also the enzyme comprising an endoglucanase catalytic domain comprising the sequence of SEQ ID NO: 1, 2 or 3, hydrolyse the amorphous regions of cellulose more easily than the crystalline domains. Therefore, depending on the conditions under which the substrate comprising cellulose and the enzyme are contacted, nanocelullose particles of different length, diameter, aspect/ratio and crystallinity can be obtained in different proportions, that is, cellulose nanocrystals, cellulose nanofibers or a mixture thereof can be obtained.

The substrate comprising cellulose and the enzyme having endoglucanase activity can be contacted under a pH between 4 and 10, preferably greater than 4.5, particularly, at least 5.0, at least 5.5, at least 6.0, at least 6.5, at least 6.8, at least 7.0, at least 7.2, at least 7.4, at least 7.6, at least 7.8, at least 8.0, at least 8.5, at least 9.0, at least 9.5, at least 10, , more particularly between 6.5 and 7.5.

In a particular embodiment, the substrate comprising cellulose and the enzyme having endoglucanase activity are contacted under a temperature between 25°C and 80°C, between 30°C and 70°C, between 40°C and 60°C, between 45°C and 55°C, for example approximately 30°C, approximately 35°C, approximately 40°C, approximately 45°C, approximately 50°C, approximately 55°C, approximately 60°C, approximately 65°C, approximately 70°C, approximately 75°C, approximately 80°C, preferably approximately 50°C.

In a particular embodiment, the sample comprising cellulose is chopped into smaller pieces to accelerate the process of enzymatic hydrolysis.

In a particular embodiment, the substrate comprising cellulose and the enzyme are mixed together in water to form a suspension.

In a particular embodiment, the substrate comprising cellulose and the enzyme are contacted for a period of time of less than 72 hours, preferably less than 48 hours. In a more particular embodiment, the substrate comprising cellulose and the enzyme are contacted for a period of time comprised between 1 and 48 hours, for example, 2 hours, 4, hours, 5 hours, 6 hours, 8 hours, 10 hours, 12 hours, 14 hours, 16 hours, 18 hours, 20 hours, 22 hours, 24 hours, 26 hours, 28 hours, 30 hours, 32 hours, 34 hours, 36 hours, 38 hours, 40 hours, 42 hours, 44 hours, 46 hours, or 48 hours.

In a particular embodiment, the substrate comprising cellulose and the enzyme are contacted under agitation.

Once the above period of time is completed, that is, once the desired cellulose fibers are obtained, the hydrolysis reaction can be stopped, for example, by decreasing the temperature of the reaction mixture to a temperature at which no hydrolysis is performed, for example, by incubation on ice.

Once the above period of time is completed, that is, once the desired cellulose fibers are obtained, the hydrolysis reaction can be subjected to sonication. The optional sonication step may improve the homogeneity of the hydrolysis.

In a particular embodiment, the resulting cellulose fibers can be isolated from the rest of the components of the reaction mixture, for example, substrate that has not been hydrolysed, enzyme, or other products of the reaction, such as sugars. In a more particular embodiment, the cellulose fibers are isolated by filtration and/or centrifugation and/or any other suitable technique known by the skilled person. In a particular embodiment, the cellulose fibers are isolated by filtration. In another particular embodiment, the cellulose fibers are isolated by centrifugation. In another particular embodiment, the cellulose fibers are isolated by filtration and centrifugation. In a particular embodiment, said cellulose fibers are cellulose nanocrystals. In another particular embodiment, said cellulose fibers are cellulose nanofibers. In yet another particular embodiment, said cellulose fibers are a mixture comprising both cellulose nanocrystals and cellulose nanofibers.

The filtration can be performed by using microfiltration, rotatory drum filter or using a nylon filter of 5 to 20 μm mesh. To reach the maximum recovery of cellulose nanocrystals and/or cellulose nanofibers, the retentate can be resuspended in a lower volume of water and washed several times, repeating the filtration until the filtrate is clear. The cellulose nanocrystals and/or cellulose nanofibers can be recovered from the filtrate and washings by centrifugation at 15000 g (relative centrifuge force) for 30 minutes to 1 hour.

The isolation by centrifugation can be performed in two steps. The first step is to wash the undigested fibers, recovering the cellulose nanocrystals and/or cellulose nanofibers from the supernatant of successive washings with decreasing volumes of water followed by centrifugation at 4000 g (relative centrifuge force) for 5 minutes. The washings should be repeated by suspending the sediment in water until the supernatant is clear and free of nanocellulose material. The second recovery step concentrates the suspended material of the supernatant of previous washings by higher speed centrifugation at 15000 g for 30 minutes to 1 hour. After centrifugation, the sediment comprising the cellulose nanocrystals and/or cellulose nanofibers can be dried, for example by spray drying or lyophilisation.

In a particular embodiment, the method of the invention does not comprise any step directed to the chemical hydrolysis of the substrate comprising cellulose, and in particular, it does not comprise treating the substrate comprising cellulose with sulphuric acid and/or with hydrochloric acid.

In a particular embodiment, the substrate comprising cellulose is a lignocellulosic material. The term "lignocellulosic material" or "lignocellulosic substrate" has been previously defined.

In a more particular embodiment, when the substrate comprising cellulose is a lignocellulosic material, the step of hydrolyzing the substrate comprising cellulose further comprises contacting the lignocellulosic material with a xylanase and/or a lytic polysaccharide monooxygenase (LPMO).

The term "xylanase", as used herein, refers to a 1,4-beta-D-xylan-xylohydrolase (E.C. 3.2.1.8) that catalyses the endohydrolysis of 1,4-beta-D-xylosidic linkages in xylans.

Xylanase activity can be determined with 0.2% AZCL-arabinoxylan as substrate in 0.01 percent TRITON(R) X-100 and 200 mM sodium phosphate pH 6 at 37°C. One unit of xylanase activity is defined as 1.0 μmol of azurine produced per minute at 37°C, pH 6 from 0.2% AZCL-arabinoxylan as substrate in 200 mM sodium phosphate pH 6.

Xylanases (e.g. endo- beta -xylanases (E.C. 3.2.1.8), which hydrolyse the xylan backbone chain, can be from bacterial sources (e.g., Bacillus, Streptomyces, Clostridium, Acidothermus, Microtetrapsora or Thermonospora) or from fungal sources (Aspergillus, Trichoderma, Neurospora, Humicola, Penicillium or Fusarium (See, e.g., EP473 545; U.S. Pat. No. 5,612,055; WO 92/06209; and WO 97/20920)). Xylanases useful in the invention include commercial preparations (e.g., MULTIFECT(R) and FEEDTREAT(R) Y5 (Danisco Genencor), RONOZYME(R) WX (Novozymes A/S) Pulpzyme(R) HC (Novozymes A/S) and NATUGRAIN WHEAT(R) (BASF). In some embodiments the xylanase is from *Trichoderma reesei* or a variant xylanase from *Trichoderma reesei,* or the inherently thermostable xylanase described in EP1222256B1, as well as other xylanases from *Aspergillus niger, Aspergillus kawachii, Aspergillus tubigensis, Bacillus circulans, Bacillus pumilus, Bacillus subtilis, Neocallimastix patriciarum, Penicillium species, Streptomyces lividans, Streptomyces thermoviolaceus, Thermomonospora fusca, Trichoderma harzianum, Trichoderma reesei* and *Trichoderma viridae.*

In a particular embodiment, the xylanase is from *Trichoderma viridae.* The term *"Trichoderma viridae",* as used herein, refers to a fungus identified in the NCBI database by the Taxonomy ID: 5547.

The term "lytic polysaccharide monooxigenase" or "LPMO", as used herein, refers to copper-dependent enzymes that catalyse the oxidative cleave on glycosidic bonds in polysaccharides such as chitin, cellulose and hemicellulose by oxidation. LPMO oxidation is proposed to produce chain cleavage, the chain break is made on C1 or C4 carbon of glucose, even in C6 in some cases. Although originally classified as glycoside hydrolases, these enzymes are currently classified in the auxiliary activity families AA9, AS10 and AA11 in the CAZy database (Levasseur A. et al., Biotechnol Biofuels. 2013, 6: 41).

Illustrative non-limitative examples of LPMOs that can be used in the method of the invention LPMOs that oxidaze both C1 and C4, like LPMO from *Streptomyces coelicolor,* or LPMO that selectively oxidaze one of the C, like LPMO from *Myceliophthora thermophile.*

LPMO activity can be assayed using polysaccharide substrates. The assay usually comprises incubation of the substrate and the LPMO with a reductant followed by analysis of soluble products, that is, the oxidized oligosaccharides) by MALDI-TOF mass spectrometry or HPLC (high performance liquid chromatography), as described for example by Eijsink et al., Biotechnol Biofuels. 2019, 12: 58).

In a particular embodiment, the step of hydrolyzing the substrate comprising cellulose comprises contacting the lignocellulosic material with a xylanase. In a particular embodiment, step of hydrolyzing the substrate comprising cellulose comprises contacting the lignocellulosic material with a lytic polysaccharide monooxygenase (LPMO). In a particular embodiment, the step of hydrolyzing the substrate comprising cellulose comprises contacting the lignocellulosic material with a xylanase and a lytic polysaccharide monooxygenase (LPMO).

The lignocellulosic material can be contacted with the xylanase and/or LPMO previously, simultaneously or after the lignocellulosic material is contacted with the enzyme having endoglucanase activity. In a preferred embodiment, the lignocellulosic material is contacted with the xylanase and/or with the LPMO at the same time it is contacted with the enzyme having endoglucanase activity. In this particular embodiment, the enzyme having endoglucanase activity, the xylanase and/or the LPMO can be mixed together forming an enzyme cocktail.

Obtainable composite materials

In a fourth aspect, the present invention is directed to a cellulose composite materials obtainable by the method of the invention.

Therefore, all the definitions, particular and preferred embodiments of the composite material and of the method of the invention fully apply to the cellulose fibers obtainable by enzymatic hydrolysis and also to the cellulose composite material obtainable by the method of the invention described and claimed herein.

Uses and compositions

The cellulose composite material of the invention comprises flexible cellulose fibers which provide advantageous properties as support for the conductive carbon material. The resulting electrically conductive composite is surprisingly stable against both temperature and water. This is particularly surprising because when the material is prepared with cellulose fibers obtained from chemical hydrolysis, it does not hold its integrity in water. The cellulose composite material of the invention has several advantageous properties, especially in terms of stability, flexibility and mechanical strength, electric conductivity and cost.

The cellulose composite material of the invention thus finds applications in the field of the printed electronics, in all of its forms ranging from screen-printing, pad-printing and inkjet printing to the different mass-printing methods, such as offset printing, engraving and flexography. The material of the invention finds use in manufacturing low-cost electronic products on common-use materials, such as papers or textiles.

Therefore, in a fifth aspect, the invention contemplates the use of the cellulose composite material of the invention in dyes, including conductive dyes or paints, electronics, optoelectronics, transistors, touch screens, medicine, biomedicine, tissue engineering, health diagnostics, sensors and biosensors, food security, packaging, textiles, including electronic textiles, labels, radio frequency tags, automotive industry, energy industry including production and storage and construction materials. Preferably, the invention contemplates the use of the cellulose composite material of the invention in conductive dyes or paints, electronics, medicine, tissue engineering, health diagnostics, sensors and biosensors, textiles, including electronic textiles, labels and construction materials.

A sixth aspect of the invention is directed to a product comprising the cellulose composite material of the invention, in particular to a conductive paint composition, comprising the cellulose composite material of the invention. In this context, paint is to be interpreted as synonym of ink or dye. The skilled person in the art will be familiar with the essential requirements to obtain a conductive paint composition comprising the cellulose composite material of the invention.

In a particular embodiment, the conductive paint composition comprises from 0.1 to 90 wt% of the cellulose composite material of the invention. Preferably, from 0.1 to 70 wt%, from 0.1 to 50 wt%, from 0.1 to 30 wt%, from 0.1 to 15 wt% and from 0.1 to 10%. In another particular embodiment, the conductive paint composition comprises at least 0.01 wt% of the cellulose composite material of the invention, preferably at least 0.05 wt%, at least 0.1 wt%, at least 0.3 wt%, at least 0.4 wt%, at least 0.5 wt%, at least 1 wt%, at least 1.5 wt%, at least 2 wt%, at least 5 wt%, or at least 10 wt% of the cellulose composite material of the invention.

The composition may comprise further additives, such as a binder, a carrier solvent, a dispersant, or a mixture thereof, as disclosed for example in US20130207294A1 or US4490282A.

In a particular embodiment, the conductive paint composition of the invention further comprises a binder, preferably a fluid hardenable binder. Examples of such binders may include one or more selected from the group consisting of polyamides, polypropylenes, polybutylenes, phenolics, urethanes or polyurethanes, epoxy, melamine, acetal, acrylic, acrylic-styrene-containing emulsion, vinyl acrylic-based emulsion, carbonates or polycarbonates, styrenes or polystyrenes, esters or polyesters, vinyl, polyphenylene ether resin, acrylonitrile-butadiene-styrene, polysiloxane, organometallic complexes, and a copolymer thereof. In a particular embodiment, the conductive paint composition of the invention comprises from 5 to 15 wt % of binder.

In a particular embodiment, the conductive paint composition of the invention further comprises a carrier solvent. A carrier solvent may be one or more selected from the group consisting of water, alcohols, ketones, amines, esters, amides, alkyl halides, ethers, furans, sulfur-containing solvents, and a mixture thereof. In a particular embodiment, the solvent is one or more selected from the group consisting of water, methyl alcohol, ethyl alcohol, propyl alcohol, butyl alcohol, isopropyl alcohol, isobutyl alcohol, ethylene glycol, acetone, methylethylketone, cyclohexanone, hexane, diethyl amine, triethyl amine, octadecyl amine, cyclohexane, ethyl acetate, acetone, dimethyl formamide, dimethylacetamide, methylene chloride, chloroform, carbon tetrachloride, dimethyl sulfoxide, dioxin, nitromethane, toluene, xylene, dichlorobenzene, dimethyl benzene, trimethyl benzene, methyl naphthalene, tetrahydrofuran, N-methyl-2-pyrrolidone, pyridine, acrylonitrile, aniline, sorbitol, carbitol, carbitol acetate, methyl cellosolve, and ethyl cellosolve. In a particular embodiment, the solvent is pure water or a water-based solvent in which water and organic solvent are mixed.

In a particular embodiment, the conductive paint composition of the invention further comprises a dispersant made of a block copolymer consisting of a hydrophilic polymer unit and a hydrophobic polymer unit. Examples of the block copolymer consisting being a styrene-acrylic based water-soluble resin. In a particular embodiment, the conductive paint composition of the invention comprises from 0.05 to 5 wt % of the dispersant.

In the present invention, one or more conductive additives selected from metal powders, metal particles, metal-coated inorganic powders, and metal fibers, besides the carbon material of the cellulose composite material of the invention, may be mixed in the conductive paint composition, thereby being used as a further conductive material. In a particular embodiment, the conductive paint composition of the sixth aspect of the invention further comprises metal conducting particles. Preferably, said metal conducting particles are selected from silver, copper, gold, aluminum, beryllium, magnesium, cobalt, tungsten, molybdenum, zinc, nickel, cadmium, iron, tin, platinum, chromium, lead, zirconium, lithium, titanium and manganese, preferably silver, copper, gold, aluminum, nickel, iron and lithium. In a preferred embodiment, the metal conducting particles are present at a concentration of from 0.05 to 80 wt %, preferably from 10 to 80 wt %, from 20 to 80 wt %, from 30 to 80 wt %, from 40 to 80 wt %, more preferably from 50 to 80 wt %.

Additional aspects of the invention

1. A cellulose composite material comprising:

   a. Type I cellulose fibers selected from the group consisting of cellulose nanocrystals, cellulose nanofibers and a mixture thereof; and
   b. A carbon material selected from the group consisting of carbon nanotubes, carbon nanofibers, graphene, graphene oxide and a mixture thereof.

2. The cellulose composite material according to embodiment 1, wherein:

   - the cellulose nanocrystals have a length comprised between 10 and 900 nm, and a length to width aspect ratio comprised between 5 and 100; and/or
   - the cellulose nanofibers have a length greater than 900 nm and lower or equal to 10 $\mu$m, and a length to width aspect ratio comprised between 100 and 2000.

3. The cellulose composite material according to any one of embodiments 1 or 2, wherein the cellulose fibers are cellulose nanocrystals and wherein the carbon material is graphene, graphene oxide or a mixture thereof.

4. The cellulose composite material according to any one of embodiments 1 to 3, wherein the cellulose fibers are obtained by hydrolyzing a substrate comprising cellulose with an enzyme having endoglucanase activity.

5. The cellulose composite material according to embodiment 4, wherein the enzyme having endoglucanase activity comprises an endoglucanase catalytic domain comprising a sequence selected from the group consisting of SEQ ID NO: 1, SEQ ID NO: 2, SEQ ID NO: 3 and a functionally equivalent variant thereof that substantially maintains or improves its catalytic activity.

6. The cellulose composite material according to any one of embodiments 4 or 5, wherein the enzyme having endoglucanase activity comprises a carbohydrate binding domain.

7. The cellulose composite material according to any one of embodiments 1 to 6, wherein the cellulose fibers have an intrinsic net charge of zero.

8. The cellulose composite material according to any one of embodiments 1 to 7, wherein the crystallinity index of the cellulose nanocrystals is ≥ 70%, preferably ≥ 80%, more preferably ≥ 85%.

9. The cellulose composite material according to any one of embodiments 1 to 8, wherein the composite material is an electrically conductive composite material.

10. The cellulose composite material according to any one of embodiments 1 to 9, wherein the cellulose fibers and the carbon material are homogeneously distributed within the composite material.

11. The cellulose composite material according to embodiment 10, comprising at least 0.1%, at least 2%, at least 10% or at least 20% w/w of said carbon material, with regards to the sum of the weights of the cellulose fibers and said carbon material.

12. The cellulose composite material according to any one of embodiments 1 to 10, characterized by a conductivity value equal to or greater than 5 S/m, measured by the four-terminal sensing technique.

13. The cellulose composite material according to any one of embodiments 1 to 9, wherein the composite is a layered composite material comprising a matrix of cellulose fibers and at least one layer of said carbon material on top of at least a first surface of said matrix.

14. The cellulose composite material of embodiment 13, wherein the at least one layer of said carbon material is a layer deposited by means of chemical vapor deposition.

15. The cellulose composite material of embodiment 13 and 14, wherein the at least one layer of carbon material has an average thickness of one $sp^2$ hybridized carbon atom.

16. A method for the preparation of the cellulose composite material of any one of embodiments 1 to 12, comprising the steps of:

   a. preparing a suspension comprising type I cellulose fibers selected from the group consisting of cellulose nanocrystals, cellulose nanofibers and a mixture thereof, and a carbon material selected from the group consisting of carbon nanotubes, carbon nanofibers, graphene, graphene oxide and a mixture thereof;
   b. mixing the suspension; and
   c. optionally reducing said carbon material in the mixture of the previous step.

17. The method according to embodiment 16, wherein optional step c comprises the reduction of graphene oxide to graphene.

18. A method for the preparation of the cellulose composite material of any one of embodiments 1 to 9 or any one of embodiments 13 to 15, comprising the step of:

   a. transferring at least one layer of a carbon material selected from the group consisting of carbon nanotubes, carbon nanofibers, graphene, graphene oxide and a mixture thereof onto a matrix of type I cellulose fibers selected from the group consisting of cellulose nanocrystals, cellulose nanofibers and a mixture thereof to form a layered composite material.

19. The method according to any one of embodiments 16 to 18, wherein the cellulose fibers are prepared by hydrolyzing a substrate comprising cellulose with an enzyme having endoglucanase activity.

20. The method according to embodiment 19, wherein the enzyme having endoglucanase activity comprises an endoglucanase catalytic domain comprising a sequence selected from the group consisting of SEQ ID NO: 1, SEQ ID NO: 2, SEQ ID NO: 3 and a functionally equivalent variant thereof that substantially maintains or improves its catalytic activity.

21. The method according to any one of embodiments 19 or 20, wherein the enzyme having endoglucanase activity comprises a carbohydrate binding domain.

22. The method according to any one of embodiments 19 to 21, wherein the cellulose fibers are isolated after the hydrolysis step.

23. The method according to any one of embodiments 19 to 22, wherein the cellulose fibers are dried after the hydrolysis step.

24. A cellulose composite material obtainable by the method according to any one of embodiments 16 to 23.

25. Use of the cellulose composite material according to any one of embodiments 1 to 15 or embodiment 24 in dyes, including conductive dyes or paints, electronics, optoelectronics, transistors, touch screens, medicine, biomedicine, tissue engineering, health diagnostics, sensors and biosensors, food security, packaging, textiles, including electronic textiles, labels, radio frequency tags, automotive industry, energy industry including production and storage and construction materials.

26. Conductive paint composition, comprising:

    a. the cellulose composite material according to any one of embodiments 1 to 15 or embodiment 24;
    b. a polymer binder; and
    c. a carrier solvent.

27. The conductive paint composition according to embodiment 26, further comprising metal conducting particles, wherein the metal is selected from silver, copper, gold, aluminum, beryllium, magnesium, cobalt, tungsten, molybdenum, zinc, nickel, cadmium, iron, tin, platinum, chromium, lead, zirconium, lithium, titanium and manganese, preferably silver, copper, gold, aluminum, nickel, iron and lithium.

The invention will be described by way of the following examples which are to be considered as merely illustrative and not limitative of the scope of the invention.

## EXAMPLES

### Materials and methods

Unless stated otherwise, all references of cellulose fiber are to be interpreted as cellulose fibers as defined above, i.e., cellulose nanocrystals, cellulose nanofibers or a mixture thereof. For the isolation of the cellulose fibers with endoglucanase treatment, Whatman® qualitative filter paper, Grade 1 (11 $\mu$m (medium flow filter paper)), was used as substrate. The Whatman® filter paper was used as received, and is described as being manufactured from cotton liners, which have been treated to achieve a minimum alpha cellulose content of 98%. Cellulose nanocrystals produced by sulfuric acid treatment purchased from Maine University were used for comparison, which were named as AcCNC. Cellulase from *Trichoderma reseei* was purchased from Sigma Aldrich (C2730). Graphene oxide (4 wt% water suspension) was kindly supplied by Graphenea.

The term "EGs" refers to endoglucanase(s). "ANC EG" means an enzyme including just the endoglucanase catalytic domain. "ANC EG+CBM" means an enzyme including the endoglucanase catalytic domain and the carbohydrate binding module.

### Protein expression and purification

ANC EG, ANC EG+CBM, *T. maritima* EGs protein encoding genes were synthetized and codon optimized for *E.coli* cell expression. ANC EG encodes a protein with the sequence SEQ ID NO: 1. ANC EG, ANC EG+CBM, *T. maritima* were cloned in pQE80L expression vector (Qiagen) and transform in E.coli BL21 (DE3) (Life Technologies) for protein expression. Cells were incubated in LB medium at 37°C until OD600 reached 0.6, IPTG was added to the medium to 1 mM concentration for protein induction overnight. Cells were pelleted by centrifugation at 4000 rpm. Pellets were resuspended in extraction buffer (50 mM sodium phosphate, pH 7.0, 300 mM NaCl) and mechanically lysed using French Press. Cell debris was separated by ultracentrifugation at 33000 G for 1 hour. For purification, the supernatants were mixed with His GraviTrap affinity column (GE Healthcare) and eluted in elution buffer (50 mM sodium phosphate, pH 7.0, 300 mM NaCl, 150 mM Imidazole). Proteins were further purified by size exclusion chromatography using a Superdex 200 HR column (GE Healthcare) and eluted in 50 mM citrate buffer pH 4.8. For protein purification verification sodium dodecyl sulfate-polyacrylamide gel electrophoresis (SDS-PAGE) was used with 12% gels. The protein concentration was calculated by measuring the absorbance at 280 nm in Nanodrop 2000C.

**Enzymatic cellulose fibers isolation**

0.4 g of filter paper were used for each hydrolysis, it was chopped in small squares of 1 cm². The reaction was made in 40 ml of water and 5 mg were used of each EGs (ANC EG, ANC EG+CBM and *T. maritima, B.subtillis* and *T.reesei* EG) per gram of substrate. Hydrolysis was incubated at 50°C in agitation for different times (1, 5, 24, and 48 h). Reactions were stopped by incubation on ice followed by sonication of the mixtures with a micro tip sonicator UPH100H Ultrasonic Processor (Hielscher) for 25 min at 75%. The cellulose fibers were isolated by gradual centrifugation steps and concentrated by ultracentrifugation at 33000 G for 1 hour. Pellets were re-suspended in water and lyophilized in a Telstar Lyoquest for physicochemical characterization by freeze-drying for 24 hours.

**Characterization**

*Cellulose fiber yield*

Cellulose fiber conversion was calculated by weighting the freeze-dried cellulose fibers from each hydrolysis and used the following equation to calculate the yield.

$$\text{Cellulose fiber yield (\%)} = \text{Cellulose fiber mass/Initial cellulose mass} \times 100$$

*Atomic force microscopy*

Atomic force microscopy (AFM) was used for studying the cellulose fiber morphology. Images were captured at room temperature, in tapping mode, using a Nanoscope V scanning probe microscope (Multimode 8 Bruker Digital instruments) with an integrated force generated by cantilever/silicon probes. The applied resonance frequency was 320 kHz. The cantilever had a tip radius of 5-10 nm and was 125 μm long. The samples were prepared by spin-coating (Spincoater P6700) at 2000 rpm for 130 s by casting a droplet of cellulose fiber suspension on mica substrate. AFM height and phase images were collected simultaneously from all the samples. From the images the length and the diameter of 100 nanoparticles for each sample were measured to calculate the average of the length and the diameter and the aspect ratio (Length/Diameter).

*Fourier transform infrared spectroscopy*

Fourier transform infrared spectroscopy (FTIR) was used for studying cellulose functional groups spectra. FTIR spectroscopy measurements were conducted using a Nicolet Nexus spectrometer provided with a MKII Golden Gate accessory (Specac) with a diamond crystal at a nominal incidence angle of 45° and ZnSe lens. Spectra were measured in attenuated reflection (ATR) mode between 4000 and 650 cm$^{-1}$, with averaging 32 scans and a resolution of 4 cm$^{-1}$.

*Nanoscale-resolved Fourier transform infrared spectroscopy*

Scattering-type scanning near-field optical microscopy (IR s-SNOM) and nanoscale-resolved Fourier transform infrared (nano-FTIR) permit to study the infrared spectra from a single cellulose fiber particle of a suspension. The IR s-SNOM images and nano-FTIR spectra, as well as the corresponding AFM images, were recorded with a near SNOM system (Neaspec GmbH, Germany) comprising both s-SNOM and nano-FTIR capabilities. Pt-Si coated AFM tips were used. Nano-FTIR spectroscopy of the cellulose fiber suspension was performed with illumination from a mid-infrared laser supercontinuum, using Au coated AFM tips. The final nano-FTIR spectrum was obtained by averaging 5 individual spectra. The total acquisition time was 5 min and the spectral resolution 16 cm-1. The spectra were normalized to that obtained on a clean gold surface (reference measurements).

*Sulfur content calculation by conductometric titration*

Sulfate content on AcCNC was measured by conductometric titration at 25°C with a Crison ECMeter GLP 31 conductometer that was calibrated with 147, 1413 and 12.88 μS/cm standards. AcCNC powder was diluted in 25 ml of water and sonicated for 30 min, then 10 ml of 10 mM HCl were added. The dispersion was continuously titrated with 10 mM NaOH 25. NaOH consumption was measured for the sample titration and the blank, and the sulfur content was calculated using the equation:

$$(\%) = (32 \times M \times V) \times 100$$

where M is the NaOH concentration, V is the volume of NaOH solution that needed to titrate the sample (without the volume used to titrate the blank) and $\omega$ is the weight of AcCNC used.

*X-ray diffraction*

X-ray diffraction (XRD) was used for the analysis of the cellulose crystalline structure, crystallinity and crystallite size. X-ray powder diffraction patterns were collected by using a Philips X'pert PRO automatic diffractometer operating at 40 kV and 40 mA, in theta-theta configuration, a secondary monochromator with Cu-Ka radiation ($\lambda$ = 1.5418 A) and a PIXcel solid state detector (active length in $2\theta$ 3.347°). Data were collected from 5 to 50° $2\theta$ (step size 0.026 and time per step 80 s) at room temperature. A fixed divergence and antiscattering slit giving a constant volume of sample illumination were used.

From the diffractograms, the crystallinity index (CI%) was calculated using the Segal equation:

$$\text{Crystallinity index } (\%) = (I_{200}-I_{am})/I_{200} \times 100$$

where $I_{200}$ is the intensity of the cellulose crystalline peak and $I_{am}$ is the intensity of the amorphous peak.

The crystallite size was measured from the diffractograms using the Scherrer's equation:

$$\beta = \kappa\lambda/\tau cos\theta \times 100$$

where $\lambda$ is the wavelength of the incident X-ray, $\theta$ is the angle of the (200) plane, $\beta$ is the full-width at half maximum of the (200) peak, $\tau$ is the crystallite size and $\kappa$ is a constant value.

*Solid-state cross-polarization magic angle spinning $^{13}$C nuclear magnetic resonance*

Solid-state cross-polarization magic angle spinning $^{13}$C nuclear magnetic resonance ($^{13}$C CP/MAS NMR) was used to study the cellulose structure. $^{13}$C CP/MAS NMR spectra were recorded on a 400 MHz BRUKER system equipped with a 4 mm MASDVT TRIPLE Resonance HYX MAS probe. Larmor frequencies were 400.17 MHz and 100.63 MHz for $^1$H and $^{13}$C nuclei, respectively. Chemical shifts were reported relative to the signals of $^{13}$C nuclei in glycine. Sample rotation frequency was 12 kHz and relaxation delay was 5 s. The number of scans was 2k. Polarization transfer was achieved with RAMP cross-polarization (ramp on the proton channel) with a contact time of 5 ms. High-power SPINAL 64 heteronuclear proton decoupling was applied during acquisition.

*Thermogravimetric analysis*

Thermal stability of the cellulose fibers was measured by thermogavimetric analysis (TGA). The analysis was performed using TGA/SDTA 851 Mettler Toledo equipment. Up to 10 mg of the samples were used and were heated from 30 to 800°C in a nitrogen atmosphere at a scanning rate of 10°C/min. The initial degradation temperature (To) is described as the loss of 5% of the weight of the total sample and the maximum degradation temperature (Td) is the minimum of the degradation peak in the derivative of thermogravimetric curves (DTG).

*Mechanical properties*

The mechanical properties of the nanopapers were measured using a Miniature materials testerMinimat 2000, Rheometric Scientific, equipment, at room temperature with a 20 N load cell and an elongation rate of 2 mm/min. The samples were cut with a width of 0.5 cm and a length of 1 cm.

*Water contact angle*

The hydrophilicity of the surface of the nanopapers was measured by static water contact angle (WCA) using a Dataphysics OCA20 equipment at room temperature. In this technique, a deionized water drop was deposited in the film surface to analyze the contact angle value formed by the water drop, which depended on the chemical interactions between the water and the material surface. When the material is hydrophilic, the contact angle is small and increases with the hydrophobicity. The contact angle ($\theta$c) values of ten water drops deposited by a syringe tip were averaged for each sample.

*Scanning electron microscopy*

The morphologies were analyzed by scanning electron microscopy (SEM) using a FEI ESEM Quanta 200 microscope operating at 5-20 kV. Samples were put on a carbon tape for adhesion.

*Electrical conductivity measurement*

Electrical conductivity was measured by a fourpoint probe method using a Probe Station 4 Everbeing. The specific resistances (q) were calculated using the equation:

$$q = Rs \times t$$

wherein Rs ($\Omega$/squares) is the sheet resistance and t (cm) is the thickness of the nanopapers.
Calculated specific resistance values were used to infer the corresponding conductivity (S/cm) with the following equation, transformed to S/m.

$$r = 1 / q.$$

**Example 1 - Enzymatic cellulose fibers isolation**

The isolation of cellulose fibers using enzymes requires the incubation of an EG with a cellulosic substrate such as filter paper that contains both crystalline and amorphous regions. The ANC EG used to hydrolyze the cellulose belongs to the last common ancestor of firmicutes (LFCA) and was obtained from a phylogeny using modern sequences from the family Cel5A of bacterial EG enzymes. The ANC EG displayed high activity over a wide range of conditions and in several substrates. This enzyme can be used in two forms, only the catalytic domain and linking the catalytic domain to a carbohydrate binding module (CBM). In nature, most bacterial EG have a CBM that helps in the degradation of cellulose over recalcitrant substrates. However, the CBM is poorly conserved across species and the reconstruction of ancestral from of it was not possible. In the present invention, two enzymes were obtained and used: (i) an enzyme including just the endoglucanase catalytic domain (ANC EG) and (ii) an enzyme including the endoglucanase catalytic domain and the CBM belonging to *Bacillus subtilis,* the species used as query sequence in the reconstruction (ANC EG+CBM). Three different modern EGs from *T. maritima, B. subtilis* and *T. reesei* where also used to compare their efficiency to that of the ancestral ones.

*Hydrolysis yields*

Hydrolysis was carried out in water to avoid nanoparticles flocculation and to skip posterior dialysis steps to remove salts in buffers. Reactions were incubated at 50°C in agitation for different reaction times (1, 5, 24 and 48 hours) to monitor cellulose fibers production and sizes *versus* time. Cellulose fiber conversion was determined by weighing the lyophilized cellulose fibers produced in each hydrolysis. Cellulose fiber conversion yields from hydrolysis under different EGs are shown in **Figure 1a.** ANC EG+CBM achieved higher hydrolysis yields in comparison with ANC EG and also modern EGs. Conversion reached around 15% after 24 hours and increased up to 19% after 48 hours. ANC EG+CBM yield was around three times higher in comparison with ANC EG and eight, five and four times higher respect to modern EGs from *T. maritima, B. subtilis* and *T. reesei,* respectively. The low conversion from modern EGs may be due to water used as hydrolysis media. In general, saline buffers are needed for proper enzyme activity. In addition, the temperature of the hydrolysis could limit EG efficiency. Similar temperature to that used for chemical hydrolysis was chosen for better comparison to this treatment.

*Morphology*

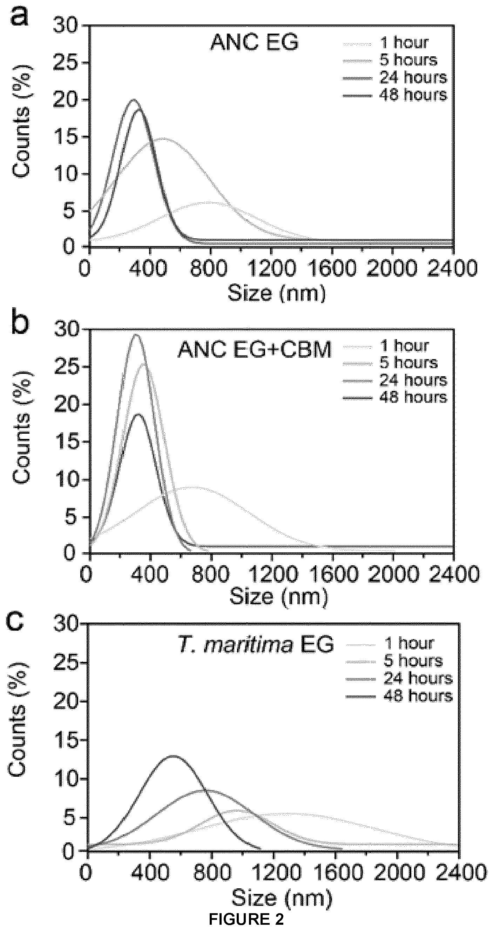

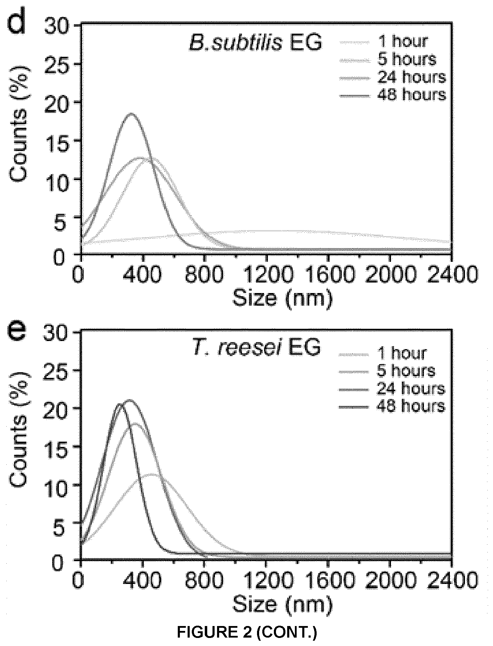

The cellulose fiber morphology was analyzed by Atomic Force Microscopy (AFM) from the suspensions produced during EG hydrolysis (**Figure 1b and Table 1**). After 1 and 5 hours of ANC EG hydrolysis, long thin fibers with lengths around 1-2 $\mu$m and diameters around 30 nm were observed. This morphology is typical of cellulose nanofibers. When hydrolysis was continued for 24 hours, smaller particles with cellulose nanocrystals dimensions, lengths around 540 nm and diameters of 20 nm were observed. ANC EG+CBM hydrolysis produced smaller particles in comparison with ANC EG. At 1 hour hydrolysis cellulose nanofibers and cellulose nanocrystals mixture was observed, and after 24 hours hydrolysis a homogenous cellulose nanocrystals suspension was obtained. These cellulose nanocrystals had an average length of 408 nm and diameters of 12.5 nm. After 48 hours hydrolysis bigger particles in both cases was observed due to cellulose nanocrystals aggregations (**Figure 1c**). Cellulose nanocrystals and cellulose nanofibers produced with this method have no charge in their surfaces and they tend to agglomerate. Enzyme activity started to saturate after 24 hours (**Figure 1a**). EG from *T. maritima* produced bigger particles in length and diameter due to low substrate digestion. *T. reesei* produced similar suspension to ANC EG and *B. subtilis* EG produced particles with size smaller than *T. maritima* EG but bigger than ancestral endoglucanases. The size population distribution from all enzymatic hydrolysis was plotted to analyze the suspension homogeneity by measuring 100 particles of each suspension (**Figure 2**). ANC EG+CBM achieved the more homogenous populations as confirmed by the AFM images (**Figure 1b**). No modern enzyme reached the same homogeneous population reached by ANC EG+CBM after 24 hours hydrolysis (**Figure 2**).

**Table 1.** Average length and diameter and L/D aspect ratio of the cellulose fibers produced at different hydrolysis times with ANC EG, ANC EG+CBM, T. maritima, B.subtillis and T. reesei EGs using filter paper as a cellulose substrate, and the cellulose nanocrystals produced by acid sulfuric treatment. The average and S.E.M (standard error mean) were calculated with the lengths and diameters of 100 particles each condition.

| Enzyme | Time (hours) | Length (nm) | Diameter (nm) | L/D |
|---|---|---|---|---|
| ANC EG | 1 | 1476.8±99.8 | 31.0±1.8 | 47.6 |
| | 5 | 960.0±59.3 | 25.3±1.6 | 37.9 |
| | 24 | 540.8±40.3 | 20.8±1.2 | 26.0 |
| | 48 | 856.3±71.1 | 21.7±1.4 | 39.5 |
| ANC EG+CBM | 1 | 1017.6±49.7 | 23.0±1.6 | 44.2 |
| | 5 | 482.3±14.8 | 19.0±1 | 25.4 |
| | 24 | 408.0±15.1 | 12.5±0.8 | 32.6 |
| | 48 | 933.3±82.2 | 17.5±0.9 | 53.3 |
| T. maritima EG | 1 | 1872.3±244.1 | 40.0±4.5 | 46.8 |
| | 5 | 1919.2±142.5 | 31.3±2.2 | 61.3 |
| | 24 | 1275.7±90.5 | 23.0±1.7 | 55.5 |
| | 48 | 970.0±58.8 | 27.9±2.3 | 34.8 |
| B. subtilis EG | 1 | 1840.0±244.0 | 38.5±5.0 | 57.8 |
| | 5 | 1175.0±140.1 | 25.8±2.0 | 45.5 |
| | 24 | 810.5±70.3 | 20.5±2.1 | 39.5 |
| | 48 | 725.1±61.7 | 21.5±1.8 | 33.7 |
| T. reesei EG | 1 | 1056.3±122.0 | 30.5±4.6 | 34.6 |
| | 5 | 691.0±35.1 | 28.5±2.3 | 24.2 |
| | 24 | 524.3±23.3 | 15.8±1.9 | 33.1 |
| | 48 | 630.7±25.0 | 20.4±2.3 | 30.8 |
| AcCNC | - | 173.9±6.3 | 10.0±0.4 | 17.3 |

The enzymatically obtained cellulose fiber suspension was compared with a commercial sample produced by sulfuric acid hydrolysis (AcCNC). The suspension from the ANC EG+CBM was the most similar to AcCNC (**Figure 1d**).

The morphology of the enzymatically obtained cellulose fibers and AcCNC was further analyzed. AFM images show the different shapes from both samples (**Figure 1e**). Enzymatically obtained cellulose fibers produced after 24 hours with ANC EG+CBM was longer but maintained similar diameters to AcCNC. This difference in size was due to aggressiveness of acid hydrolysis in comparison to enzyme activity. The enzymatically obtained cellulose fiber were typically EnCNC and showed a needle-like morphology due to its high aspect ratio, 32. AcCNC had a ribbon-like and its aspect ratio was 17. Based on these morphologies, reported in the literature from different cellulose polymorphs, EnCNC can be assigned to cellulose I and AcCNC to cellulose II (De Souza Lima, M. M. and Borsali, R., Macromol. Rapid Commun. 2004, 25, 771-787). These results demonstrate that enzymatic treatment does not modify the native cellulose structure of filter paper, whereas acid hydrolysis could affect cellulose chain arrangement, transforming cellulose I to cellulose II.

**Example 2 - Cellulose fiber physicochemical characterization**

The physicochemical properties of the enzymatically obtained cellulose fibers can be determined by the isolation method. To fully characterize these properties an unprecedented arsenal of techniques including Fourier Transform Infrared Spectroscopy (FTIR); nano-FTIR, which combines FTIR and scanning near-filed optical microscopy (s-NON); X-ray Powder Diffraction (XRD); Solid-state Cross Polarization Magnetic Angle Spinning Carbon-13 Nuclear Magnetic Resonance (CP/MAS $^{13}$C NMR) and Thermogravimetric Analysis (TGA) was set up. Using these techniques, EnCNC was compared to both the cellulosic substrate filter paper and commercial AcCNC.

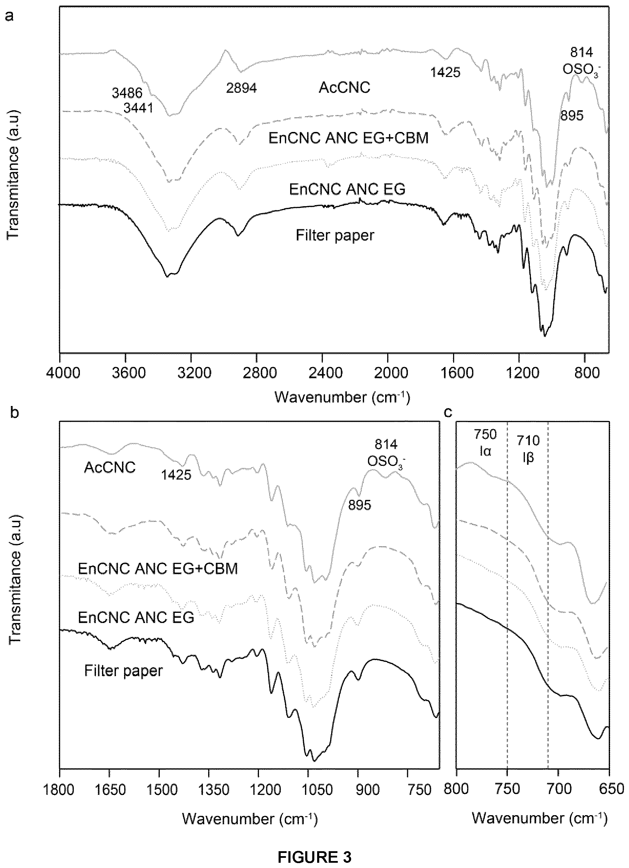

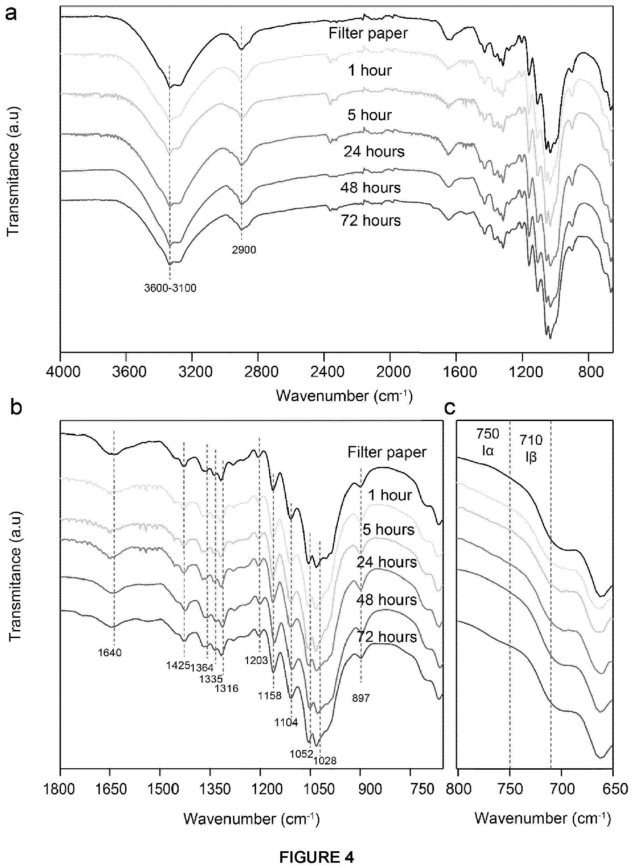

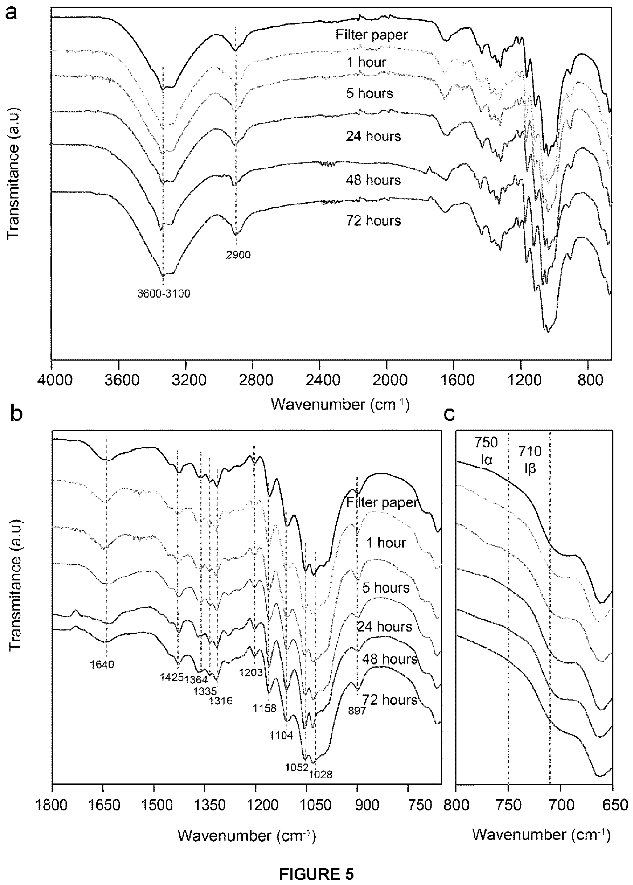

FTIR spectra measurements allow to study cellulose structure and its functional groups (**Figure 3**). Filter paper spectrum shows the typical functional groups from native cellulose. The wide band between 3600 and 3100 cm$^{-1}$ corresponds to -OH groups stretching vibration. At 2900 cm$^{-1}$ appeared the band of -CH and -CH$_2$ groups stretching vibration. The spectrum presents a peak at 1640 cm$^{-1}$ attributed to the bending mode of absorbed water. Also, the spectrum had the characteristic peaks of cellulose at 1425, 1364, 1335, 1316, 1227, 1203 and 1158 cm$^{-1}$. The peak at 897 cm$^{-1}$ is attributed to the C-O asymmetric stretching of β-linkages between glucose monomers, related with cellulose rotation around β-glycosidic linkages. This spectrum is typical of cellulose I polymorph. Enzymatic hydrolysis does not generate any dramatic changes in cellulose structure at different reaction times (**Figure 4 and 5**). The main peaks were maintained in all EnCNC spectra and no band in the Amide II interval around 1540 cm$^{-1}$ is observed, suggesting no protein residues and highly pure nanocellulose samples. Although samples were freeze-dried prior characterization, the water absorption peak is maintained due to strong cellulose-water hydrogen bonding interaction. Both, filter paper and EnCNC spectra show the typical peak of cellulose Iβ at 710 cm$^{-1}$, suggesting that these samples have cellulose Iβ structure. Indeed, the peak at 750 cm$^{-1}$ from Cellulose Iα polymorph is not observed.

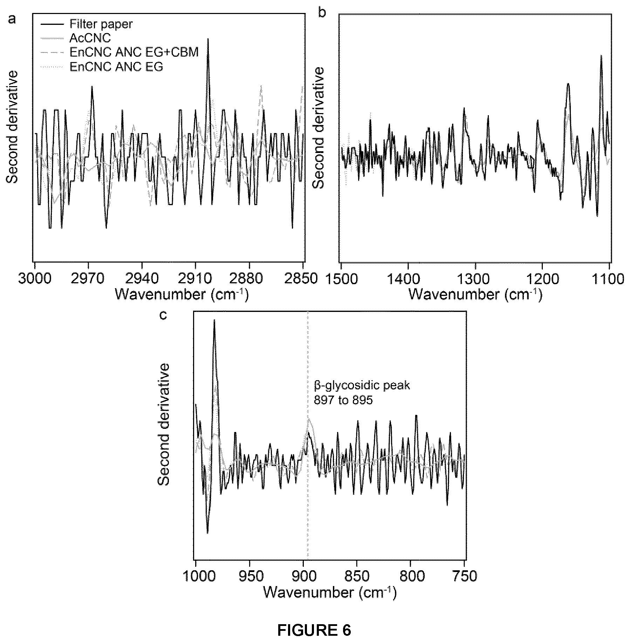

The AcCNC FTIR spectrum (**Figure 3**) presents some differences, two small shoulders can be observed at 3486 cm$^{-1}$ and 3441 cm$^{-1}$ assigned to the intra-molecular hydrogen bonding in the allomorph cellulose I. The peak at 2894 cm$^{-1}$ was sharper in AcCNC sample in comparison with EnCNC, this shape is typical from crystal structure of cellulose II. The band at 1425 cm$^{-1}$ that corresponds to -CH$_2$ bending was smaller in AcCNC indicating new inter and intra molecular hydrogen bonds. In addition, a shift in the β-glycosidic peak was observed, in EnCNC and filter paper appeared at 897 cm$^{-1}$, while in the spectra of AcCNC it appeared at 895 cm$^{-1}$, and was sharper than that of EnCNC. This alteration is typical from cellulose II. A second derivative analysis from FTIR spectra was performed for further analysis (**Figure 6**), in the range from 3000 to 2850 cm$^{-1}$, some shifts in EnCNC spectra in comparison to AcCNC sample where observed, due to new inter and intra molecular interactions and small torsion in the angle of the β-glycosidic bonds of AcCNC. Moreover, in the range from 1000 to 750 cm$^{-1}$, the shift to 895 cm$^{-1}$ of the β-glycosidic peak in AcCNC sample was observed. The spectra presented similar pattern in the range from 1500 to 1100 cm$^{-1}$. These differences corroborate the changes in cellulose chain arrangement between polymorphs.

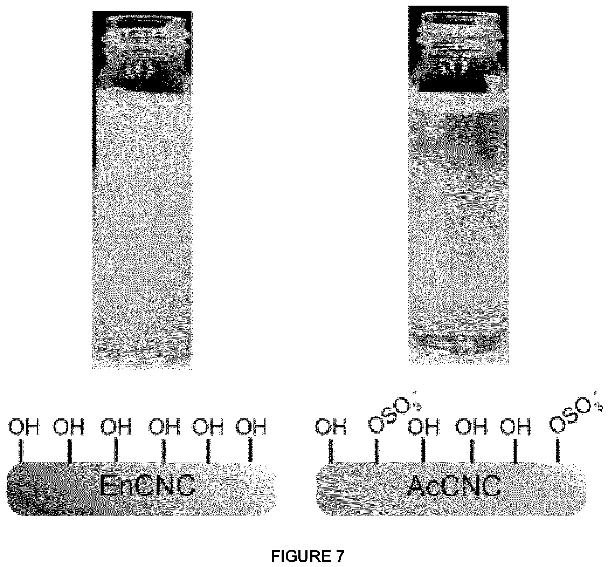

A typical chemical signature of AcCNC is sulfate groups from the acidic hydrolysis that could be observed in the spectrum as a peak at 814 cm$^{-1}$ (**Figure 3**). Sulfuric acid hydrolysis disrupts the cellulose structure degrading the original parallel chain crystalline structure from cellulose I to an antiparallel orientation in cellulose II. This sulfate groups are anchored in the nanocrystals surface during hydrolysis by an esterification reaction. The percentage of Sulphur in AcCNC was quantified by conductometric titration, measuring 0.95±0.04 sulfur content, in agreement with the 0.94% reported by the manufacturer. Sulfate groups have a direct impact in the physicochemical properties of nanocrystals. For example, the water dispersability observed for each EnCNC and AcCNC suspension was different due to the functional groups in the surface of the hydrolyzed cellulose substrates (**Figure 7**). Without wishing to be bound by any particular theory, it is believed that the -OH groups of the EnCNC surface induce interaction between the cellulose hydrolyzed material by hydrogen bonding, which translates into an opaque solution. The negatively charged OSO$_3^-$ groups in the AcCNC surface leads to a stable dispersion in water, probably due to electrostatic repulsion forces between crystals.

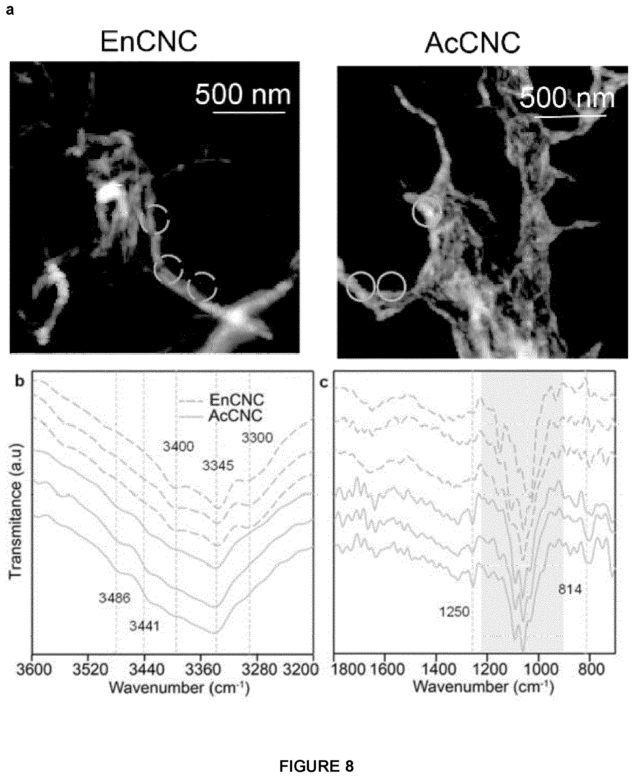

The infrared absorption was also investigated using nano-FTIR. The infrared absorption of an EnCNC suspension was measured and different spectra from different nanocrystals were generated. **Figure 8a** shows an AFM image of an EnCNC suspension prepared after 5 hours of hydrolysis of ANC EG+CBM and of a commercially available AcCNC suspension (hydrolysis with H$_2$SO$_4$). The aim was to find an area that contained crystals of different sizes to see if size affects nano-FTIR resolution. The circles drawn in **Figure 8a** mark the crystals selected for the FTIR measurements and each spectrum is shown in **Figure 8b and c**. Larger crystals spectra have better resolution than the smaller ones. In all cases two prominent peaks were observed at 1158 cm$^{-1}$, associated to the C-O-C stretching of the β-1,4-glycosidic linkage, and the 1052 cm$^{-1}$ band that corresponds to C-O bending peak.

*Crystallinity*

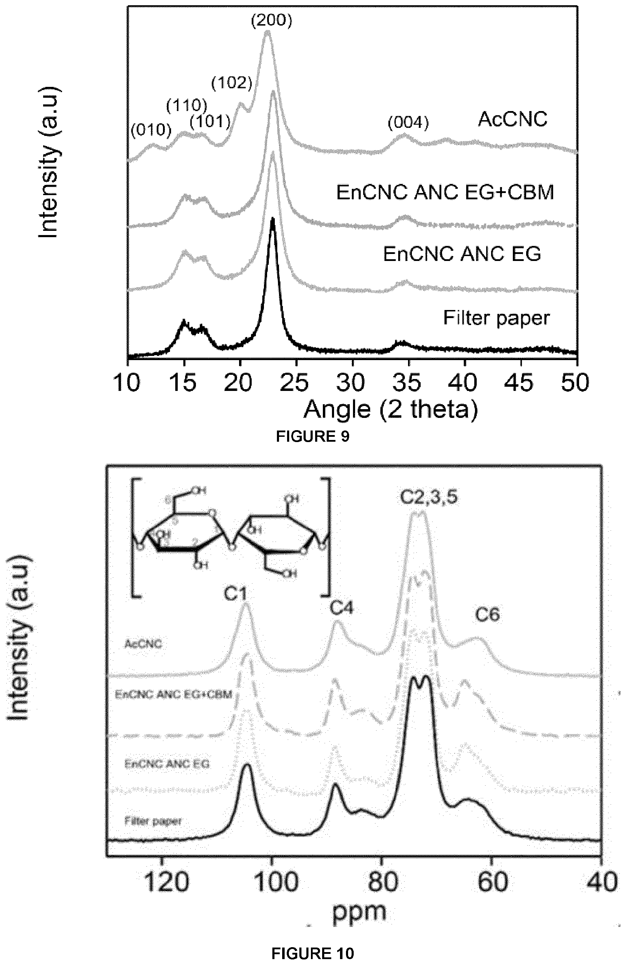

One of the most important features of cellulose nanocrystals is its crystallinity because it is correlated to its thermal and mechanical properties. XRD was used to analyze cellulose nanocrystals crystal structure and determine the Crystallinity Index (CI) (**Figure 9**), and compare the crystallinity between different cellulose nanocrystals types. From the diffractograms in **Figure 9**, CI and crystallite size were calculated. It was observed that filter paper diffractogram presented the classical crystallography pattern from cellulose I. At 22.9° appeared a sharp peak that corresponded to (200) plane and three smaller ones at 15°, 16.5° and 34° attributed to (110), (101), (004) planes, respectively. After 24 hours of enzymatic hydrolysis with ANC EG, no change in the diffractogram pattern is observed as the (110), (101), (004) and (200) were in the same position. Enzymatic treatment keeps the native cellulose structure, in agreement with the above disclosed FTIR analysis. It was found that the AcCNC pattern was different, the plane (200) is shifted to 22.3°, new peaks appear at 19.9° and 12.3° attributed to (102) and (010) planes, respectively. These two peaks are produced by transformation of crystalline structure of cellulose I to cellulose II. The two peaks at 15° and 16.5° are maintained from (110) and (101) plane but less intense, and (004) plane at 34° is sharper. This diffractogram pattern has been attributed to a mixture of cellulose I and II. It was determined that filter paper has a CI% of 92.5% and a crystallite size of 6.5 nm (**Table 2**). EnCNC samples presented a CI% of 85.0% with ANC EG and 87.9% with ANC EG+CBM, and around 5 nm of crystallite size. The reduction in both parameters in comparison with filter paper may be caused by the freeze-dried process as well as small disruption in cellulose structure during EG treatment. AcCNC sample has a CI% of 80.5% and a crystallite size of 4.1 nm. Sulfuric acid treatment produced a partial transformation from cellulose I to cellulose II, forming a less crystalline polymer by breaking cellulose crystalline structure.

**Table 2.** Crystallinity index (CI%) and crystallite size of the filter paper, EnCNC produced by 24 hours hydrolysis by ANC EG and ANC EG-CBM and AcCNC produced by sulfuric acid hydrolysis. The CI% was calculated by Segal method and the Scherrer's equation was used for the crystallite size.

| Enzyme | CI (%) | Crystallite size (nm) |
|---|---|---|
| Filter paper | 92.5 | 6.5 |
| EnCNC ANC EG | 85.0 | 4.9 |
| EnCNC ANC EG+CBM | 87.9 | 5.1 |
| AcCNC | 80.5 | 4.1 |

To further investigate the changes in cellulose nanocrystals observed in FTIR and XRD analysis, CP/MAS $^{13}$C NMR was used, which permits to study the lignocellulose biomass, the cellulose structure and the effect that different degradation treatments have. All the cellulose nanocrystals samples analyzed present the same cellulose pattern (**Figure 10**). The peak between 60 and 70 ppm corresponds to C6 of the primary alcohol group. Peaks between 70 and 80 ppm are assigned to the carbons C2, C3, and C5. The peak between 80 and 95 ppm is attributed to C4 and the peak from 100 to 110 ppm is the C1 anomeric carbon. These features appears in both crystalline and amorphous components of cellulose. In both EnCNC samples, C6 peak was sharp and appeared at 65 ppm, typical in cellulose I. C6 peak in AcCNC sample was shifted to 63 ppm, a feature from cellulose II. There is a small shift between the C4 positions, the peak appears at 89 ppm in EnCNC and filter paper, and corresponds to cellulose I. In AcCNC, this peak is found at 88 ppm, which is the position in cellulose II. Also, in EnCNC spectra the C4 band is sharper; this means that the anhydroglucose units is in a very ordered environment attributed to the crystalline cellulose region. The shoulder at 84 ppm is assigned to a more heterogeneous distribution in the structural order; this is typical of the anhydroglucoses located at the surface. The reduction in the areas of C1 and C6 in AcCNC can be associated to the lower crystallinity. These results match with the XRD data and confirm the partial transformation to cellulose II in AcCNC.

*Thermal stability*

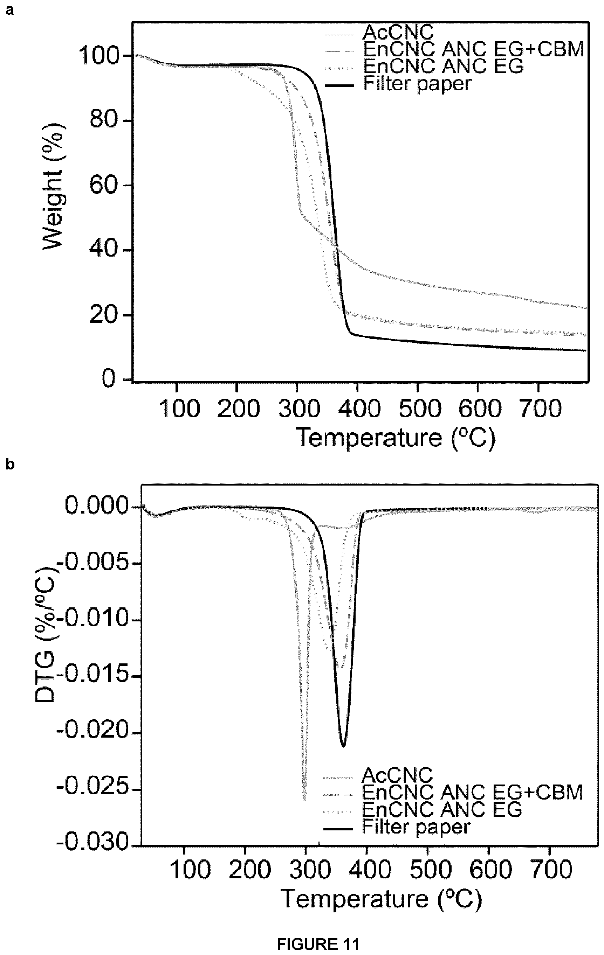

After determining the chemical and structural features of EnCNC the thermal stability was investigated. The thermal stability can be determined using TGA analysis, which provides a measure of the weight loss (**Figure 11**). The derivatives of the thermogravimetric curves (DTG) for filter paper, EnCNC and AcCNC are shown in **Figure 11b.** In **Table 3**, the onset degradation temperature (To), the maximum degradation temperature (Td) and char weight (%) are shown. All the samples present a weak weight loss around 100 °C that corresponds to water evaporation that was absorbed by the material. Filter paper has the classical degradation behavior of native cellulose, starting at 311 °C (To) with maximum degradation temperature at 361 °C, as observed in the DTG curve. EnCNC samples show a different thermal behavior. EnCNC produced by ANC EG has a To of 210 °C and a Td of 337 °C. In the case of EnCNC produced by ANC EG+CBM a higher To and Td, 262 and 356 °C, respectively, were found. This variation could be explained by the different catalytic action between EGs. ANC EG acts randomly in the fiber in comparison with ANC EG+CBM that is processive. This translates into a more heterogeneous length population with ANC EG in which larger particles have more amorphous regions. Nevertheless, it is worth noting that EnCNC obtained by hydrolysis with ANC EG+CBM has nearly the Td of the substrate filter paper. This contrasts with the thermal stability of AcCNC which has a To of 269 °C and a Td of 298 °C. Although it has been described that cellulose II is more thermostable than cellulose I, the amount of sulfate groups on the crystal surface of AcCNC clearly decreases the thermal stability. The lower crystallinity and polymerization degree of AcCNC also likely play a role in the lower To and Td.

**Table 3**. Onset degradation temperature (To), maximum thermal degradation temperature (Td) and char residue (%) of the filter paper, EnCNC produced by 24 hours hydrolysis with ANC EG and ANC EG+CBM, and AcCNC produced by sulfuric acid hydrolysis.

| Enzyme | To (°C) | Td (°C) | Char residue (wt%) |
|---|---|---|---|
| Fitter Paper | 311 | 361 | 9.2 |
| EnCNC ANC EG | 210 | 337 | 14.2 |
| EnCNC ANC EG+CBM | 262 | 356 | 13.9 |
| AcCNC | 269 | 298 | 22.2 |

Overall, these results show that the enzymatic method maintain native cellulose structure as well as its properties. EnCNC show slightly lower crystallinity and thermal stability than those of filter paper used as substrate. Sulfuric acid hydrolysis had a stronger effect in cellulose structure, partially transforming cellulose chain arrangement, degrading the cellulose crystalline structure, and even changing the functional groups on the crystal surface by anchoring sulfate groups that seems to reduce the AcCNC thermal stability.

**Example 3 - Fabrication of exemplary cellulose composite materials**

The cellulose fibers used herein were prepared as disclosed in the last two examples.

The inventors have found that the enzymatically obtained cellulose fibers of the invention (EnCNC) form stable matrices in which the physical properties of graphene combine with those of EnCNC, generating an advanced hybrid material suitable for novel biocompatible devices.

Thus, nanopapers with EnCNC obtained by hydrolysis with ANC EG+CBM (as disclosed in the previous examples) and with commercial AcCNC were prepared.

The nanopapers were prepared by solvent casting method using a 1 wt% EnCNC suspension. The suspension was sonicated for 1 hour and then casted in a Teflon mold and dried for two days at 37 °C.

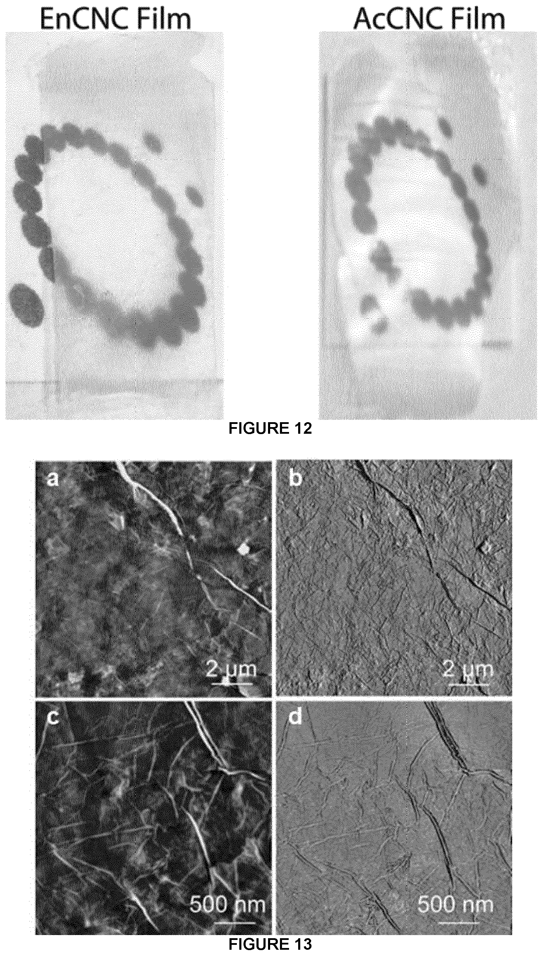

A thin film was obtained from EnCNC suspension with very good integrity and transparency; however, while the AcCNC film maintained good transparency (**Figure 12**), its integrity was very weak. The stability of the films in water was evaluated to observe if nanopapers could hold their shape after immersion in water. The EnCNC film maintained its shape and did not disintegrate. Conversely, comparative the AcCNC film dissolved in water after a few seconds of immersion. After one minute, the AcCNC films were undetectable in water.

Prior to mixing EnCNC with the GO suspension, AFM was used to reveal the morphology of GO suspension in water. The size of graphene sheets and how they are structured was thus analyzed (**Figure 13**). It was observed that graphene particles have a size of around 1 $\mu$m and show a tendency to stack together composing a film. The phase images show the edges of each sheet.

The conductive nanopapers were produced by mixing graphene oxide (GO) with EnCNC and its subsequent reduction once the paper was assembled. Graphene was integrated in EnCNC by preparing water suspensions comprising EnCNC as a matrix and graphene oxide (GO) in different relative proportions, 2, 5 and 10 wt%. EnCNC and GO were mixed by sonication for 1 hour and then casted in Teflon molds and dried for two days at 37 °C, obtaining films with a thickness around 100 $\mu$m. The GO in the nanopapers was then reduced by submerging the films in an ascorbic acid solution (30 g/l) for 2 hours at 95 °C. The reduced films were washed with miliQ water for ascorbic acid removal and dried out at room temperature overnight. The mixing was made with GO suspension because it is easier to disperse in water than rGO due to its hydrophobic nature.

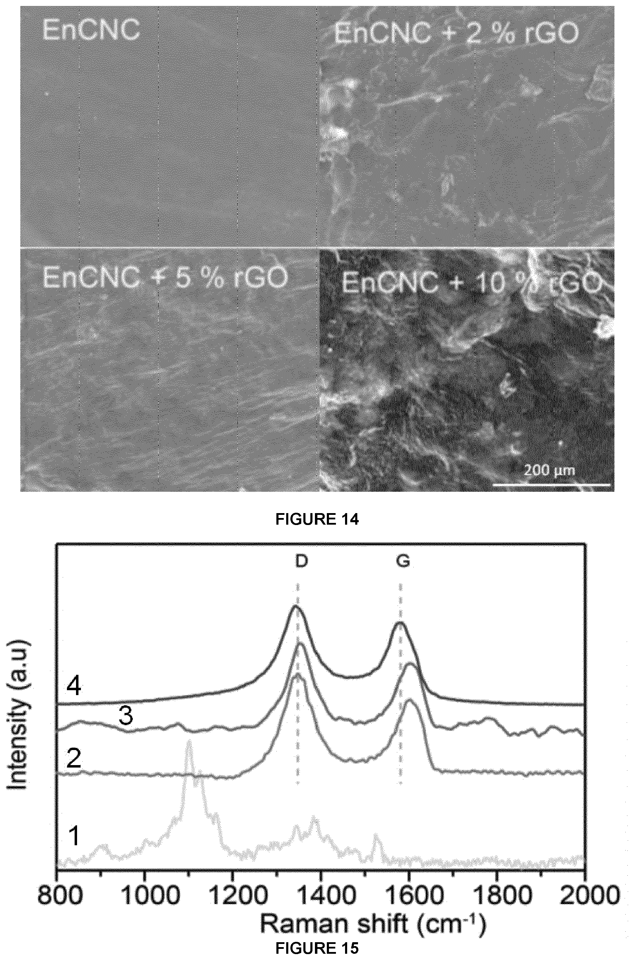

To analyze the effect of rGO integration in the morphology of the material, Scanning Electron Microscopy (SEM) images of the nanopapers were taken (**Figure 14**). It was observed that the roughness of the surface increased with rGO content. This effect was more visible in the 10 wt% rGO nanocomposites where the highest heterogeneous surface was observed.

Graphene sheets stacked together during the drying process. Raman spectroscopy was used to analyze structural changes in the EnCNC rGO films (**Figure 15**). EnCNC spectra show the typical peaks from cellulose, the main peaks in the spectrum were at 1025 cm$^{-1}$ from C-O-C stretching vibration. In the samples with rGO, the D band was visible around 1350 cm$^{-1}$ and assigned to graphene sheet defects and G band around 1587 cm$^{-1}$ related to the order structure of sp$^2$ hybridized carbon atom of graphene. The ratio between the intensity of D band over the G band (ID/IG) is around 1.3 for all the samples, suggesting that graphene structure presented defects. Also, it was observed a shift in D and G bands on the 2 and 5 wt% rGO nanopapers respect to 10 wt% rGO sample, this could mean that in the 10 wt% rGO nanopaper, graphene reached a more ordered structure in comparison with the 2 and 5 wt% rGO samples. In addition, this shift suggests that GO reduction may have been only partial and this is more evident in the 10 wt% sample due to the higher concentration.

FTIR spectra from GO, rGO, EnCNC film and EnCNC films with different rGO contents were taken to study the physicochemical interaction between rGO and EnCNC (**Figure 16**). In the GO spectrum, a broad band from 3600 to 2500 cm$^{-1}$ assigned to -OH stretching vibration is observed. The peak at 1726 cm$^{-1}$ was attributed to the stretching vibrations of C=O carboxylic groups and the 1616 cm$^{-1}$ peak corresponded to C=C aromatic bond. The peaks from -OH and C=O were decreased in rGO spectrum after ascorbic acid incubation, confirming partial reduction of GO as the peak of C=O did not fully disappear. The main peaks from EnCNC films were present in the films with rGO but less intense. A loss in the intensity of the peak at 3340 cm$^{-1}$ assigned to -OH group stretching vibration of cellulose was observed, as rGO content increased. Also, the water absorption peak at 1640 cm$^{-1}$ is reduced in the films with rGO content due to the hydrophobic behavior of the material. To further analyze the rGO effect in the nanocellulose structure, a second derivative analysis was conducted, from the spectra of EnCNC film and EnCNC film with 10 wt% rGO content at different wavenumber intervals. It was observed that the spectrum of EnCNC film with 10 wt% rGO content was shifted with respect to the EnCNC film spectrum in the interval between 3400 and 3200 cm$^{-1}$, confirming the change in the -OH stretching vibration groups. The spectra in the area from 1500 to 1100 cm$^{-1}$ were very similar, but the β-glycosidic peak at 897 cm$^{-1}$ varied due to graphene disposition between the cellulose chains or by new interactions between rGO and EnCNC.

An important new feature that rGO gave to the EnCNC film was electric conductivity. The conductivity of the prepared films was measured (**Figure 17 and Table 4**) and an increment in the conductivity values with rGO content was observed. The conductivity behavior was due to the chemical reduction of GO as Raman and FTIR analysis demonstrated. The measured conductivity of EnCNC film was 0 because cellulose is an insulator if not modified. The conductivity in the 2 wt% rGO film was 5.1 S m$^{-1}$ and was increased in the 5 and 10 wt% rGO films, reaching a plateau of 25.2 S m$^{-1}$.

**Table 4**. EnCNC film and EnCNC films with different rGO concentrations. Conductive properties (average values and S.D. from three experiments); Mechanical properties (Young's modulus, Tensile Strength and Strain at break, average values and S.D. from five experiments); and Thermal properties (onset degradation temperature (To), maximum thermal degradation temperature (Td) and char residue (%)).

| Sample | Conductivity (S/m) | Young's modulus (MPa) | Tensile strength (MPa) | Strain at break (%) | To (°C) | Td (°C) | Char residue (wt%) |
|---|---|---|---|---|---|---|---|
| EnCNC film | 0 | 2852.3±325.2 | 42.9±5.1 | 3.1±0.8 | 270 | 333 | 23.7 |
| EnCNC film + 2% rGO | 5.1±1 | 1800.6±255.3 | 38.7±2.7 | 2.3±0.3 | 275 | 332 | 25.8 |
| EnCNC film + 5% rGO | 21.4±1.4 | 1500.6±145.8 | 41.0±3.5 | 5.0±0.4 | 270 | 330 | 28.8 |
| EnCNC film + 10%rGo | 25.2±2 | 4000.1±179.2 | 49.6±8.3 | 2.6±0.1 | 270 | 322 | 30.1 |

**Mechanical properties of EnCNC/rGO**

The mechanical properties of EnCNC/rGO were also analyzed. A tensile strength of 42.9 MPa and a Young's modulus of 2852.3 MPa (**Figure 17b and Table 4**) was determined for the EnCNC film. When the rGO content increased, differences were observed in the mechanical behavior. The composite material's tensile strength decreased for films containing 2 and 5 wt% rGO and increased at the highest rGO concentration, reaching 49.6 MPa in the EnCNC film with 10 wt% rGO content (**Table 4**). The Young's modulus changed similarly with rGO addition. In the films with 2 and 5 wt% content, a reduction in the modulus was observed. In EnCNC film with 10 wt% rGO content, an enhancement in Young's modulus with respect to EnCNC film was observed, a 40% increment.

Regarding strain at break values, a decrease was observed for the EnCNC film containing 2wt% rGO, compared to EnCNC alone, which could be attributed to the removal of the oxygenated functional groups involved in hydrogen bonds with CNC during the reduction process, weakening the nanopaper assembly. EnCNC film with 5 wt% rGO concentration showed higher strain at break value, suggesting that effective hydrogen bonds could still be formed. Moreover, a peak related to C=O was observed in the FTIR spectrum of EnCNC + 5% rGO. EnCNC film with 10 wt% rGO content showed lower value in comparison with the EnCNC film with 5wt% rGO and although a small peak related to oxygenated functional groups that contributed to improve the strength could be seen in the FTIR spectrum, the further addition of a stiff nanoentity as the Young's modulus increment suggested it did not contribute further to improve the strain at break.

## Hydrophobicity of the hybrid materials

The hydrophobicity of the hybrid materials was determined using Water Contact Angle technique (WCA) (**Figure 17c**). Surprisingly, the EnCNC displays a high contact angle, indication of hydrophobic character, which is in contrast with the lower contact angles reported for other CNC films. In addition, the contact angle further increased with the addition of rGO.

## Thermal performance

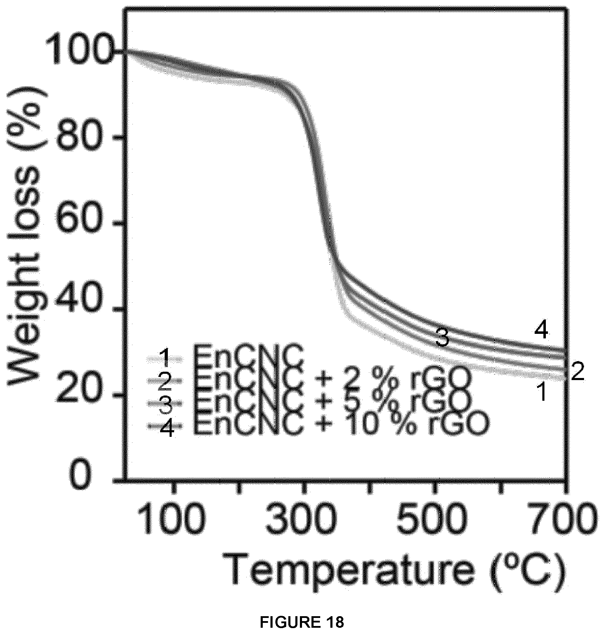

The addition of rGO does not change the thermal performance of EnCNC **(Figure 18).** These results are indication that the conductive nanopapers of the invention could be used in high temperatures applications where plastic materials fail.
The thermal stability of EnCNCs, measured by TGA analysis, was higher than that of the AcCNC sample (**Table 3**).

## Example 4

## CVD synthesis

Apart from fabricating a mixture of the cellulose fibers and rGO, the inventors have also fabricated composite materials according to the invention by transferring graphene sheets synthesized by Chemical Vapor Deposition (CVD) onto pre-fabricated EnCNC films.
To this end, monolayer and bilayer graphene was grown on copper (Cu) foils and deposited by CVD. The graphene synthesis was carried out in a cold walled CVD reactor (Aixtron BM) at 1000 °C and low-pressure using methane as the carbon source. Prior to the growth the Cu foils were annealed at 1000 °C under a hydrogen and argon flow. After the synthesis, a poly(methyl methacrylate) (PMMA) support layer was spin coated onto the graphene covered Cu foil. The Cu was etched using an iron chloride solution and the graphene monolayer or bilayer transferred onto the cellulose film. Finally, the PMMA layer was removed by dipping into acetone and isopropanol.
Similar materials could not be prepared with AcCNC because this substrate dissolves in water and hydrophobicity is required for the transfer step.

## Material characterization

A graphene monolayer over EnCNC (EnCNC ML) was hard to see with naked eye due to its high transparency. In the graphene bilayer sample (EnCNC BL), the layer was visible, despite maintaining good transparency.

Spectroscopy

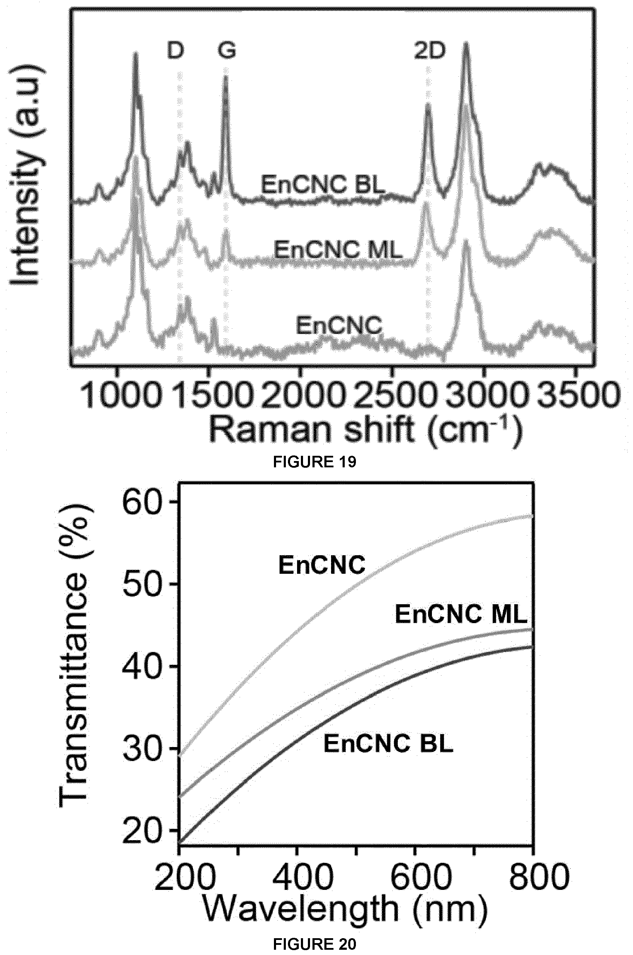

To confirm that the graphene layers were successfully transferred, the EnCNC, EnCNC ML and EnCNC BL films were submitted to Raman spectroscopy (**Figure 19**). Cellulose peaks described previously were present in both EnCNC ML and EnCNC BL films. Typical peaks of graphene were also observed. In both cases, the 2D band around 2690 cm$^{-1}$ and the G band around 1570 cm$^{-1}$ from graphene was observed. The presence of graphene characteristic bands confirmed the successful transfer of both mono and bilayers on top of the EnCNC substrate, highlighting the good integration over the substrate as graphene maintained its order structure. The ratio between 2D and G bands (I2D/IG) varies with graphene layer accumulation. The ratio calculated for EnCNC ML was 1.7 and for EnCNC BL it was 0.8. These ratios are assigned to single and two layers respectively.
The UV-Vis transmittance of the fabricated samples was measured in order to quantify the loss of transparency (**Figure 20**). The EnCNC film shows a transmittance of 44% at 400 nm. Upon the addition of the graphene layers, the transmittance decreases to 34% (EnCNC ML) and further down to 30% (EnCNC BL) due to the extra layer stacking.

## Electrical response

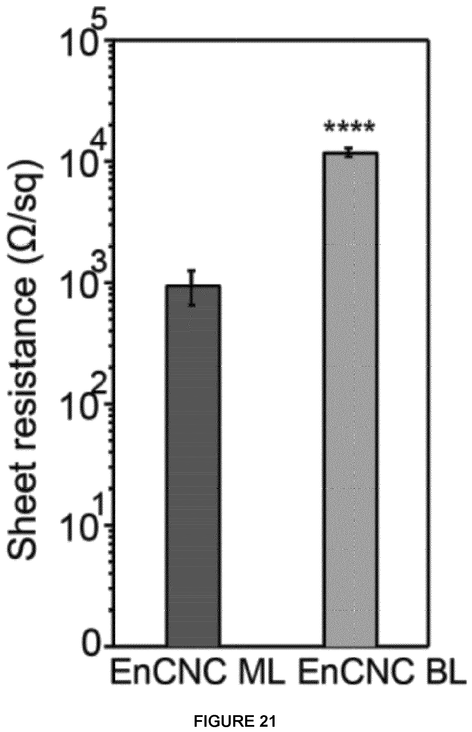

Graphene sheets hold high conductive properties due to their ordered structure which can be improved by sheet layering (**Figure 21**). EnCNC ML showed a resistivity of $949\pm300\ \Omega$ per sq, a value that increases up to $11873\pm300\ \Omega$ per sq in EnCNC BL.

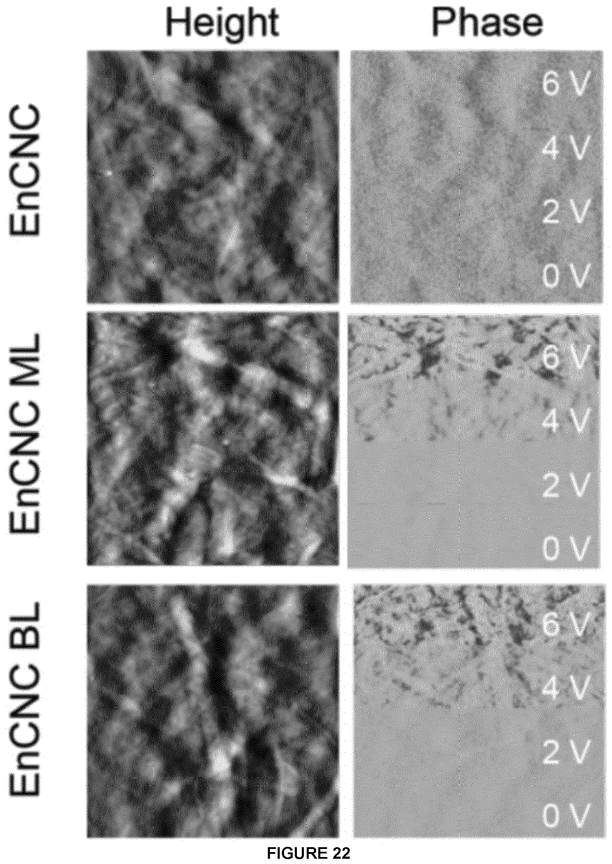

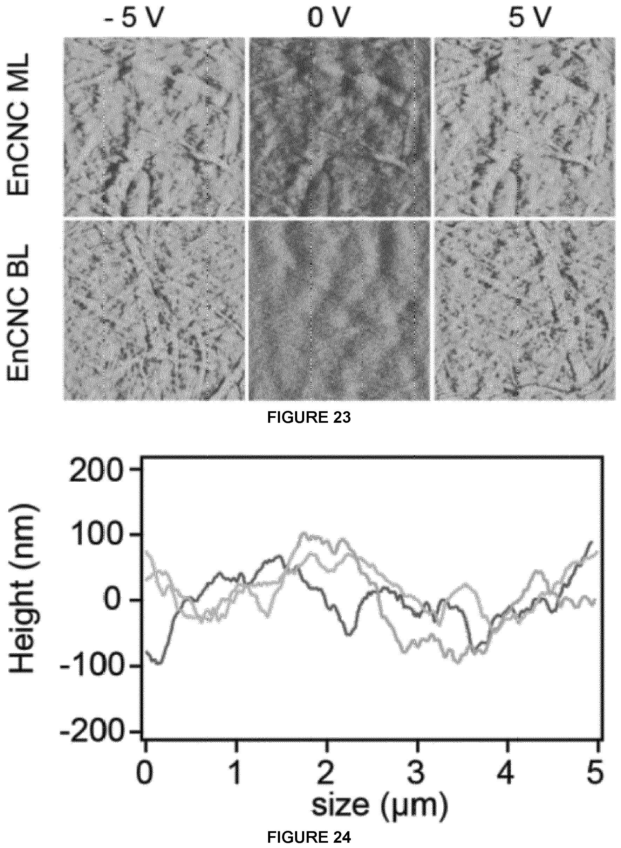

The electrical response of the surface was studied by means of EFM imaging of the films with CVD graphene monolayers and bilayers (**Figure 22**). The topography of both EnCNC ML and EnCNC BL was similar to that of EnCNC film. No changes in the topography were observed in EFM phase images, when different bias voltage was applied between the conductive AFM tip and the sample (0, 2, 4, 6 V). Resolution and detail increased with voltage due to higher conductive response of CVD layers. Phase images with a constant voltage were taken at 5, 0 and -5 V, of both EnCNC ML and EnCNC BL (**Figure 23**). They show that the material was uniformly conductive. It seems that both graphene mono and bilayer were deposited uniformly over the film, achieving a good integration with EnCNC. When 0 V voltage was applied, the same response was not observed as when voltage was applied, thus confirming the conductive response of the CVD prepared materials. When -5 V were applied, a lower intensity was observed. Conversely, when 5 V were applied, the response was brighter. This is typical of positively charge substrates where induced polarization effects are lower than Coulomb interactions. The topography does not change in either condition **(Figure 24).** The topography profile measured in the EFM height images showed a similar pattern, highlighting the good contact and adhesion of graphene over the EnCNC film.

**Claims**

1. A cellulose composite material comprising:

   a. Type I cellulose fibers selected from the group consisting of cellulose nanocrystals, cellulose nanofibers and a mixture thereof; and
   b. A carbon material selected from the group consisting of carbon nanotubes, carbon nanofibers, graphene, graphene oxide and a mixture thereof.

2. The cellulose composite material according to claim 1, wherein the cellulose fibers are cellulose nanocrystals and wherein the carbon material is graphene, graphene oxide or a mixture thereof.

3. The cellulose composite material according to any one of claims 1 to 2, wherein the cellulose fibers are obtained by hydrolyzing a substrate comprising cellulose with an enzyme having endoglucanase activity.

4. The cellulose composite material according to claim 3, wherein the enzyme having endoglucanase activity comprises an endoglucanase catalytic domain comprising a sequence selected from the group consisting of SEQ ID NO: 1, SEQ ID NO: 2, SEQ ID NO: 3 and a functionally equivalent variant thereof that substantially maintains or improves its catalytic activity.

5. The cellulose composite material according to any one of claims 1 to 4, wherein the cellulose fibers have an intrinsic net charge of zero.

6. The cellulose composite material according to any one of claims 1 to 5, wherein the composite material is an electrically conductive composite material.

7. The cellulose composite material according to any one of claims 1 to 6, wherein the cellulose fibers and the carbon material are homogeneously distributed within the composite material.

8. The cellulose composite material according to any one of claims 1 to 6, wherein the composite is a layered composite material comprising a matrix of cellulose fibers and at least one layer of said carbon material on top of at least a first surface of said matrix.

9. A method for the preparation of the cellulose composite material of any one of claims 1 to 7, comprising the steps of:

   a. preparing a suspension comprising type I cellulose fibers selected from the group consisting of cellulose nanocrystals, cellulose nanofibers and a mixture thereof, and a carbon material selected from the group consisting of carbon nanotubes, carbon nanofibers, graphene, graphene oxide and a mixture thereof;
   b. mixing the suspension; and
   c. optionally reducing said carbon material in the mixture of the previous step.

10. A method for the preparation of the cellulose composite material of any one of claims 1 to 6 or claim 8, comprising the step of:

    a. transferring at least one layer of a carbon material selected from the group consisting of carbon nanotubes, carbon nanofibers, graphene, graphene oxide and a mixture thereof onto a matrix of type I cellulose fibers selected from the group consisting of cellulose nanocrystals, cellulose nanofibers and a mixture thereof to form a layered composite material.

11. The method according to any one of claims 9 to 10, wherein the cellulose fibers are prepared by hydrolyzing a substrate comprising cellulose with an enzyme having endoglucanase activity.

**12.** The method according to claim 11, wherein the enzyme having endoglucanase activity comprises an endoglucanase catalytic domain comprising a sequence selected from the group consisting of SEQ ID NO: 1, SEQ ID NO: 2, SEQ ID NO: 3 and a functionally equivalent variant thereof that substantially maintains or improves its catalytic activity.

**13.** A cellulose composite material obtainable by the method according to any one of claims 9 to 12.

**14.** Use of the cellulose composite material according to any one of claims 1 to 8 or claim 13 in dyes, including conductive dyes or paints, electronics, optoelectronics, transistors, touch screens, medicine, biomedicine, tissue engineering, health diagnostics, sensors and biosensors, food security, packaging, textiles, including electronic textiles, labels, radio frequency tags, automotive industry, energy industry including production and storage and construction materials.

**15.** Conductive paint composition, comprising:

    a. the cellulose composite material according to any one of claims 1 to 8 or claim 13;
    b. a polymer binder; and
    c. a carrier solvent.

FIGURE 1

FIGURE 1 (CONT.)

FIGURE 2

FIGURE 2 (CONT.)

FIGURE 3

**FIGURE 4**

FIGURE 5

FIGURE 6

FIGURE 7

FIGURE 8

FIGURE 9

FIGURE 10

a

b

FIGURE 11

FIGURE 12

FIGURE 13

FIGURE 14

FIGURE 15

FIGURE 16

FIGURE 17

FIGURE 18

FIGURE 19

FIGURE 20

FIGURE 21

FIGURE 22

FIGURE 23

FIGURE 24

Europäisches
Patentamt

European
Patent Office

Office européen
des brevets

## EUROPEAN SEARCH REPORT

Application Number

EP 20 38 2140

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MARIA M. PÉREZ-MADRIGAL ET AL: "Powering the future: application of cellulose-based materials for supercapacitors", GREEN CHEMISTRY, vol. 18, no. 22, 1 January 2016 (2016-01-01), pages 5930-5956, XP055697295, GB ISSN: 1463-9262, DOI: 10.1039/C6GC02086K * figures 3,4; table 3 * | 1-15 | INV. C12N9/42 C12P7/10 B82Y30/00 C01B32/20 C08K3/04 D21H11/18 D21H13/50 |
| X | US 2016/340520 A1 (KONAGAYA SHIGEJI [JP] ET AL) 24 November 2016 (2016-11-24) * claim 1 * | 1-15 | |
| X | WO 2013/076372 A1 (TEKNOLOGIAN TUTKIMUSKESKUS VTT OY [FI]) 30 May 2013 (2013-05-30) * claims 1-4; figure 4c; example 1 * | 1-15 | |
| X | WO 2015/200232 A2 (API IP HOLDINGS LLC [US]) 30 December 2015 (2015-12-30) * paragraph [0170]; claims 1,20 * | 1-15 | TECHNICAL FIELDS SEARCHED (IPC) |
| A | EP 3 192 866 A1 (CIC NANOGUNE - ASOCIACIÓN CENTRO DE INVESTIGACIÓN COOP EN NANOCIENCIAS) 19 July 2017 (2017-07-19) * sequences 2,3,45 * | 1-15 | C12N C12P D21H C08K C01B B82Y |
| A | EP 3 502 126 A1 (CIC NANOGUNE ASOCIACION CENTRO DE INVESTIG COOPERATIVA EN NANOCIENCIAS) 26 June 2019 (2019-06-26) * sequences 11,12,22 * | 1-15 | |

-/--

The present search report has been drawn up for all claims

| Place of search | Date of completion of the search | Examiner |
|---|---|---|
| Munich | 27 May 2020 | Griesinger, Irina |

EPO FORM 1503 03.82 (P04C01)

Europäisches
Patentamt

European
Patent Office

Office européen
des brevets

# EUROPEAN SEARCH REPORT

Application Number

EP 20 38 2140

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GUNNAR HENRIKSSON ET AL: "Monocomponent endoglucanase treatment increases the reactivity of softwood sulphite dissolving pulp", JOURNAL OF INDUSTRIAL MICROBIOLOGY & BIOTECHNOLOGY ; OFFICIAL JOURNAL OF THE SOCIETY FOR INDUSTRIAL MICROBIOLOGY, SPRINGER, BERLIN, DE, vol. 32, no. 5, 1 May 2005 (2005-05-01), pages 211-214, XP019357727, ISSN: 1476-5535, DOI: 10.1007/S10295-005-0220-7 * page 212, paragraph 1; figure 1 * | 1-15 | |
| A | QING YAN ET AL: "A comparative study of cellulose nanofibrils disintegrated via multiple processing approaches", CARBOHYDRATE POLYMERS, APPLIED SCIENCE PUBLISHERS, LTD. BARKING, GB, vol. 97, no. 1, 4 May 2013 (2013-05-04), pages 226-234, XP028568606, ISSN: 0144-8617, DOI: 10.1016/J.CARBPOL.2013.04.086 * page 231, right-hand column, paragraph 1 * | 1-15 | |
| A | ISTVAN SIRO ET AL: "Microfibrillated cellulose and new nanocomposite materials: a review", CELLULOSE, vol. 17, no. 3, 21 February 2010 (2010-02-21), pages 459-494, XP055068118, ISSN: 0969-0239, DOI: 10.1007/s10570-010-9405-y * page 466, last paragraph * | 1-15 | |

TECHNICAL FIELDS SEARCHED (IPC)

The present search report has been drawn up for all claims

| Place of search | Date of completion of the search | Examiner |
|---|---|---|
| Munich | 27 May 2020 | Griesinger, Irina |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
......................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 20 38 2140

This annex lists the patent family members relating to the patent documents cited in the above-mentioned European search report.
The members are as contained in the European Patent Office EDP file on
The European Patent Office is in no way liable for these particulars which are merely given for the purpose of information.

27-05-2020

| Patent document cited in search report | | Publication date | Patent family member(s) | | Publication date |
|---|---|---|---|---|---|
| US 2016340520 | A1 | 24-11-2016 | JP | WO2016043145 A1 | 03-08-2017 |
| | | | JP | WO2016043146 A1 | 03-08-2017 |
| | | | US | 2016319176 A1 | 03-11-2016 |
| | | | US | 2016340520 A1 | 24-11-2016 |
| | | | WO | 2016043145 A1 | 24-03-2016 |
| | | | WO | 2016043146 A1 | 24-03-2016 |
| WO 2013076372 | A1 | 30-05-2013 | FI | 20116175 A | 25-05-2013 |
| | | | WO | 2013076372 A1 | 30-05-2013 |
| WO 2015200232 | A2 | 30-12-2015 | US | 2015368368 A1 | 24-12-2015 |
| | | | US | 2017190800 A1 | 06-07-2017 |
| | | | US | 2019292277 A1 | 26-09-2019 |
| | | | WO | 2015200232 A2 | 30-12-2015 |
| EP 3192866 | A1 | 19-07-2017 | CA | 3011534 A1 | 20-07-2017 |
| | | | EP | 3192866 A1 | 19-07-2017 |
| | | | EP | 3402882 A1 | 21-11-2018 |
| | | | US | 2019062717 A1 | 28-02-2019 |
| | | | WO | 2017121902 A1 | 20-07-2017 |
| EP 3502126 | A1 | 26-06-2019 | EP | 3502126 A1 | 26-06-2019 |
| | | | WO | 2019121719 A1 | 27-06-2019 |

EPO FORM P0459

For more details about this annex : see Official Journal of the European Patent Office, No. 12/82

# EP 3 872 172 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 473545 A
- US 5612055 A
- WO 9206209 A
- WO 9720920 A
- EP 1222256 B1
- US 20130207294 A1
- US 4490282 A

### Non-patent literature cited in the description

- **IWAMOTO, S. et al.** *Appl. Phys. A 220,* vol. 89, 461-466
- **HENRIKSSON, M. et al.** *Eur. Polym. J.,* 2007, vol. 43, 3434-3441
- **ZHU, J.Y. et al.** *Green Chem.,* 2011, vol. 13, 1339-1344
- **SATYARMURTHY, P. et al.** *Carbohydr. Polym.,* 2011, vol. 83, 122-129
- **YARBROUGH, J. M. et al.** *Acs Nano,* 2017, vol. 11, 3101-3109
- **A. KAFY et al.** *Sensors and Actuators A,* 2016, vol. 247, 221-226
- **DA SILVA, T.A. et al.** *BioResources,* 2007, vol. 2 (4), 616-629
- **HU, J. et al.** *Sci. Rep.,* 2018, vol. 8 (1), 3195
- **KARLIN et al.** *Proc. Natl. Acad. Sci.,* 1990, vol. 87, 2264-8
- **KARLIN et al.** *Proc. Natl. Acad. Sci.,* 1993, vol. 90, 5873-7
- **ALTSCHUL et al.** *Nucleic Acids Res.,* 1991, vol. 25, 3389-402
- **ALTSCHUL et al.** *Nucleic Acids Res.,* 1997, vol. 25, 3389-402
- **ALTSCHUL et al.** *Methods in Enzymology,* 1996, vol. 266, 460-80
- **NEEDLEMAN ; WUNSCH.** *J. Mol. Biol.,* 1970, vol. 48, 444-53
- **MYERS ; MILLER.** *CABIOS,* 1989, vol. 4, 11-7
- **SMITH ; WATERMAN.** *Advances in Applied Mathematics,* 1981, vol. 2, 482-9
- **DORDO et al.** *J. Mol. Biol,* 1999, vol. 217, 721-739
- **TAYLOR et al.** *J. Theor. Biol.,* 1986, vol. 119, 205-218
- **KIM et al.** *Biotecnhology and bioprocess engineering,* 2013, vol. 19, 575-580
- **LEVASSEUR A. et al.** *Biotechnol Biofuels.,* 2013, vol. 6, 41
- **EIJSINK et al.** *Biotechnol Biofuels.,* 2019, vol. 12, 58
- **DE SOUZA LIMA, M. M. ; BORSALI, R.** *Macromol. Rapid Commun.,* 2004, vol. 25, 771-787